(19) Europäisches Patentamt
European Patent Office
Office européen des brevets

(11) EP 4 043 465 A1

(12) **EUROPEAN PATENT APPLICATION**
published in accordance with Art. 153(4) EPC

(43) Date of publication:
**17.08.2022 Bulletin 2022/33**

(21) Application number: **20869191.5**

(22) Date of filing: **25.09.2020**

(51) International Patent Classification (IPC):
***C07D 487/04*** *(2006.01)* ***A61K 31/41*** *(2006.01)*
***A61K 31/519*** *(2006.01)* ***A61K 31/5377*** *(2006.01)*
***A61P 35/00*** *(2006.01)* ***A61P 35/02*** *(2006.01)*

(52) Cooperative Patent Classification (CPC):
**A61K 31/41; A61K 31/519; A61K 31/53; A61K 31/5377; A61P 35/00; A61P 35/02; C07D 487/04**

(86) International application number:
**PCT/CN2020/117586**

(87) International publication number:
**WO 2021/057877 (01.04.2021 Gazette 2021/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.09.2019 CN 201910915840**

(71) Applicant: **Shenzhen TargetRx, Inc.**
**Shenzhen, Guangdong 518057 (CN)**

(72) Inventors:
- **WANG, Yihan**
  **Shenzhen, Guangdong 518057 (CN)**
- **XING, Qingfeng**
  **Shenzhen, Guangdong 518057 (CN)**
- **AI, Yixin**
  **Shenzhen, Guangdong 518057 (CN)**
- **LI, Huanyin**
  **Shenzhen, Guangdong 518057 (CN)**

(74) Representative: **Novitas Patent AB**
**P.O. Box 55557**
**102 04 Stockholm (SE)**

(54) **SUBSTITUTED AROMATIC FUSED RING DERIVATIVE AND COMPOSITION COMPRISING SAME, AND USE THEREOF**

(57) Provided in the present invention are a substituted aromatic fused ring derivative, a composition comprising the compound, and the use thereof. The substituted aromatic fused ring derivative is a compound as shown as formula (I) or a tautomer, stereoisomer, prodrug, crystal form, pharmaceutically acceptable salt, hydrate or solvate thereof. The compound and the composition of the present invention can be used to treat various protein tyrosine kinase-mediated diseases or conditions.

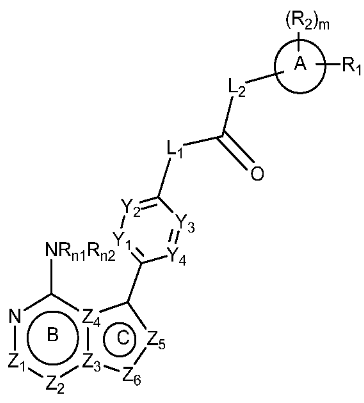

(I)

EP 4 043 465 A1

## Description

## FIELD OF THE INVENTION

The present disclosure belongs to a technical field of medicine, and particularly relates to a substituted aromatic fused ring derivative with inhibitory effect on protein tyrosine kinase, a pharmaceutical composition containing the same, and a preparation method and use thereof.

## BACKGROUND OF THE INVENTION

Protein kinases (PKs) are enzymes that catalyze the phosphorylation of specific serine, threonine or tyrosine in cellular proteins. Post-translational modifications of these substrate proteins act as molecular switches that play key roles in various biological processes, such as the control of cell growth, metabolism, tumor microenvironment (e.g., VEGFR), differentiation, and apoptosis. Abnormal, excessive or inappropriate PK activity has been observed in several disease states, including malignant proliferative diseases, such as functional mutations of medullary thyroid carcinoma (MTC) and other human malignant tumors, ITD (internal tandem duplication)-mutation in FLT3 of acute myeloid leukemia (AML), c-Kit mutation in gastrointestinal stromal tumor (GIST), and RET obtained from BCR-ABL rearrangement in chronic myelogenous leukemia (CML). In addition, the activation and/or overexpression of tyrosine kinases (e.g., TrkA, TrkB, TrkC, and RET) cause cancer. Many tyrosine kinases are homologous to each other: inhibition of one tyrosine kinase can also produce a certain inhibitory activity on other tyrosine kinases. Several targets for cancer therapy and the problems involved are briefly described below.

RET

RET (Rearranged during transfection) belongs to the family of receptor tyrosine kinase proteins and is a cell surface molecule that signaling cell growth and differentiation. RET gene mutation or RET gene fusion has been identified as a driving factor for certain cancers. The incidence of RET gene fusion in non-small cell lung cancer is about 2%, and the incidence of RET gene fusion in papillary thyroid cancers (PTCs) is 10% ~ 20%. The most common fusion partners include KIF5B, TRIM33, CCDC6 and NCOA4. The incidence of RET gene mutation in medullary thyroid cancers (MTCs) is about 60%, and the most common mutation site is M918T. RET inhibitor resistance mutations include, but are not limited to, amino acid position 804 (V804M, V804L, V804E), amino acid position 805 (E805K), and amino acid position 806 (Y806C, Y806E).

TRK

Trk (tropomyosin-related kinase) is high affinity receptor tyrosine kinase activated by a group of soluble growth factors called neurotrophin (NT). Trk receptor family has three members, namely TrkA, TrkB and TrkC. The neurotrophin includes (1) nerve growth factor (NGF) which can activate TrkA, (2) brain-derived neurotrophic factor (BDNF) and NT4/5 which can activate TrkB, and (3) NT3 which can activate TrkC. Trk is widely expressed in neuronal tissues and is involved in the maintenance, signaling and survival of neuronal cells. The overexpression, activation, amplification and/or mutation of Trk is associated with many cancers including neuroblastoma, ovarian cancer, breast cancer, prostate cancer, pancreatic cancer, multiple myeloma, astrocytoma and medulloblastoma, glioma, melanoma, thyroid cancer, pancreatic cancer, large cell neuroendocrine tumor and colorectal cancer. In addition, inhibitors of Trk/neurotrophin pathway have been shown to be effective in a variety of preclinical animal models for the treatment of pain and inflammatory diseases.

FLT3

FLT3 (FMS-like tyrosine kinase 3) belongs to the kinase protein of the class III receptor tyrosine kinase family. FLT3 is a receptor tyrosine kinase that plays a role in regulating production of normal hematopoietic cells and is overexpressed in leukemic embryonic cells. Mutations in the FLT3 gene are characterized by 30% of AML cases. Internal tandem duplication (ITD) mutation (accounting for about 23% of AML cases) in FLT3 is associated with a particularly poor prognosis. It is advantageous to inhibit FLT3 and mutations thereof.

c-Kit

c-KIT (also known as CD117) is a type of transmembrane receptor protein with tyrosine kinase activity encoded by retroviral proto-oncogene c-kit. The c-KIT kinase consists of an extracellular domain, a transmembrane domain and an intracellular domain. Ligand of c-KIT is a stem cell factor (SCF), which binds to the extracellular domain of c-KIT to

induce receptor dimerization and activate downstream signal transduction pathways. Mutations of c-KIT usually occur in DNA (exon 11) that encodes the domain of juxtamembrane regions. They also occur in exons 7, 8, 9, 13, 14, 17, and 18 at a lower frequency. The mutations make the function of c-KIT independent of activation by SCF, resulting in high cell division rate and possible genomic instability. The mutations of c-KIT have been involved in the pathogenesis of several diseases and conditions, including systemic mastocytosis (SM), gastrointestinal stromal tumor (GIST), acute myeloid (myelocytic) leukemia (AML), melanoma and seminoma. Therefore, there is a need to develop therapeutic agents inhibiting c-KIT, and in particular drugs inhibiting mutant c-KIT.

PDGFR

PDGFR (Platelet Derived Growth Factor Receptor) is a cell surface tyrosine kinase receptor that is a member of the platelet-derived growth factor (PDGF) family. PDGF subunits PDGF$\alpha$ and PDGF$\beta$ are vital factors in regulation of cell proliferation, cell differentiation, cell growth, development, and various diseases including cancers. D842V mutation of PDGFR$\alpha$ has been found in different isoforms of gastrointestinal stromal tumor (GIST), usually from the stomach. The D842V mutation is known to be associated with resistance to tyrosine kinase inhibitors.

VEGFR

VEGFR (vascular endothelial growth factor) is a vital signal transduction protein involved in angiogenesis and vasculogenesis. As the name implies, the activity of VEGFR is mainly limited to vascular endothelial cells, although VEGFR also has an effect on a limited number of other cell types. In vitro, it has been confirmed that VEGFR stimulates mitogenesis and migration of endothelial cells. VEGFR also promotes microvascular permeability, and is sometimes referred to as a vascular permeability factor. The VEGFR kinase has been used as a target for solid tumors, such as highly vascularized malignant tumors such as kidney cancer, glioblastoma, and liver cancer.

**SUMMARY OF THE INVENTION**

The present disclosure provides a novel aromatic fused ring derivative, a composition containing the compound, and use thereof. The aromatic fused ring derivative has better inhibitory activity and selectivity for some wild-type and mutant RET, KIF5B-RET, CCDC6-RET, TrkA, TrkB, TrkC, FLT3, FLT3-ITD, c-Kit, PDGFR, and VEGFR kinases, and has better pharmacodynamic and/or pharmacokinetic properties. The aromatic fused ring derivative can treat diseases mediated by protein kinases.

In this regard, the following technical solutions are used in the present disclosure:

In one aspect, the present disclosure relates to a compound of formula (I):

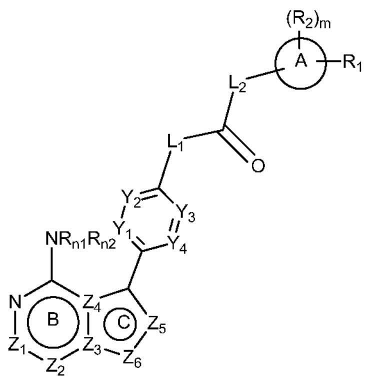

(I)

wherein

- ring A is a 5-membered heteroaryl group containing 1 to 3 heteroatoms selected from N, O and S;

  $R_1$ is -C($R_{a1}$)($R_{a2}$)($R_{a3}$), wherein $R_{a1}$, Ra2 and Ra3 are each independently selected from H, D, halogen, $C_{1-6}$ alkyl, $C_{1-6}$ haloalkyl, $C_{3-7}$ cycloalkyl, 3- to 7-membered heterocyclyl, $C_{1-6}$ alkoxy, $C_{1-6}$ haloalkoxy, -O$C_{3-7}$ cycloalkyl, and -O-3- to 7-membered heterocyclyl, wherein the $C_{1-6}$ alkyl, $C_{1-6}$ haloalkyl, $C_{3-7}$ cycloalkyl, 3- to 7-membered heterocyclyl, $C_{1-6}$ alkoxy, $C_{1-6}$ haloalkoxy, -O$C_{3-7}$ cycloalkyl, and -O-3- to 7-membered heterocyclyl are optionally substituted with one or more R;

each instance of $R_2$ is independently H, D, -OH, halogen, -CN, $-NO_2$, $-R_a$, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)OR_a$, or $-OC(O)NR_bR_c$;
m is selected from 0, 1, 2, and 3;

• $L_1$ is selected from bond, O, S, $NR_{L1}$, and $C(R_{L1})_2$,
• $L_2$ is selected from bond, O, S, $NR_{L2}$, and $C(R_{L2})_2$,
wherein each instance of $R_{L1}$ and $R_{L2}$ is independently selected from H, D, halogen, $C_{1-6}$ alkyl, and $C_{1-6}$ haloalkyl, wherein the $C_{1-6}$ alkyl and $C_{1-6}$ haloalkyl are optionally substituted with one or more R;
• $Y_1$, $Y_2$, $Y_3$ and $Y_4$ are each independently selected from $CR_Y$ and N;
wherein $R_Y$ is independently selected from H, D, -OH, halogen, -CN, $-NO_2$, $-R_a$, - $C(O)R_a$, $-C(O)OR_a$, $-C(O)NR_bR_c$, $-NR_bR_c$, $-NR_aC(O)R_b$, $-NR_aC(O)OR_b$, - $NR_aC(O)NR_bR_c$, $-OR_a$, $-OC(O)R_a$, $-OC(O)OR_a$, and $-OC(O)NR_bR_c$;
• ring B and ring C form an aromatic fused ring;

$R_{n1}$ and $R_{n2}$ are each independently selected from H, D, $C_{1-6}$ alkyl, $C_{1-6}$ haloalkyl, $C_{2-6}$ alkenyl and $C_{2-6}$ alkynyl;
$Z_1$ is selected from CRzi and N;
$Z_2$ is selected from $CR_{Z2}$ and N;
$Z_3$ and $Z_4$ are each independently selected from N atom and C atom;
$Z_5$ is selected from N atom and C atom, which are optionally substituted with $R_{Z5}$;
$Z_6$ is selected from N atom and C atom, which are optionally substituted with $R_{Z6}$;
wherein Rzi, $R_{Z2}$, $R_{Z5}$ and Rz6 are each independently selected from H, D, -OH, halogen, -CN, $-NO_2$, $-R_a$, $-C(O)R_a$, $-C(O)OR_a$, $-C(O)NR_bR_c$, $-NR_bR_c$, $-NR_aC(O)R_b$, - $NR_aC(O)OR_b$, $-NR_aC(O)NR_bR_c$, $-OR_a$, $-OC(O)R_a$, $-OC(O)OR_a$, and $-OC(O)NR_bR_c$;

• each of $R_a$, $R_b$ and $R_c$ is independently selected from H, D, $C_{1-6}$ alkyl, $C_{1-6}$ haloalkyl, $C_{2-6}$ alkenyl and $C_{2-6}$ alkynyl, or $R_b$ and $R_c$ together with the nitrogen atom to which they are attached form a 3- to 7-membered heterocyclyl or 5- to 10-membered heteroaryl group, wherein the group is optionally substituted with one or more R;

each R is independently selected from H, D, -OH, $-NH_2$, halogen, -CN, $-R_d$, - $C(O)R_d$, $-C(O)OR_d$, $-C(O)NR_eR_f$, $-NR_eR_f$, $-NR_dC(O)R_e$, $-NR_dC(O)OR_e$, - $NR_dC(O)NR_eR_f$, -ORd, $-OC(O)R_d$, $-OC(O)OR_d$, and $-OC(O)NR_eR_f$, or two R groups on the same atom or adjacent atoms can together form a $C_{3-7}$ cycloalkyl, 3- to 7-membered heterocyclyl, $C_{6-10}$ aryl or 5- to 10-membered heteroaryl group, wherein each group in the definition of R is optionally substituted with one or more D until fully deuterated;
each of $R_d$, $R_e$ and $R_f$ is independently selected from $C_{1-6}$ alkyl, $C_{1-6}$ haloalkyl, $C_{2-6}$ alkenyl and $C_{2-6}$ alkynyl, or $R_e$ and $R_f$ together with the nitrogen atom to which they are attached form a 3- to 7-membered heterocyclyl or 5- to 10-membered heteroaryl group, wherein each group in the definition of $R_d$, $R_e$ and $R_f$ is optionally substituted with one or more D until fully deuterated;

or a tautomer, stereoisomer, prodrug, crystal form, pharmaceutically acceptable salt, hydrate or solvate thereof.

In another aspect, the present disclosure provides a pharmaceutical composition, which comprises a compound disclosed herein, or a pharmaceutically acceptable salt, hydrate or solvate thereof, and pharmaceutically acceptable excipient (s). In a specific embodiment, the compound disclosed herein is provided in a therapeutically effective amount. In a specific embodiment, the compound disclosed herein is provided in a prophylactically effective amount.

In another aspect, the present disclosure provides use of a compound disclosed herein, or a pharmaceutically acceptable salt, hydrate or solvate thereof, or a pharmaceutical composition disclosed herein in the manufacture of a medicament for the treatment of diseases mediated by protein kinases.

In another aspect, the present disclosure provides a method of treating diseases, such as diseases mediated by protein kinases, in a subject, comprising administering to the subject a compound disclosed herein, or a pharmaceutically acceptable salt, hydrate or solvate thereof, or a pharmaceutical composition disclosed herein.

In another aspect, the present disclosure provides a compound disclosed herein, or a pharmaceutically acceptable salt, hydrate or solvate thereof, or a pharmaceutical composition disclosed herein, for treating diseases, such as diseases mediated by protein kinases.

In a specific embodiment, the diseases are mediated by at least one of wild-type or mutant RET, KIF5B-RET, CCDC6-RET, Trk, FLT3, c-Kit, PDGFR, or VEGFR kinases. In a specific embodiment, the mutant RET, KIF5B-RET, and CCDC6-RET are selected from V804L, V804M, V804E, M918T, E805K, Y806C, Y806E, C634Y, C634W, and G810R. In a specific embodiment, the Trk kinase is selected from Trk A, TrkB, and TrkC; in a specific embodiment, the mutant TrkA is G595R. In a specific embodiment, the mutant FLT3 and FLT3-ITD is selected from F691L, D835Y, D835V, D835H, D835F, D835E, Y842C, Y842D, Y842H, Y842N, and Y842S. In a specific embodiment, the mutant c-Kit is

selected from D816V, D816Y, D816F, D816K, D816A, and D816G. In a specific embodiment, the mutant PDGFR is D842V.

Other objects and advantages of the present disclosure will be apparent to those skilled in the art from the following specific embodiments, examples, and claims.

## Definitions

### Chemical definitions

The definitions of specific functional groups and chemical terms are described in more detail below.

When a range of values is listed, it is intended to encompass each value and sub-range within the range. For example, "$C_{1-6}$ alkyl" is intended to encompass $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$, and $C_{5-6}$ alkyl.

"$C_{1-6}$ alkyl" refers to a linear or branched, saturated hydrocarbon group having 1 to 6 carbon atoms, and is also referred to herein as "lower alkyl". In some embodiments, $C_{1-4}$ alkyl is alternative. Examples of alkyl include, but are not limited to: methyl ($C_1$), ethyl ($C_2$), n-propyl ($C_3$), iso-propyl ($C_3$), n-butyl ($C_4$), t-butyl ($C_4$), sec-butyl ($C_4$), iso-butyl ($C_4$), n-pentyl ($C_5$), 3-pentyl ($C_5$), pentyl ($C_5$), neo-pentyl ($C_5$), 3-methyl-2-butyl ($C_5$), t-pentyl ($C_5$) and n-hexyl ($C_6$). Regardless of whether or not the alkyl group is modified with "substituted", each alkyl group is independently optionally substituted, for example, with 1 to 5 substituents, 1 to 3 substituents or 1 substituent. Appropriate substituents are defined as follows.

"$C_{2-6}$ alkenyl" refers to a linear or branched, hydrocarbon group having 2-6 carbon atoms and one or more carbon-carbon double bonds (e.g., 1, 2, or 3 carbon-carbon double bonds). One or more carbon-carbon double bonds can be internal (e.g., in 2-butenyl) or terminal (e.g., in 1-butenyl). In some embodiments, $C_{2-4}$ alkenyl is alternative. Examples of alkenyl include, but are not limited to: vinyl ($C_2$), 1-propenyl ($C_3$), 2-propenyl ($C_3$), 1-butenyl ($C_4$), 2-butenyl ($C_4$), butadienyl ($C_4$), pentenyl ($C_5$), pentadienyl ($C_5$), hexenyl ($C_6$), etc. Regardless of whether or not the alkenyl group is modified with "substituted", each alkenyl group is independently optionally substituted, for example, with 1 to 5 substituents, 1 to 3 substituents or 1 substituent. Appropriate substituents are defined as follows.

"$C_{2-6}$ alkynyl" refers to a linear or branched, hydrocarbon group having 2-6 carbon atoms, one or more carbon-carbon triple bonds (e.g., 1, 2 or 3 carbon-carbon triple bonds) and optionally one or more carbon-carbon double bonds (e.g., 1, 2 or 3 carbon-carbon double bonds). In some embodiments, $C_{2-4}$ alkynyl is alternative. In some embodiments, alkynyl does not contain any double bond. One or more carbon-carbon triple bonds can be internal (e.g., in 2-butynyl) or terminal (e.g., in 1-butynyl). Examples of alkynyl include, but are not limited to: ethynyl ($C_2$), 1-propynyl ($C_3$), 2-propynyl ($C_3$), 1-butynyl ($C_4$), 2-butynyl ($C_4$), pentynyl ($C_5$), hexynyl ($C_6$), etc. Regardless of whether or not the alkynyl group is modified with "substituted", each alkynyl group is independently optionally substituted, for example, with 1 to 5 substituents, 1 to 3 substituents or 1 substituent. Appropriate substituents are defined as follows.

"$C_{1-6}$ alkoxyl" refers to a -OR group, wherein R is substituted or unsubstituted $C_{1-6}$ alkyl. In some embodiments, $C_{1-4}$ alkoxyl is alternative. Specifically, alkoxyl includes, but is not limited to: methoxy, ethoxy, n-propoxy, iso-propoxy, n-butoxy, t-butoxy, sec-butoxy, n-pentyloxy, n-hexyloxy and 1,2-dimethylbutoxy.

"Halo" or "halogen" refers to fluoro (F), chloro (Cl), bromo (Br) and iodo (I). In some embodiments, the halo group is F, Cl or Br. In some embodiments, the halo group is F or Cl. In some embodiments, the halo group is F.

Therefore, "$C_{1-6}$ haloalkyl" and "$C_{1-6}$ haloalkoxyl" refer to the above "$C_{1-6}$ alkyl" and "$C_{1-6}$ alkoxyl" substituted with one or more halo groups. In some embodiments, $C_{1-4}$haloalkyl is alternative, and $C_{1-2}$ haloalkyl is yet alternative. In some embodiments, $C_{1-4}$ haloalkoxyl is alternative, and $C_{1-2}$ haloalkoxyl is yet alternative. Examples of haloalkyl include, but are not limited to: $-CF_3$, $-CH_2F$, $-CHF_2$, $-CHFCH_2F$, $-CH_2CHF_2$, $-CF_2CF_3$, $-CCl_3$, $-CH_2Cl$, $-CHCl_2$, 2,2,2-trifluoro-1,1-dimethyl-ethyl, etc. Examples of haloalkoxyl include, but are not limited to: - $OCH_2F$, $-OCHF_2$, $-OCF_3$, etc.

"$C_{3-10}$ cycloalkyl" refers to a non-aromatic cyclic hydrocarbon group having 3-10 ring carbon atoms and zero heteroatoms. In some embodiments, $C_{3-7}$ cycloalkyl is alternative, $C_{3-6}$ cycloalkyl is alternative, and $C_{5-6}$ cycloalkyl is yet alternative. Cycloalkyl also includes a ring system in which the above cycloalkyl ring is fused with one or more aryl or heteroaryl groups, wherein the point of attachment is on the cycloalkyl ring, and in such instances, the number of carbons continue to designate the number of carbons in the cycloalkyl system. Examples of cycloalkyl include, but are not limited to: cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), cyclooctyl ($C_8$), cyclooctenyl ($C_8$), bicyclo[2.2.1]heptyl ($C_7$), bicyclo[2.2.2]octyl ($C_8$), cyclononyl ($C_9$), cyclononenyl ($C_9$), cyclodecyl ($C_{10}$), cyclodecenyl ($C_{10}$), octahydro-1H-indenyl ($C_9$), decahydronaphthyl ($C_{10}$), spiro[4.5]decyl ($C_{10}$), etc. Regardless of whether or not the cycloalkyl group is modified with "substituted", each cycloalkyl group is independently optionally substituted, for example, with 1 to 5 substituents, 1 to 3 substituents or 1 substituent. Appropriate substituents are defined as follows.

"3- to 10-membered heterocyclyl" refers to a radical of a 3- to 10-membered non-aromatic ring system having ring carbon atoms and 1 to 4 ring heteroatoms, wherein each heteroatom is independently selected from nitrogen,

oxygen, sulfur, boron, phosphorus, and silicon. In heterocyclyl groups that contain one or more nitrogen atoms, the point of attachment can be a carbon or nitrogen atom, as valency permits. In some embodiments, 3- to 7-membered heterocyclyl is alternative, and it is a 3- to 7-membered non-aromatic ring system having ring carbon atoms and 1-3 ring heteroatoms. In some embodiments, 3- to 6-membered heterocyclyl is alternative, and it is a 3- to 6-membered non-aromatic ring system having ring carbon atoms and 1-3 ring heteroatoms. 5- to 6-membered heterocyclyl is yet alternative, and it is a 5- to 6-membered non-aromatic ring system having ring carbon atoms and 1-3 ring heteroatoms. "Heterocyclyl" also includes ring systems wherein the heterocyclyl, as defined above, is fused with one or more cycloalkyl, aryl or heteroaryl groups wherein the point of attachment is on the heterocyclyl ring, and in such instances, the number of ring members continues to designate the number of ring members in the heterocyclyl ring system. Regardless of whether or not the heterocyclyl group is modified with "substituted", each heterocyclyl group is independently optionally substituted, for example, with 1 to 5 substituents, 1 to 3 substituents or 1 substituent. Appropriate substituents are defined as follows.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing three heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a $C_6$ aryl ring (also referred to herein as a 5,6-bicyclic heterocyclyl) include, without limitation, indolinyl, isoindolinyl, dihydrobenzofuranyl, dihydrobenzothienyl, benzoxazolinonyl, and the like. Exemplary 6-membered heterocyclyl groups fused to a $C_6$ aryl ring (also referred to herein as a 6,6-bicyclic heterocyclyl) include, without limitation, tetrahydroquinolinyl, tetrahydroisoquinolinyl, and the like.

"$C_{6-14}$ aryl" refers to a radical of a monocyclic or polycyclic (e.g., bicyclic or tricyclic) 4n+2 aromatic ring system (e.g., having 6, 10, or 14 pi electrons shared in a cyclic array) having 6-14 ring carbon atoms and zero heteroatoms. In some embodiments, an aryl group has six ring carbon atoms (C6 aryl"; e.g., phenyl). In some embodiments, an aryl group has ten ring carbon atoms ("$C_{10}$ aryl"; e.g., naphthyl such as 1-naphthyl and 2-naphthyl). In some embodiments, an aryl group has fourteen ring carbon atoms ("$C_{14}$ aryl"; e.g., anthracyl). In some embodiments, $C_{6-10}$ aryl is alternative, and $C_6$ aryl is yet alternative. "Aryl" also includes ring systems wherein the aryl ring, as defined above, is fused with one or more cycloalkyl or heterocyclyl groups wherein the point of attachment is on the aryl ring, and in such instances, the number of carbon atoms continues to designate the number of carbon atoms in the aryl ring system. Regardless of whether or not the aryl group is modified with "substituted", each aryl group is independently optionally substituted, for example, with 1 to 5 substituents, 1 to 3 substituents or 1 substituent. Appropriate substituents are defined as follows.

"5- to 10-membered heteroaryl" refers to a radical of a 5- to 10-membered monocyclic or bicyclic 4n+2 aromatic ring system (e.g., having 6 or 10 pi electrons shared in a cyclic array) having ring carbon atoms and 1-4 ring heteroatoms, wherein each heteroatom is independently selected from nitrogen, oxygen and sulfur. In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" also includes ring systems wherein the heteroaryl ring, as defined above, is fused with one or more cycloalkyl or heterocyclyl groups wherein the point of attachment is on the heteroaryl ring, and in such instances, the number of carbon atoms continues to designate the number of carbon atoms in the heteroaryl ring system. In some embodiments, 5- to 6-membered heteroaryl is alternative, and it is a 5- to 6-membered monocyclic or bicyclic 4n+2 aromatic ring system having ring carbon atoms and 1-4 ring heteroatoms. Regardless of whether or not the heteroaryl group is modified with "substituted", each heteroaryl group is independently optionally substituted, for example, with 1 to 5 substituents, 1 to 3 substituents or 1 substituent. Appropriate substituents are defined as follows.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary

7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

Exemplary substituents on carbon atoms include, but are not limited to: halo, -CN, - $NO_2$, $-N_3$, $-SO_2H$, $-SO_3H$, -OH, $-OR^{aa}$, $-ON(R^{bb})_2$, $-N(R^{bb})_2$, $-N(R^{bb})_3^+X^-$, $-N(OR^{cc})R^{bb}$, -SH, - $SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, - $C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, - $C(=NR^{bb})R^{aa}$, $-C(=NR^{bb})OR^{aa}$, $-OC(=NR^{bb})R^{aa}$, $-OC(=NR^{bb})OR^{aa}$, $-C(=NR^{bb})N(R^{bb})_2$, - $OC(=NR^{bb})N(R^{bb})_2$, $-NR^{bb}C(=NR^{bb})N(R^{bb})_2$, $-C(=O)NR^{bb}SO_2R^{aa}$, $-NR^{bb}SO_2R^{aa}$, $-SO_2N(R^{bb})_2$, - $SO_2R^{aa}$, $-SO_2OR^{aa}$, $-OSO_2R^{aa}$, $-S(=O)R^{aa}$, $-OS(=O)R^{aa}$, $-Si(R^{aa})_3$, $-OSi(R^{aa})_3$, $-C(=S)N(R^{bb})_2$, - $C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, $-SC(=O)R^{aa}$, $-P(=O)_2R^{aa}$, $-OP(=O)_2R^{aa}$, $-P(=O)(R^{aa})_2$, $-OP(=O)(R^{aa})_2$, $-OP(=O)(OR^{cc})_2$, $-P(=O)_2N(R^{bb})_2$, - $OP(=O)_2N(R^{bb})_2$, $-P(=O)(NR^{bb})_2$, $-OP(=O)(NR^{bb})_2$, $-NR^{bb}P(=O)(OR^{cc})_2$, $-NR^{bb}P(=O)(NR^{bb})_2$, - $P(R^{cc})_2$, $-P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, alkyl, haloalkyl, alkenyl, alkynyl, carbocyclyl, heterocyclyl, aryl and heteroaryl, wherein each of alkyl, alkenyl, alkynyl, carbocyclyl, heterocyclyl, aryl and heteroaryl is independently substituted with 0, 1, 2, 3, 4 or 5 $R^{dd}$ groups;

or two geminal hydrogens on a carbon atom are replaced with the group =O, =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=NR^{bb}$ or $=NOR^{cc}$;

each $R^{aa}$ is independently selected from alkyl, haloalkyl, alkenyl, alkynyl, carbocyclyl, heterocyclyl, aryl and heteroaryl, or two $R^{aa}$ groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 $R^{dd}$ groups;

each $R^{bb}$ is independently selected from: hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{cc})OR^{aa}$, $-C(=NR^{cc})N(R^{cc})_2$, $-SO_2N(R^{cc})_2$, $-SO_2R^{cc}$, - $SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, - $P(=O)_2N(R^{cc})_2$, $-P(=O)(NR^{cc})_2$, alkyl, haloalkyl, alkenyl, alkynyl, carbocyclyl, heterocyclyl, aryl and heteroaryl, or two $R^{bb}$ groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 $R^{dd}$ groups;

each $R^{cc}$ is independently selected from hydrogen, alkyl, haloalkyl, alkenyl, alkynyl, carbocyclyl, heterocyclyl, aryl and heteroaryl, or two $R^{cc}$ groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 $R^{dd}$ groups;

each $R^{dd}$ is independently selected from: halo, -CN, $-NO_2$, $-N_3$, $-SO_2H$, $-SO_3H$, -OH, $-OR^{ee}$, $-ON(R^{ff})_2$, $-N(R^{ff})_2$, , $-N(R^{ff})_3^+X^-$, $-N(OR^{ee})R^{ff}$, -SH, $-SR^{ee}$, $-SSR^{ee}$, $-C(=O)R^{ee}$, $-CO_2H$, $-CO_2R^{ee}$, $-OC(=O)R^{ee}$, $-OCO_2R^{ee}$, $-C(=O)N(R^{ff})_2$, $-OC(=O)N(R^{ff})_2$, $-NR^{ff}C(=O)R^{ee}$, $-NR^{ff}CO_2R^{ee}$, - $NR^{ff}C(=O)N(R^{ff})_2$, $-C(=NR^{ff})OR^{ee}$, $-OC(=NR^{ff})R^{ee}$, $-OC(=NR^{ff})OR^{ee}$, $-C(=NR^{ff})N(R^{ff})_2$, - $OC(=NR^{ff})N(R^{ff})_2$, $-NR^{ff}C(=NR^{ff})N(R^{ff})_2$, $-NR^{ff}SO_2R^{ee}$, $-SO_2N(R^{ff})_2$, $-SO_2R^{ee}$, $-SO_2OR^{ee}$, - $OSO_2R^{ee}$, $-S(=O)R^{ee}$, $-Si(R^{ee})_3$, $-OSi(R^{ee})_3$, $-C(=S)N(R^{ff})_2$, $-C(=O)SR^{ee}$, $-C(=S)SR^{ee}$, - $SC(=S)SR^{ee}$, $-P(=O)_2R^{ee}$, $-P(=O)(R^{ee})_2$, $-OP(=O)(R^{ee})_2$, $-OP(=O)(OR^{ee})_2$, alkyl, haloalkyl, alkenyl, alkynyl, carbocyclyl, heterocyclyl, aryl, and heteroaryl, wherein each of alkyl, alkenyl, alkynyl, carbocyclyl, heterocyclyl, aryl and heteroaryl is independently substituted with 0, 1, 2, 3, 4 or 5 $R^{gg}$ groups, or two geminal $R^{dd}$ substituents can be bound to form =O or =S;

each $R^{ee}$ is independently selected from alkyl, haloalkyl, alkenyl, alkynyl, carbocyclyl, aryl, heterocyclyl and heteroaryl, wherein each of alkyl, alkenyl, alkynyl, carbocyclyl, heterocyclyl, aryl and heteroaryl is independently substituted with 0, 1, 2, 3, 4 or 5 $R^{gg}$ groups;

each $R^{ff}$ is independently selected from hydrogen, alkyl, haloalkyl, alkenyl, alkynyl, carbocyclyl, heterocyclyl, aryl and heteroaryl, or two $R^{ff}$ groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 $R^{gg}$ groups;

each $R^{gg}$ is independently: halo, -CN, $-NO_2$, $-N_3$, $-SO_2H$, $-SO_3H$, -OH, $-OC_{1-6}$ alkyl, - $ON(C_{1-6}\ alkyl)_2$, $-N(C_{1-6}\ alkyl)_2$, $-N(C_{1-6}\ alkyl)_3^+X^-$, $-NH(C_{1-6}\ alkyl)_2^+X^-$, $-NH_2(C_{1-6}\ alkyl)^+X^-$, - $NH_3^+X^-$, $-N(OC_{1-6}\ alkyl)(C_{1-6}\ alkyl)$, $-N(OH)(C_{1-6}\ alkyl)$, -NH(OH), -SH, $-SC_{1-6}$ alkyl, $-SS(C_{1-6}\ alkyl)$, $-C(=O)(C_{1-6}\ alkyl)$, $-CO_2H$, $-CO_2(C_{1-6}\ alkyl)$, $-OC(=O)(C_{1-6}\ alkyl)$, $-OCO_2(C_{1-6}\ alkyl)$, - $C(=O)NH_2$, $-C(=O)N(C_{1-6}\ alkyl)_2$, $-OC(=O)NH(C_{1-6}\ alkyl)$, $-NHC(=O)(C_{1-6}\ alkyl)$, $-N(C_{1-6}\ alkyl)C(=O)(C_{1-6}\ alkyl)$, $-NHCO_2(C_{1-6}\ alkyl)$, $-NHC(=O)N(C_{1-6}\ alkyl)_2$, $-NHC(=O)NH(C_{1-6}\ alkyl)$, $-NHC(=O)NH_2$, $-C(=NH)O(C_{1-6}\ alkyl)$, $-OC(=NH)(C_{1-6}\ alkyl)$, $-OC(=NH)OC_{1-6}$ alkyl, - $C(=NH)N(C_{1-6}\ alkyl)_2$, $-C(=NH)NH(C_{1-6}\ alkyl)$, $-C(=NH)NH_2$, $-OC(=NH)N(C_{1-6}\ alkyl)_2$, - $OC(NH)NH(C_{1-6}\ alkyl)$, $-OC(NH)NH_2$, $-NHC(NH)N(C_{1-6}\ alkyl)_2$, $-NHC(=NH)NH_2$, - $NHSO_2(C_{1-6}\ alkyl)$, $-SO_2N(C_{1-6}\ alkyl)_2$, $-SO_2NH(C_{1-6}\ alkyl)$, $-SO_2NH_2$, $-SO_2C_{1-6}$ alkyl, $-SO_2OC_{1-6}$ alkyl, $-OSO_2C_{1-6}$ alkyl, $-SOC_{1-6}$ alkyl, $-Si(C_{1-6}\ alkyl)_3$, $-OSi(C_{1-6}\ alkyl)_3$, $-C(=S)N(C_{1-6}\ alkyl)_2$, $C(=S)NH(C_{1-6}\ alkyl)$, $C(=S)NH_2$, $-C(=O)S(C_{1-6}\ alkyl)$, $-C(=S)SC_{1-6}$ alkyl, $-SC(=S)SC_{1-6}$ alkyl, - $P(=O)_2(C_{1-6}\ alkyl)$, $-P(=O)(C_{1-6}\ alkyl)_2$, $-OP(=O)(C_{1-6}\ alkyl)_2$, $-OP(=O)(OC_{1-6}\ alkyl)_2$, $C_{1-6}$ alkyl, $C_{1-6}$ haloalkyl, $C_2$-$C_6$ alkenyl, $C_2$-$C_6$ alkynyl, $C_3$-$C_7$ carbo-

cyclyl, $C_6$-$C_{10}$ aryl, $C_3$-$C_7$ heterocyclyl, or $C_5$-$C_{10}$heteroaryl; or two geminal $R^{gg}$ substituents can be bound to form =O or =S, wherein $X^-$ is a counter ion.

Exemplary substituents on the nitrogen atom include but are not limited to: hydrogen, - OH, -$OR^{aa}$, -$N(R^{cc})_2$, -CN, -C(=O)$R^{aa}$, -C(=O)$N(R^{cc})_2$, -$CO_2R^{aa}$, -$SO_2R^{aa}$, -C(=$NR^{bb}$)$R^{aa}$, - C(=$NR^{cc}$)$OR^{aa}$, -C(=$NR^{cc}$)$N(R^{cc})_2$, -$SO_2N(R^{cc})_2$, -$SO_2R^{cc}$, -$SO_2OR^{cc}$, -$SOR^{aa}$, -C(=S)$N(R^{cc})_2$, - C(=O)$SR^{cc}$, -C(=S)$SR^{cc}$, -$P(=O)_2R^{aa}$, -P(=O)($R^{aa}$)2, -$P(=O)_2N(R^{cc})_2$, -P(=O)$(NR^{cc})_2$, alkyl, haloalkyl, alkenyl, alkynyl, carbocyclyl, heterocyclyl, aryl and heteroaryl, or two $R^{cc}$ groups connected to the nitrogen atom are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 $R^{dd}$ groups, and wherein $R^{aa}$, $R^{bb}$, $R^{cc}$ and $R^{dd}$ are as defined above.

"Deuterated", "deuteration", or "D" means that one or more hydrogens in a compound or group are replaced by deuterium; Deuteration can be mono-, di-, poly-, or fully-substituted. The term "substituted with one or more deuteriums" can be used interchangeably with "deuterated one or more times".

"Non-deuterated compound" refers to a compound whose content of deuterium atoms is not higher than the natural content (0.015%) of deuterium isotope.

The content of deuterium isotope at a deuterated position is at least greater than the natural content of deuterium isotope (0.015%), alternatively greater than 30%, yet alternatively greater than 50%, yet alternatively greater than 75%, yet alternatively greater than 95%, or yet alternatively greater than 99%.

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al., describe pharmaceutically acceptable salts in detail in J. Pharmaceutical Sciences (1977) 66:1-19. Pharmaceutically acceptable salts of the compounds disclosed herein include those derived from suitable inorganic and organic acids and bases. Examples of pharmaceutically acceptable, nontoxic acid addition salts are salts formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid. Salts formed using conventional methods in the art such as ion exchange are also included. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and $N^+(C_{1-4}alkyl)_4$ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

A "subject" to which administration is contemplated includes, but is not limited to, humans (i.e., a male or female of any age group, e.g., a pediatric subject (e.g., infant, child, adolescent) or adult subject (e.g., young adult, middle-aged adult or senior adult)) and/or a non-human animal, e.g., a mammal such as primates (e.g., cynomolgus monkeys, rhesus monkeys), cattle, pigs, horses, sheep, goats, rodents, cats, and/or dogs. In some embodiments, the subject is a human. In some embodiments, the subject is a non-human animal. The terms "human," "patient," and "subject" are used interchangeably herein.

"Disease", "disorder" and "condition" are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplates an action that occurs before a subject begins to suffer from the specified disease, disorder or condition ("prophylactic treatment").

"Combination", "combined", and related terms refer to the simultaneous or sequential administration of the therapeutic agents of the present disclosure. For example, the compound disclosed herein may be administered with another therapeutic agent simultaneously or sequentially in separate unit dosage forms, or together in a single unit dosage form.

## BRIEF DESCRIPTION OF THE DRAWINGS

FIG. 1 is a curve of tumor volume growth of each group of mice in tumor model of Ba/F3 KIF5B-RET cell line.
FIG. 2 is a curve of body weight change of each group of mice over treatment time in tumor model of Ba/F3 KIF5B-RET cell line.
FIG. 3 is a curve of weight percentage change of each group of mice over treatment time in tumor model of Ba/F3 KIF5B-RET cell line.
FIG. 4 is a curve of tumor volume growth of each group of mice in tumor model of Ba/F3 KIF5B-RET$^{G810R}$ cell line.
FIG. 5 is a curve of body weight change of each group of mice over treatment time in tumor model of Ba/F3 KIF5B-RET$^{G810R}$ cell line.
FIG. 6 is a curve of weight percentage change of each group of mice over treatment time in tumor model of Ba/F3 KIF5B-RET$^{G810R}$ cell line.

## DETAILED DESCRIPTION OF THE INVENTION

Compound

As used herein, "compound of the present disclosure" refers to the following compound of formula (I) (including subsets of each formula), or a pharmaceutically acceptable salt, hydrate, or solvate thereof.
In one embodiment, the present disclosure relates to a compound of formula (I):

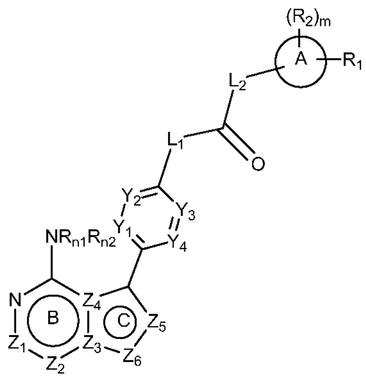
(I)

wherein

- ring A is a 5-membered heteroaryl group containing 1 to 3 heteroatoms selected from N, O and S;

  $R_1$ is $-C(R_{a1})(R_{a2})(R_{a3})$, wherein $R_{a1}$, Ra2 and Ra3 are each independently selected from H, D, halogen, $C_{1-6}$ alkyl, $C_{1-6}$ haloalkyl, $C_{3-7}$ cycloalkyl, 3- to 7-membered heterocyclyl, $C_{1-6}$ alkoxy, $C_{1-6}$ haloalkoxy, $-OC_{3-7}$ cycloalkyl, and -O-3- to 7-membered heterocyclyl, wherein the $C_{1-6}$ alkyl, $C_{1-6}$ haloalkyl, $C_{3-7}$ cycloalkyl, 3- to 7-membered heterocyclyl, $C_{1-6}$ alkoxy, $C_{1-6}$ haloalkoxy, $-OC_{3-7}$ cycloalkyl, and -O-3- to 7-membered heterocyclyl are optionally substituted with one or more R;
  each instance of $R_2$ is independently H, D, -OH, halogen, -CN, $-NO_2$, $-R_a$, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)OR_a$, or $-OC(O)NR_bR_c$;
  m is selected from 0, 1, 2, and 3;

- $L_1$ is selected from bond, O, S, $NR_{L1}$, and $C(R_{L1})_2$,
- $L_2$ is selected from bond, O, S, $NR_{L2}$, and $C(R_{L2})_2$,
  wherein each instance of $R_{L1}$ and $R_{L2}$ is independently selected from H, D, halogen, $C_{1-6}$ alkyl, and $C_{1-6}$ haloalkyl, wherein the $C_{1-6}$ alkyl and $C_{1-6}$ haloalkyl are optionally substituted with one or more R;
- $Y_1$, $Y_2$, $Y_3$ and $Y_4$ are each independently selected from CRy and N;
  wherein $R_Y$ is independently selected from H, D, -OH, halogen, -CN, $-NO_2$, $-R_a$, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)OR_a$, and $-OC(O)NR_bR_c$;
- ring B and ring C form an aromatic fused ring;

$R_{n1}$ and $R_{n2}$ are each independently selected from H, D, $C_{1-6}$ alkyl, $C_{1-6}$ haloalkyl, $C_{2-6}$ alkenyl and $C_{2-6}$ alkynyl;
$Z_1$ is selected from CRzi and N;
$Z_2$ is selected from $CR_{Z2}$ and N;
$Z_3$ and $Z_4$ are each independently selected from N atom and C atom;
$Z_5$ is selected from N atom and C atom, which are optionally substituted with $R_{Z5}$;
$Z_6$ is selected from N atom and C atom, which are optionally substituted with $R_{Z6}$;
wherein Rzi, $R_{Z2}$, $R_{Z5}$ and Rz6 are each independently selected from H, D, -OH, halogen, -CN, $-NO_2$, $-R_a$, $-C(O)R_a$, $-C(O)OR_a$, $-C(O)NR_bR_c$, $-NR_bR_c$, $-NR_aC(O)R_b$, - $NR_aC(O)OR_b$, $-NR_aC(O)NR_bR_c$, $-OR_a$, $-OC(O)R_a$, $-OC(O)OR_a$, and $-OC(O)NR_bR_c$;

• each of $R_a$, $R_b$ and $R_c$ is independently selected from H, D, $C_{1-6}$ alkyl, $C_{1-6}$ haloalkyl, $C_{2-6}$ alkenyl and $C_{2-6}$ alkynyl, or $R_b$ and $R_c$ together with the nitrogen atom to which they are attached form a 3- to 7-membered heterocyclyl or 5- to 10-membered heteroaryl group, wherein the group is optionally substituted with one or more R;

each R is independently selected from H, D, -OH, $-NH_2$, halogen, -CN, $-R_d$, - $C(O)R_d$, $-C(O)OR_d$, $-C(O)NR_eR_f$, $-NR_eR_f$, $-NR_dC(O)R_e$, $-NR_dC(O)OR_e$, - $NR_dC(O)NR_eR_f$, -ORd, $-OC(O)R_d$, $-OC(O)OR_d$, and $-OC(O)NR_eR_f$, or two R groups on the same atom or adjacent atoms can together form a $C_{3-7}$ cycloalkyl, 3- to 7-membered heterocyclyl, $C_{6-10}$ aryl or 5- to 10-membered heteroaryl group, wherein each group in the definition of R is optionally substituted with one or more D until fully deuterated;
each of $R_d$, $R_e$ and $R_f$ is independently selected from $C_{1-6}$ alkyl, $C_{1-6}$ haloalkyl, $C_{2-6}$ alkenyl and $C_{2-6}$ alkynyl, or $R_e$ and $R_f$ together with the nitrogen atom to which they are attached form a 3- to 7-membered heterocyclyl or 5- to 10-membered heteroaryl group, wherein each group in the definition of $R_d$, $R_e$ and $R_f$ is optionally substituted with one or more D until fully deuterated;

or a tautomer, stereoisomer, prodrug, crystal form, pharmaceutically acceptable salt, hydrate or solvate thereof.

Ring A

In a specific embodiment, ring A is a 5- to 6-membered heteroaryl group containing 1 to 3 heteroatoms selected from N, O and S; in another specific embodiment, ring A is selected from pyrrolyl, pyrazolyl, imidazolyl, furyl, oxazolyl, isoxazolyl, thienyl, thiazolyl and isothiazolyl; in another specific embodiment, ring A is selected from imidazolyl, isoxazolyl and isothiazolyl; in another specific embodiment, ring A is selected from isoxazolyl and isothiazolyl;
In a specific embodiment, ring A is selected from

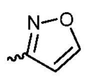, 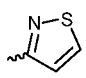, 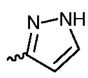, 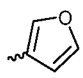,

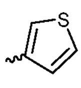 and 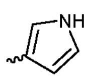;

In another specific embodiment, ring A is selected from

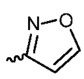, 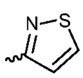

and

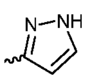;

In another specific embodiment, ring A is

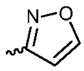.

$R_1$

In a specific embodiment, $R_1$ is $-C(R_{a1})(R_{a2})(R_{a3})$, wherein $R_{a1}$, Ra2 and Ra3 are each independently selected from H, D, halogen, $C_{1-6}$ alkyl, $C_{1-6}$ haloalkyl, $C_{3-7}$ cycloalkyl, 3- to 7-membered heterocyclyl, $C_{1-6}$ alkoxy, $C_{1-6}$ haloalkoxy, $-OC_{3-7}$ cycloalkyl, and -O-3- to 7-membered heterocyclyl, wherein the $C_{1-6}$ alkyl, $C_{1-6}$ haloalkyl, $C_{3-7}$ cycloalkyl, 3- to 7-membered heterocyclyl, $C_{1-6}$ alkoxy, $C_{1-6}$ haloalkoxy, $-OC_{3-7}$ cycloalkyl, and the -O-3- to 7-membered heterocyclyl are optionally substituted with one or more R.

In another specific embodiment, $R_1$ is $-C(R_{a1})(R_{a2})(R_{a3})$, wherein $R_{a1}$, Ra2 and Ra3 are each independently selected from H, D, halogen, $C_{1-6}$ alkyl, $C_{1-6}$ haloalkyl, $C_{3-7}$ cycloalkyl, and 3- to 7-membered heterocyclyl, wherein the $C_{1-6}$ alkyl, $C_{1-6}$ haloalkyl, $C_{3-7}$ cycloalkyl and 3- to 7-membered heterocyclyl are optionally substituted with one or more R.

In another specific embodiment, $R_1$ is $-C(R_{a1})(R_{a2})(R_{a3})$, wherein $R_{a1}$, Ra2 and Ra3 are each independently selected from H, D, halogen, $C_{1-6}$ alkyl, and $C_{1-6}$ haloalkyl, wherein the $C_{1-6}$ alkyl and $C_{1-6}$ haloalkyl are optionally substituted with one or more R.

In another specific embodiment, $R_1$ is $-C(R_{a1})(R_{a2})(R_{a3})$, wherein $R_{a1}$, Ra2 and Ra3 are each independently selected from $C_{1-4}$ alkyl, and $C_{1-4}$ haloalkyl, wherein the $C_{1-4}$ alkyl and $C_{1-4}$ haloalkyl are optionally substituted with one or more R.

In another specific embodiment, $R_1$ is $-C(R_{a1})(R_{a2})(R_{a3})$, wherein $R_{a1}$, $R_{a2}$ and Ra3 are each independently selected from methyl and halomethyl.

In another specific embodiment, $R_1$ is $-C(R_{a1})(R_{a2})(R_{a3})$, wherein $R_{a1}$, Ra2 and Ra3 are each independently selected from H, D, F, Cl, methyl, $-CD_3$, and trifluoromethyl.

In another specific embodiment, $R_1$ is $-C(R_{a1})(R_{a2})(R_{a3})$, wherein at least one of $R_{a1}$, Ra2 and Ra3 is haloalkyl.

$R_2$

In a specific embodiment, each instance of $R_2$ is independently H, D, -OH, halogen, - CN, $-NO_2$, $-R_a$, $-C(O)R_a$, $-C(O)OR_a$, $-C(O)NR_bR_c$, $-NR_bR_c$, $-NR_aC(O)R_b$, $-NR_aC(O)OR_b$, - $NR_aC(O)NR_bR_c$, $-OR_a$, $-OC(O)R_a$, $-OC(O)OR_a$, or $-OC(O)NR_bR_c$.

In another specific embodiment, each instance of $R_2$ is independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R.

In another specific embodiment, each instance of $R_2$ is independently H, D, F, methyl, or $-CD_3$.

In another specific embodiment, each group in the definition of $R_2$ is optionally substituted with one or more D until fully deuterated.

m

In a specific embodiment, m is selected from 0, 1, and 2; In another specific embodiment, m is selected from 0 and 1; In another specific embodiment, m is 0; In another specific embodiment, m is 1; In another specific embodiment, m is 2.

$L_1$ and $L_2$

In a specific embodiment, $L_1$ is a bond; In another specific embodiment, $L_1$ is O; In another specific embodiment, $L_1$ is S; In another specific embodiment, $L_1$ is $NR_{L1}$; In another specific embodiment, $L_1$ is $C(R_{L1})_2$.

In a specific embodiment, $L_2$ is a bond; In another specific embodiment, $L_2$ is O; In another specific embodiment, $L_2$ is S; In another specific embodiment, $L_2$ is $NR_{L2}$; In another specific embodiment, $L_2$ is $C(R_{L2})_2$.

In another specific embodiment, $L_1$ is selected from bond, O, S, NH, and $CH_2$, and $L_2$ is selected from bond, O, S, NH, and $CH_2$; In another specific embodiment, $L_1$ is selected from O, S, NH, and $CH_2$, and $L_2$ is selected from bond, O, S, and NH; In another specific embodiment, $L_1$ is selected from NH and $CH_2$, and $L_2$ is NH.

$Y_1$, $Y_2$, $Y_3$ and $Y_4$

In a specific embodiment, $Y_1$, $Y_2$, $Y_3$ and $Y_4$ are each independently selected from $CR_Y$ and N; In another specific embodiment, $Y_1$, $Y_2$, $Y_3$ and $Y_4$ are $CR_Y$; In another specific embodiment, $Y_1$, $Y_2$, and $Y_3$ are $CR_Y$, and $Y_4$ is N; In another specific embodiment, $Y_2$ and $Y_3$ are $CR_Y$, and $Y_1$ and $Y_4$ are N.

In a specific embodiment,

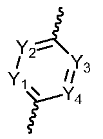

is selected from

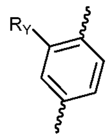, 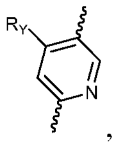, and 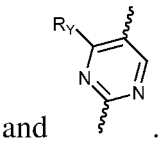.

$R_Y$

In a specific embodiment, $R_Y$ is selected from H, D, -OH, halogen, -CN, $-NO_2$, $-R_a$, - $C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)OR_a$, and $-OC(O)NR_bR_c$.
In another specific embodiment, $R_Y$ is selected from $C_{1-6}$ alkyl, $C_{1-6}$ haloalkyl, $C_{3-7}$ cycloalkyl, 3- to 7-membered heterocyclyl, $C_{6-10}$ aryl and 5- to 10-membered heteroaryl groups, wherein the groups are optionally substituted with one or more R; In another specific embodiment, $R_Y$ is selected from $C_{1-6}$ alkyl, $C_{1-6}$ haloalkyl, $C_{3-7}$ cycloalkyl, and 3- to 7-membered heterocyclyl groups, wherein the groups are optionally substituted with one or more R; In another specific embodiment, $R_Y$ is selected from methyl, ethyl, isopropyl, cyclopropyl, cyclopentyl, pyrrolidinyl and piperidinyl groups, wherein the groups are optionally substituted with one or more R; In another specific embodiment, each group in the definition of $R_Y$ is optionally substituted with one or more D until fully deuterated.

$Z_1$, $Z_2$, $Z_3$, $Z_4$, $Z_5$, and $Z_6$

In a specific embodiment, $Z_1$ is N; In another specific embodiment, $Z_1$ is CRzi; In another specific embodiment, $Z_1$ is CH.
In a specific embodiment, $Z_2$ is N; In another specific embodiment, $Z_2$ is $CR_{Z2}$; In another specific embodiment, $Z_2$ is CH.
In a specific embodiment, $Z_3$ is N atom; In another specific embodiment, $Z_3$ is C atom.
In a specific embodiment, $Z_4$ is N atom; In another specific embodiment, $Z_4$ is C atom.
In a specific embodiment, $Z_5$ is N atom, which is optionally substituted with $R_{Z5}$; In another specific embodiment, $Z_5$ is C atom, which is optionally substituted with $R_{Z5}$.
In a specific embodiment, $Z_6$ is N atom, which is optionally substituted with Rz6; In another specific embodiment, $Z_6$ is C atom, which is optionally substituted with Rz6. In a specific embodiment, Rz6 is $C_{1-6}$ alkyl, or $C_{1-6}$ haloalkyl; In another specific embodiment, Rz6 is $C_{1-6}$ alkyl; In another specific embodiment, Rz6 is isopropyl.
In another specific embodiment,

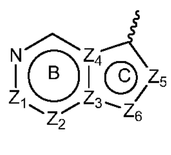

is

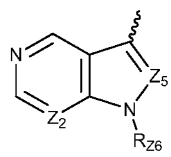;

In another specific embodiment,

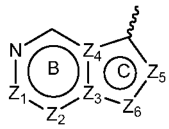

is selected from

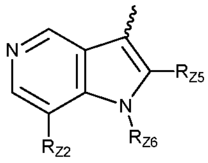
,
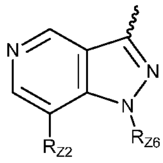
,
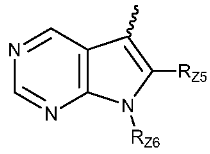

and

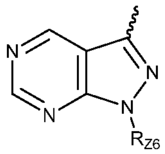
;

In another specific embodiment,

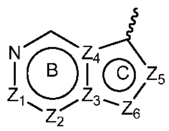

is selected from

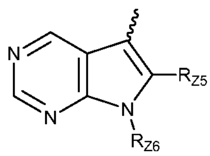
and
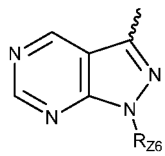
.

In another specific embodiment,

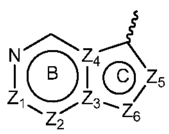

is

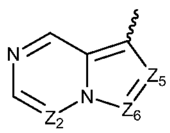
;

In another specific embodiment,

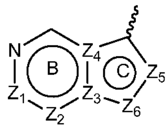

is selected from

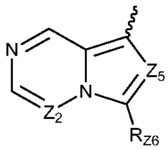 and 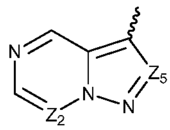 ;

In another specific embodiment,

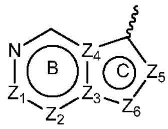

is selected from

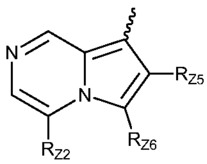 , 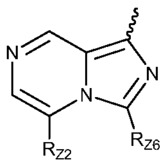 , 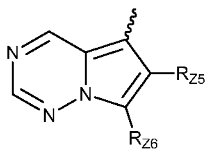 ,

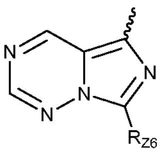 , 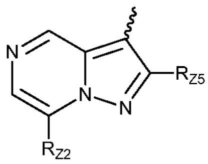 , 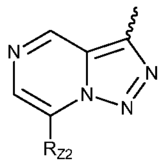 , 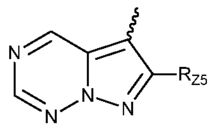 and 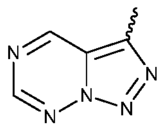 ;

In another specific embodiment,

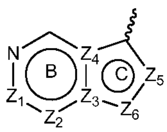

is selected from

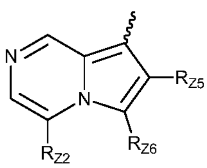 , 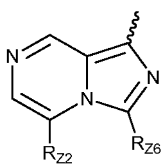 ,

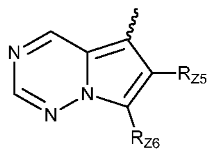 and 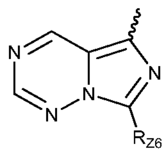 ;

In another specific embodiment,

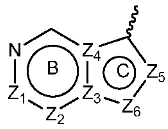

is selected from

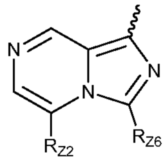 and 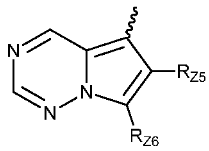 .

$R_{n1}$ and $R_{n2}$

In a specific embodiment, $R_{n1}$ and $R_{n2}$ are each independently H; In another specific embodiment, $R_{n1}$ and $R_{n2}$ are each independently D; In another specific embodiment, $R_{n1}$ and $R_{n2}$ are each independently $C_{1-6}$ alkyl; In another specific embodiment, $R_{n1}$ and $R_{n2}$ are each independently $C_{1-6}$ haloalkyl; In another specific embodiment, $R_{n1}$ and $R_{n2}$ are each independently $C_{2-6}$ alkenyl; In another specific embodiment, $R_{n1}$ and $R_{n2}$ are each independently $C_{2-6}$ alkynyl.

Any technical solution or any combination thereof in any above specific embodiment may be combined with any technical solution or any combination thereof in other specific embodiment. For example, any technical solution or any combination thereof of ring A may be combined with any technical solution or any combination thereof of $R_1$, $R_2$, m, $L_1$, $L_2$, $Y_1$ to $Y_4$, $R_{n1}$, $R_{n2}$, and $Z_1$ to $Z_6$. The present disclosure is intended to include all the combinations of these technical solutions, which are not listed one by one due to space limitation.

In a more specific embodiment, the present disclosure relates to a compound of formula (II), or a tautomer, stereoisomer, prodrug, crystal form, pharmaceutically acceptable salt, hydrate or solvate thereof:

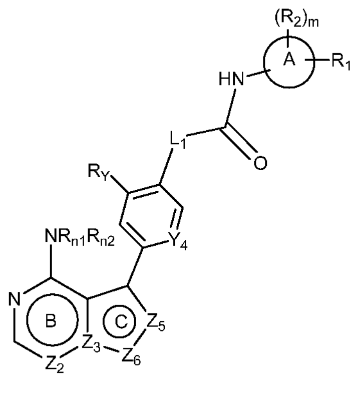

(II)

wherein

Ring A is pyrrolyl, pyrazolyl, imidazolyl, furyl, oxazolyl, isoxazolyl, thienyl, thiazolyl or isothiazolyl;

$R_1$ is $-C(R_{a1})(R_{a2})(R_{a3})$, wherein $R_{a1}$, $R_{a2}$ and Ra3 are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, Ra2 and $R_{a3}$ are independently $C_{1-4}$ alkyl or $C_{1-4}$ haloalkyl, wherein the $C_{1-4}$ alkyl and $C_{1-4}$ haloalkyl are optionally substituted

with one or more R; alternatively, $R_{a1}$, Ra2 and $R_{a3}$ are independently methyl or halomethyl; alternatively, at least one of $R_{a1}$, Ra2 and Ra3 is haloalkyl;

$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;

m is selected from 0, 1, 2, and 3;

$L_1$ is O, S, NH, ND, CHD, $CD_2$, or $CH_2$;

$R_Y$ is H, D, or halogen, alternatively fluorine;

$Y_4$ is CH, CD or N;

$Z_2$ is N or $CR_{Z2}$, wherein $R_{Z2}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;

$Z_3$ is N or C;

$Z_5$ is N, $NR_{Z5}$, or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;

$Z_6$ is $NR_{Z6}$ or $CR_{Z6}$, wherein Rz6 is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;

$R_{n1}$ and $R_{n2}$ are each independently selected from H, D, $C_{1-6}$ alkyl, $C_{1-6}$ haloalkyl, $C_{2-6}$ alkenyl and $C_{2-6}$ alkynyl;

each R is independently selected from H, D, -OH, $-NH_2$, halogen, -CN, $C_{1-6}$ alkyl, and $C_{1-6}$ haloalkyl.

In another embodiment, at most one of $Z_5$ and $Z_6$ is a substituted or unsubstituted carbon atom.

In another more specific embodiment, the present disclosure relates to a compound of formula (II-1), or a tautomer, stereoisomer, prodrug, crystal form, pharmaceutically acceptable salt, hydrate or solvate thereof:

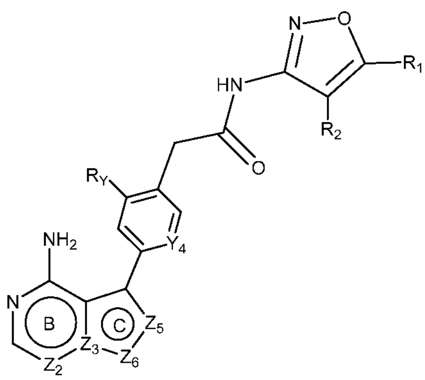

(II-1)

wherein

$R_1$ is $-C(R_{a1})(R_{a2})(R_{a3})$, wherein $R_{a1}$, $R_{a2}$ and Ra3 are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, Ra2 and Ra3 are independently $C_{1-4}$ alkyl or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, Ra2 and Ra3 is haloalkyl;

$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;

$R_Y$ is H, D, or halogen, alternatively fluorine;

$Y_4$ is CH, CD or N;

$Z_2$ is N or $CR_{Z2}$, wherein $R_{Z2}$ is H, D, halogen, $C_{1-4}$ alkyl, or $C_{1-4}$ haloalkyl, wherein the $C_{1-4}$ alkyl and $C_{1-4}$ haloalkyl are optionally substituted with one or more R;

$Z_3$ is N or C;

$Z_5$ is N, $NR_{Z5}$, or $CR_{Z5}$, wherein $R_{Z5}$ is H, D, halogen, $C_{1-4}$ alkyl, or $C_{1-4}$ haloalkyl, wherein the $C_{1-4}$ alkyl and $C_{1-4}$ haloalkyl are optionally substituted with one or more R;

$Z_6$ is $NR_{Z6}$ or CRz6, wherein Rz6 is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;

each R is independently selected from H, D, -OH, $-NH_2$, halogen, -CN, $C_{1-6}$ alkyl, and $C_{1-6}$ haloalkyl.

In another embodiment, at most one of $Z_5$ and $Z_6$ is a substituted or unsubstituted carbon atom.

In another more specific embodiment, the present disclosure relates to a compound of formula (II-2), or a

tautomer, stereoisomer, prodrug, crystal form, pharmaceutically acceptable salt, hydrate or solvate thereof:

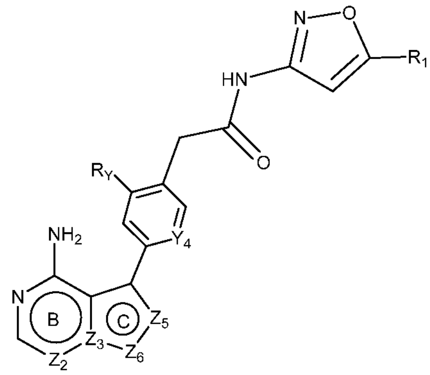

(II-2)

wherein

$R_1$ is -C($R_{a1}$)($R_{a2}$)($R_{a3}$), wherein $R_{a1}$, $R_{a2}$ and Ra3 are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, Ra2 and $R_{a3}$ are independently $C_{1-4}$ alkyl or $C_{1-4}$ haloalkyl, wherein the $C_{1-4}$ alkyl and $C_{1-4}$ haloalkyl are optionally substituted with one or more R; alternatively, $R_{a1}$, Ra2 and $R_{a3}$ are independently methyl or halomethyl; alternatively, at least one of $R_{a1}$, Ra2 and Ra3 is haloalkyl;
$R_Y$ is H, D, or halogen, alternatively fluorine;
$Y_4$ is CH, CD or N;
$Z_2$ is N, CH or CD;
$Z_3$ is N or C;
$Z_5$ is N, NH, CH or CD;
$Z_6$ is $NR_{Z6}$ or CRz6, wherein Rz6 is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;
each R is independently selected from H, D, -OH, $-NH_2$, halogen, -CN, $C_{1-6}$ alkyl, or $C_{1-6}$ haloalkyl.

In another embodiment, at most one of $Z_5$ and $Z_6$ is a substituted or unsubstituted carbon atom.

In another more specific embodiment, the present disclosure relates to a compound of formula (II-3), or a tautomer, stereoisomer, prodrug, crystal form, pharmaceutically acceptable salt, hydrate or solvate thereof:

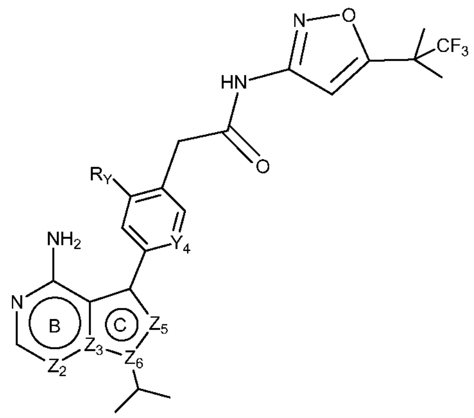

(II-3)

wherein

Ry is H, D, or halogen, alternatively fluorine;
$Y_4$ is CH, CD or N;
$Z_2$ is N, CH, or CD;
$Z_3$ is N or C;
$Z_5$ is N, CH or CD;

$Z_6$ is N or C.

In another embodiment, at most one of $Z_5$ and $Z_6$ is a substituted or unsubstituted carbon atom.

In another more specific embodiment, the present disclosure relates to a compound of formula (II-4-1) or formula (II-4-2), or a tautomer, stereoisomer, prodrug, crystal form, pharmaceutically acceptable salt, hydrate or solvate thereof:

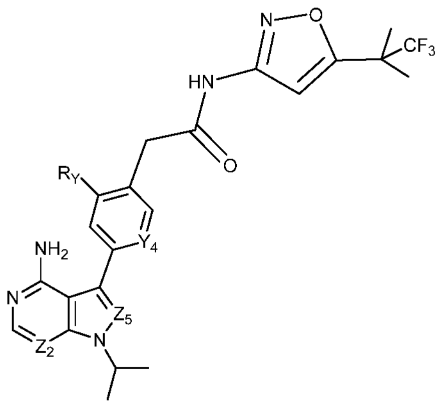

(II-4-1) or

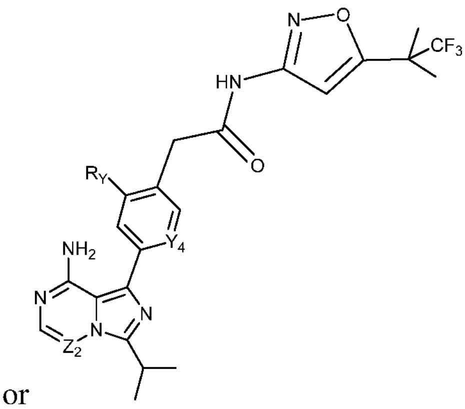

(II-4-2)

wherein

$R_Y$ is H or halogen, alternatively fluorine;
$Y_4$ is CH or N;
$Z_2$ is CH or N;
$Z_5$ is CH or N.

In another more specific embodiment, the present disclosure relates to a compound of formula (III), or a tautomer, stereoisomer, prodrug, crystal form, pharmaceutically acceptable salt, hydrate or solvate thereof:

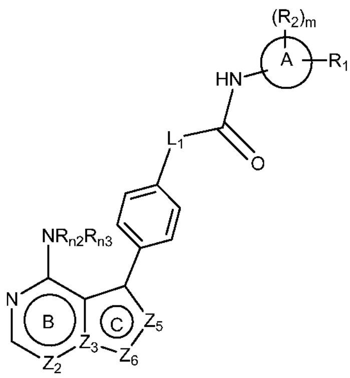

(III)

wherein

Ring A is pyrrolyl, pyrazolyl, imidazolyl, furyl, oxazolyl, isoxazolyl, thienyl, thiazolyl or isothiazolyl;
$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
m is selected from 0, 1, 2, and 3;
$L_1$ is O, S, NH, ND, CHD, $CD_2$, or $CH_2$;
$Z_2$ is N or $CR_{Z2}$, wherein $R_{Z2}$ is H, D, halogen, $C_{1-6}$ alkyl, or $C_{1-6}$ haloalkyl, wherein the $C_{1-6}$ alkyl and $C_{1-6}$ haloalkyl

are optionally substituted with one or more R;

$Z_3$ is N or C;

$Z_5$ is N, $NR_{Z5}$, or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;

$Z_6$ is NRz6or CRz6, wherein Rz6 is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;

$R_{n1}$ and $R_{n2}$ are each independently selected from H, D, $C_{1-6}$ alkyl, $C_{1-6}$ haloalkyl, $C_{2-6}$ alkenyl and $C_{2-6}$ alkynyl;

each R is independently selected from H, D, -OH, $-NH_2$, halogen, -CN, $C_{1-6}$ alkyl, and $C_{1-6}$ haloalkyl;

wherein at most one of $Z_5$ and $Z_6$ is a substituted or unsubstituted nitrogen atom.

In another more specific embodiment, the present disclosure relates to a compound of formula (III-1), or a tautomer, stereoisomer, prodrug, crystal form, pharmaceutically acceptable salt, hydrate or solvate thereof:

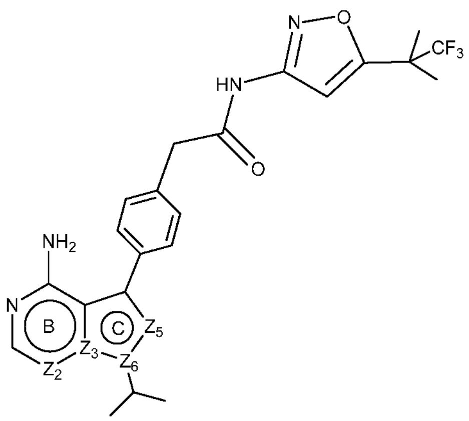

(III-1)

wherein

$Z_2$ is N or CH;

$Z_3$ is N or C;

$Z_5$ is N, NH, or CH;

$Z_6$ is Nor C;

wherein at most one of $Z_5$ and Z6 is a nitrogen atom.

In another more specific embodiment, the present disclosure relates to a compound of formula (IV), or a tautomer, stereoisomer, prodrug, crystal form, pharmaceutically acceptable salt, hydrate or solvate thereof:

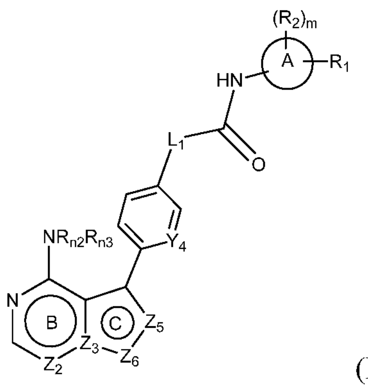

(IV)

wherein

Ring A is pyrrolyl, pyrazolyl, imidazolyl, furyl, oxazolyl, isoxazolyl, thienyl, thiazolyl or isothiazolyl;

$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and

$R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;

[00192] $R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;

m is selected from 0, 1, 2, or 3;

$L_1$ is O, S, NH, ND, CHD, $CD_2$, or $CH_2$;

$Y_4$ is CH, CD or N;

$Z_2$ is N or $CR_{Z2}$, wherein $R_{Z2}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;

$Z_3$ is N or C;

$Z_5$ is N, $NR_{Z5}$, or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;

$Z_6$ is $NR_{Z6}$ or $CR_{Z6}$, wherein $R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;

$R_{n1}$ and $R_{n2}$ are each independently selected from H, D, $C_{1-6}$ alkyl, $C_{1-6}$ haloalkyl, $C_{2-6}$ alkenyl or $C_{2-6}$ alkynyl;

each R is independently selected from H, D, -OH, $-NH_2$, halogen, -CN, $C_{1-6}$ alkyl, or $C_{1-6}$ haloalkyl;

wherein at most one of $Z_5$ and $Z_6$ is a substituted or unsubstituted carbon atom; and when both of $Z_5$ and $Z_6$ are a substituted or unsubstituted nitrogen atom, $Y_4$ is N.

In another embodiment, only one of $Z_5$ and $Z_6$ is a substituted or unsubstituted carbon atom.

In another more specific embodiment, the present disclosure relates to a compound of formula (IV-1), or a tautomer, stereoisomer, prodrug, crystal form, pharmaceutically acceptable salt, hydrate or solvate thereof:

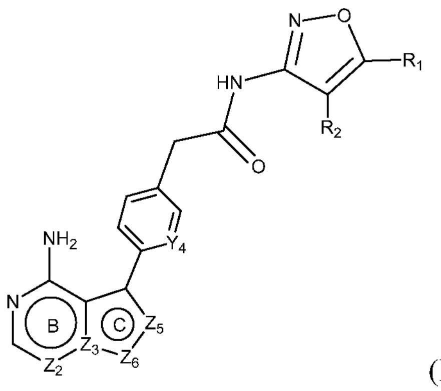

(IV-1)

wherein

$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;

$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;

$Y_4$ is CH, CD, or N;

$Z_2$ is N or $CR_{Z2}$, wherein $R_{Z2}$ is H, D, halogen, $C_{1-4}$ alkyl, or $C_{1-4}$ haloalkyl, wherein the $C_{1-4}$ alkyl and $C_{1-4}$ haloalkyl are optionally substituted with one or more R;

$Z_3$ is N or C;

$Z_5$ is N, $NR_{Z5}$, or $CR_{Z5}$, wherein $R_{Z5}$ is H, D, halogen, $C_{1-4}$ alkyl, or $C_{1-4}$ haloalkyl, wherein the $C_{1-4}$ alkyl and $C_{1-4}$ haloalkyl are optionally substituted with one or more R;

$Z_6$ is $NR_{Z6}$ or $CR_{Z6}$, wherein $R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;

each R is independently selected from H, D, -OH, $-NH_2$, halogen, -CN, $C_{1-6}$ alkyl, or $C_{1-6}$ haloalkyl;

wherein at most one of $Z_5$ and $Z_6$ is a substituted or unsubstituted carbon atom; and when both of $Z_5$ and $Z_6$ are a substituted or unsubstituted nitrogen atom, $Y_4$ is N.

In another embodiment, only one of $Z_5$ and $Z_6$ is a substituted or unsubstituted carbon atom.

In another more specific embodiment, the present disclosure relates to a compound of formula (IV-2), or a tautomer, stereoisomer, prodrug, crystal form, pharmaceutically acceptable salt, hydrate or solvate thereof:

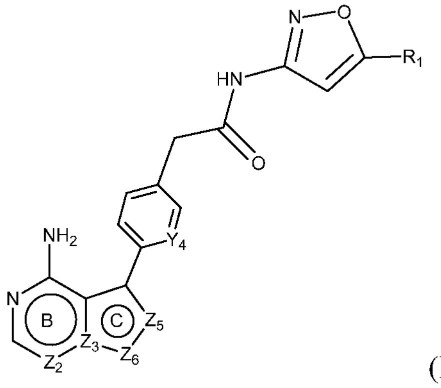

(IV-2)

wherein

$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;

$Y_4$ is CH, CD, or N;

$Z_2$ is N or CH;

$Z_3$ is N or C;

$Z_5$ is N, NH, or CH;

$Z_6$ is $NR_{Z6}$ or $CR_{Z6}$, wherein $R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;

each R is independently selected from H, D, -OH, $-NH_2$, halogen, -CN, $C_{1-6}$ alkyl, or $C_{1-6}$ haloalkyl;

wherein at most one of $Z_5$ and $Z_6$ is a substituted or unsubstituted carbon atom; and when both of $Z_5$ and $Z_6$ are a substituted or unsubstituted nitrogen atom, $Y_4$ is N.

In another embodiment, only one of $Z_5$ and $Z_6$ is a substituted or unsubstituted carbon atom.

In another more specific embodiment, the present disclosure relates to a compound of formula (IV-3), or a tautomer, stereoisomer, prodrug, crystal form, pharmaceutically acceptable salt, hydrate or solvate thereof:

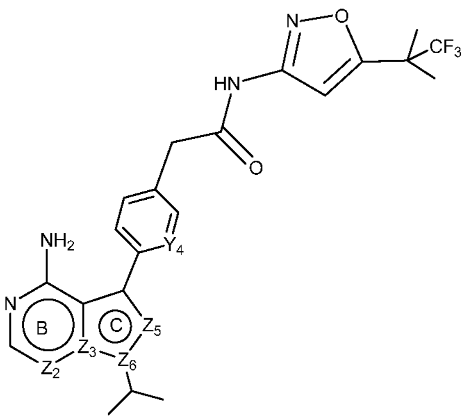

(IV-3)

wherein

$Y_4$ is CH, CD or N;

$Z_2$ is N or CH;

$Z_3$ is N or C;
$Z_5$ is N, NH, or CH;
$Z_6$ is Nor C;
wherein at most one of $Z_5$ and $Z_6$ is a substituted or unsubstituted carbon atom; and when both of $Z_5$ and $Z_6$ are a substituted or unsubstituted nitrogen atom, $Y_4$ is N.

In another embodiment, only one of $Z_5$ and $Z_6$ is a substituted or unsubstituted carbon atom.

In another more specific embodiment, the present disclosure relates to a compound of formula (V), or a tautomer, stereoisomer, prodrug, crystal form, pharmaceutically acceptable salt, hydrate or solvate thereof:

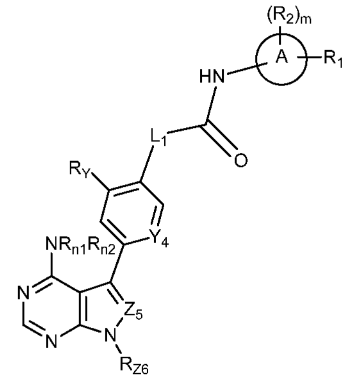

(V)

wherein

Ring A is pyrrolyl, pyrazolyl, imidazolyl, furyl, oxazolyl, isoxazolyl, thienyl, thiazolyl or isothiazolyl;
$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
m is selected from 0, 1, 2, and 3;
$L_1$ is O, S, NH, ND, CHD, $CD_2$, or $CH_2$;
$Y_4$ is N or $CR_Y$,
$R_Y$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_Y$ is H or halogen; still alternatively fluorine;
$R_{n1}$ and $R_{n2}$ are each independently selected from H, D, $C_{1-6}$ alkyl, $C_{1-6}$ haloalkyl, $C_{2-6}$ alkenyl and $C_{2-6}$ alkynyl;
$R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;
$Z_5$ is N or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
each R is independently selected from H, D, -OH, $-NH_2$, halogen, -CN, $C_{1-6}$ alkyl, and $C_{1-6}$ haloalkyl.

In another embodiment, $Z_5$ is N.

In another more specific embodiment, the present disclosure relates to a compound of formula (V-1) or (V-1'), or a tautomer, stereoisomer, prodrug, crystal form, pharmaceutically acceptable salt, hydrate or solvate thereof:

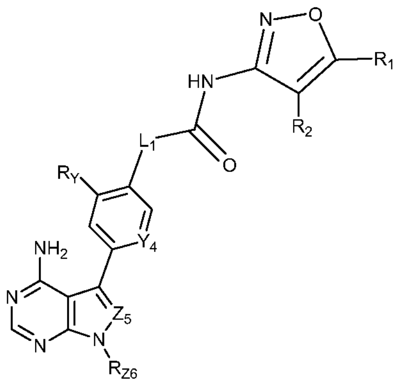
(V-1) or
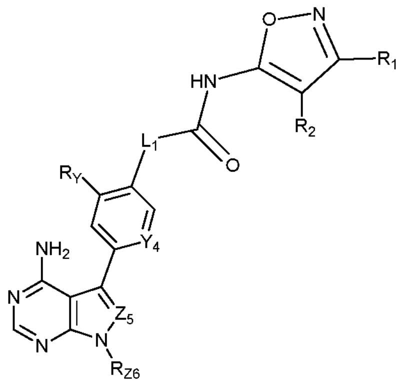
(V-1')

wherein

$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
$L_1$ is O, S, NH, ND, CHD, $CD_2$, or $CH_2$;
$Y_4$ is N or $CR_Y$,
$R_Y$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_Y$ is H or halogen; still alternatively fluorine;
$R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;
$Z_5$ is N or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
each R is independently selected from H, D, -OH, $-NH_2$, halogen, -CN, $C_{1-6}$ alkyl, and $C_{1-6}$ haloalkyl.

In another embodiment, $Z_5$ is N.
In another more specific embodiment, the present disclosure relates to a compound of formula (V-2) or (V-2'), or a tautomer, stereoisomer, prodrug, crystal form, pharmaceutically acceptable salt, hydrate or solvate thereof:

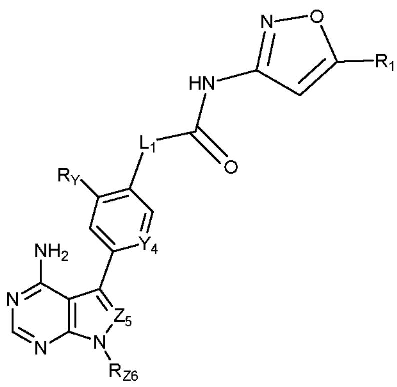
(V-2) or
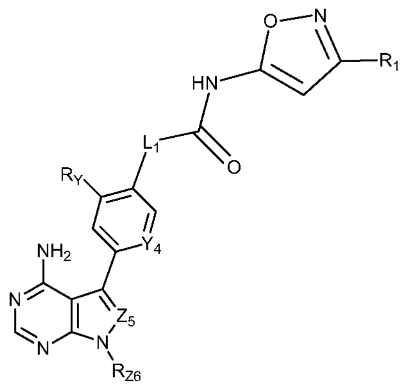
(V-2')

wherein

$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;

$L_1$ is NH, ND, CHD, $CD_2$, or $CH_2$;
$Y_4$ is N, CD, or CH;
$R_Y$ is H, D, or halogen, alternatively fluorine;
$R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;
$Z_5$ is N, CD, or CH;
each R is independently selected from H, D, -OH, $-NH_2$, halogen, -CN, $C_{1-6}$ alkyl, and $C_{1-6}$ haloalkyl.

In another embodiment, $Z_5$ is N.
In another more specific embodiment, the present disclosure relates to a compound of formula (V-3) or (V-3'), or a tautomer, stereoisomer, prodrug, crystal form, pharmaceutically acceptable salt, hydrate or solvate thereof:

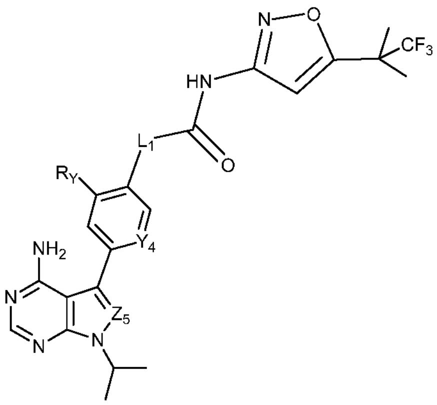
(V-3) or
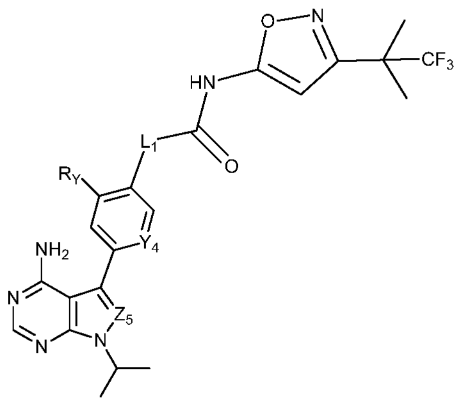
(V-3')

wherein

$L_1$ is NH or $CH_2$;
$Y_4$ is N or CH;
Ry is H or halogen, alternatively fluorine;
$Z_5$ is N or CH.

In another embodiment, $Z_5$ is N.
In another more specific embodiment, the present disclosure relates to a compound of formula (VI), or a tautomer, stereoisomer, prodrug, crystal form, pharmaceutically acceptable salt, hydrate or solvate thereof:

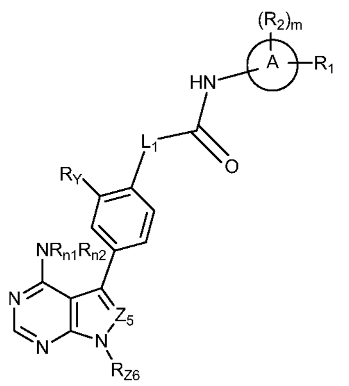
(VI)

wherein

Ring A is pyrrolyl, pyrazolyl, imidazolyl, furyl, oxazolyl, isoxazolyl, thienyl, thiazolyl or isothiazolyl;
$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted

with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
m is selected from 0, 1, 2, and 3;
$L_1$ is O, S, NH, ND, CHD, $CD_2$, or $CH_2$;
$R_Y$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_Y$ is H or halogen; still alternatively fluorine;
$R_{n1}$ and $R_{n2}$ are each independently selected from H, D, $C_{1-6}$ alkyl, $C_{1-6}$ haloalkyl, $C_{2-6}$ alkenyl and $C_{2-6}$ alkynyl;
Rz6 is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;
$Z_5$ is N or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
each R is independently selected from H, D, -OH, $-NH_2$, halogen, -CN, $C_{1-6}$ alkyl, and $C_{1-6}$ haloalkyl.

In another embodiment, $Z_5$ is N.

In another more specific embodiment, the present disclosure relates to a compound of formula (VI-1), or a tautomer, stereoisomer, prodrug, crystal form, pharmaceutically acceptable salt, hydrate or solvate thereof:

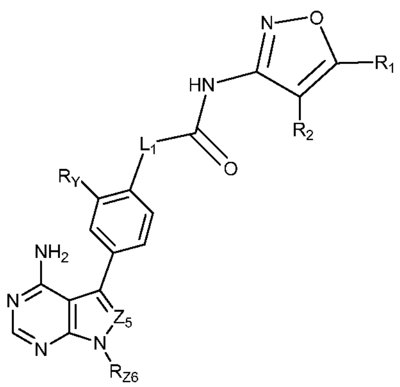

(VI-1)

wherein

$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
$L_1$ is O, S, NH, ND, CHD, $CD_2$, or $CH_2$;
$R_Y$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_Y$ is H or halogen; still alternatively fluorine;
Rz6 is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;
$Z_5$ is N or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
each R is independently selected from H, D, -OH, $-NH_2$, halogen, -CN, $C_{1-6}$ alkyl, and $C_{1-6}$ haloalkyl.

In another embodiment, $Z_5$ is N.

In another more specific embodiment, the present disclosure relates to a compound of formula (VI-2), or a tautomer, stereoisomer, prodrug, crystal form, pharmaceutically acceptable salt, hydrate or solvate thereof:

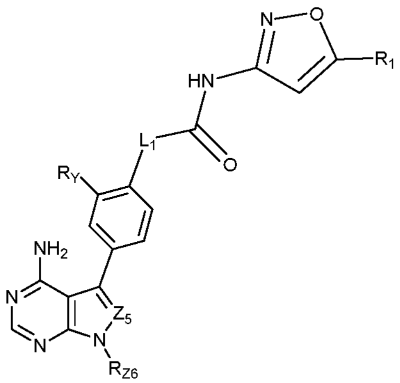

(VI-2)

wherein

$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$L_1$ is NH, ND, CHD, $CD_2$, or $CH_2$;
$R_Y$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_Y$ is H or halogen; still alternatively fluorine;
$R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, Rz6 is $C_{1-4}$ alkyl, or $C_{1-4}$ haloalkyl;
$Z_5$ is N, CD, or CH;
each R is independently selected from H, D, -OH, $-NH_2$, halogen, -CN, $C_{1-6}$ alkyl, and $C_{1-6}$ haloalkyl.

In another embodiment, $Z_5$ is N.
In another more specific embodiment, the present disclosure relates to a compound of formula (VI-3), or a tautomer, stereoisomer, prodrug, crystal form, pharmaceutically acceptable salt, hydrate or solvate thereof:

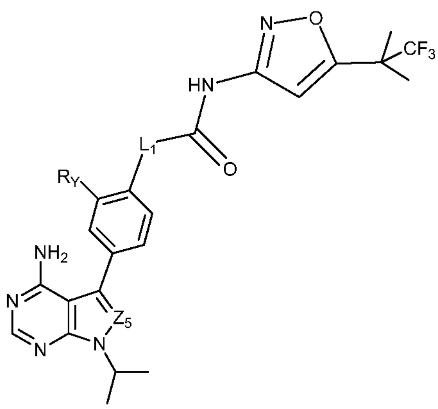

(VI-3)

wherein

$L_1$ is NH or $CH_2$;
$R_Y$ is H or halogen, alternatively fluorine;
$Z_5$ is N or CH.

In another embodiment, $Z_5$ is N.
In another more specific embodiment, the present disclosure relates to a compound of formula (II-3), or a tautomer, stereoisomer, prodrug, crystal form, pharmaceutically acceptable salt, hydrate or solvate thereof:
In another more specific embodiment, the present disclosure relates to the following compounds, or a tautomer,

stereoisomer, prodrug, crystal form, pharmaceutically acceptable salt, hydrate or solvate thereof:

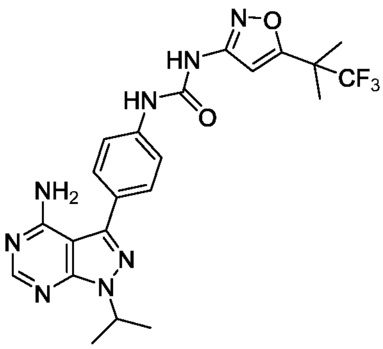
,
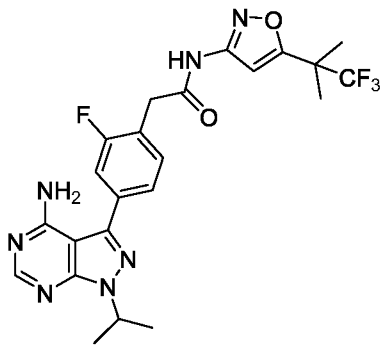
,
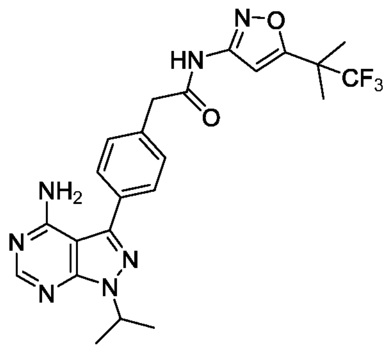
,
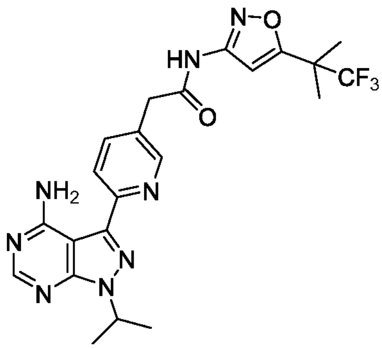
,
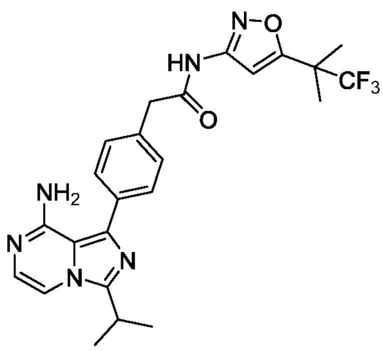
,
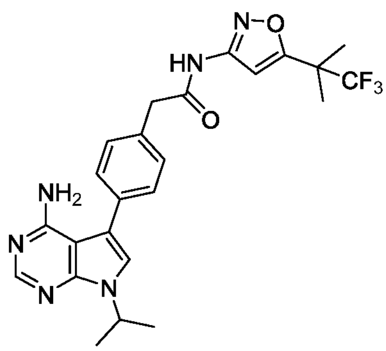
,
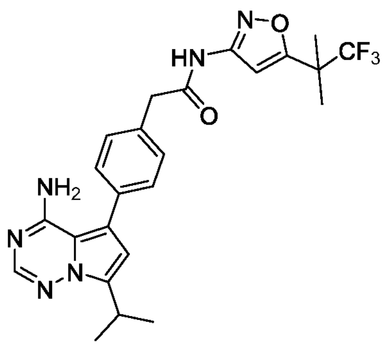
,
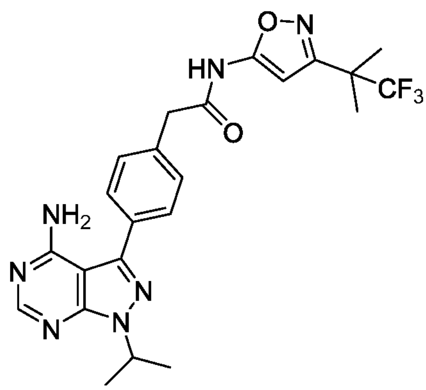
,
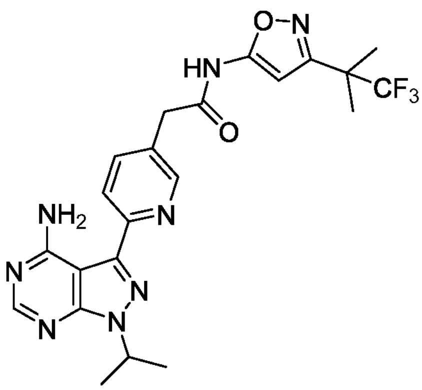
or
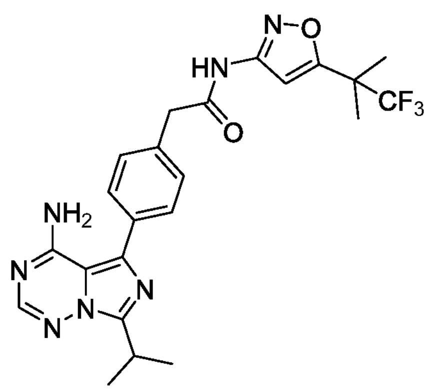
.

The compounds disclosed herein may include one or more asymmetric centers, and thus may exist in a variety of stereoisomeric forms, for example, enantiomers and/or diastereomers. For example, the compounds disclosed herein

may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

"Tautomer" refers to an isomer in which one functional group in a compound changes its structure into another functional group, wherein the compound and the isomer can quickly convert between each other, thus being in dynamic equilibrium; this two isomers are called tautomers.

It will be understood by those skilled in the art that the organic compounds can form complexes with solvents in which they are reacted or from which they are precipitated or crystallized. These complexes are known as "solvates." Where the solvent is water, the complex is known as "hydrate." The present disclosure encompasses all solvates of the compounds disclosed herein.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula $R \cdot x\ H_2O$, wherein R is the compound, and x is a number greater than 0. Given compounds can form more than one type of hydrates, including, for example, monohydrates (x is 1), lower hydrates (x is a number greater than 0 and smaller than 1, for example, hemihydrates ($R \cdot 0.5\ H_2O$)) and polyhydrates (x is a number greater than 1, for example, dihydrates ($R \cdot 2\ H_2O$) and hexahydrates ($R \cdot 6\ H_2O$)).

Compounds disclosed herein may be in an amorphous or crystalline form (crystal form or polymorph). Furthermore, the compounds disclosed herein may exist in one or more crystalline forms. Therefore, the present disclosure includes all amorphous or crystalline forms of the compounds disclosed herein within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes, which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{3S}S$, $^{18}F$ and $^{36}Cl$, respectively. Compounds disclosed herein that comprise the above isotopes and/or other isotopes of other atoms, prodrugs thereof and pharmaceutically acceptable salts of said compounds or prodrugs all are within the scope of the present disclosure. Certain isotope-labeled compounds disclosed herein, such as those incorporating radioactive isotopes (e.g., $^{3}H$ and $^{14}C$), can be used for the measurement of the distribution of drug and/or substrate in tissue. Tritium, which is $^{3}H$ and carbon-14, which is $^{14}C$ isotope, are alternative, because they are easy to prepare and detect. Furthermore, replaced by heavier isotopes, such as deuterium, which is $^{2}H$, may provide therapeutic benefits due to the higher metabolic stability, such as prolonging the half-life *in vivo* or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted *in vivo* into an active form that has medical effects by, for example, hydrolysis in blood. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

The prodrugs are any covalently bonded compounds disclosed herein, which release the parent compound *in vivo* when the prodrug is administered to a patient. Prodrugs are typically prepared by modifying functional groups in such a way that the modifications can be cleaved either by routine manipulation or decompose *in vivo* to yield the parent compound. Prodrugs include, for example, compounds disclosed herein wherein the hydroxy, amino or sulfhydryl groups are bonded to any group that, when administered to a patient, cleaves to form the hydroxy, amino or sulfhydryl groups. Thus, representative examples of prodrugs include (but are not limited to) the acetate/acetamide, formate/formamide and benzoate/benzamide derivatives of the hydroxy, amino or sulfhydryl functional groups of the compounds of formula (I). Furthermore, in the case of carboxylic acid (-COOH), esters such as methyl esters and ethyl esters, etc. can be employed. The ester itself may be active in their own and/or hydrolyzable under *in vivo* conditions in the human body. Suitable pharmaceutically acceptable *in vivo* hydrolysable ester groups include those groups that can readily break down in the human body to release the parent acids or salts thereof.

Pharmaceutical composition, formulation, and kit

In another aspect, the disclosure provides a pharmaceutical composition comprising a compound disclosed herein (also referred to as the "active ingredient") and a pharmaceutically acceptable excipient. In certain embodiments, the pharmaceutical composition comprises an effective amount of the active ingredient. In certain embodiments, the pharmaceutical composition comprises a therapeutically effective amount of the active ingredient. In certain embodiments, the pharmaceutical composition comprises a prophylactically effective amount of the active ingredient.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other materials) containing the compound disclosed herein or other therapeutic agents. In some embodiments, kits provided can also optionally include a third container containing a pharmaceutically acceptable excipient for diluting or suspending the compound disclosed herein and/or other therapeutic agent. In some embodiments, the compound disclosed herein provided in the first container and the other therapeutic agents provided in the second container is combined to form a unit dosage form.

The pharmaceutical composition provided by the present disclosure may be administered by a variety of routes including, but not limited to, oral administration, parenteral administration, inhalation administration, topical administration, rectal administration, nasal administration, oral administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the disorder disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc*., or may be continued indefinitely, for example, for the rest of the subject's life. In certain embodiments, the chronic administration is intended to provide a constant level of the compound in the blood, *e.g.*, within the therapeutic window over the extended period of time.

The pharmaceutical compositions of the present disclosure may be further delivered using a variety of dosing

methods. For example, in certain embodiments, the pharmaceutical composition may be given as a bolus, *e.g.,* in order to raise the concentration of the compound in the blood to an effective level rapidly. The placement of the bolus dose depends on the desired systemic levels of the active ingredient, *e.g.,* an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, *e.g*., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or alternatively from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with alternative doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, alternatively from about 0.1 to about 20% by weight, alternatively from about 0.1 to about 10% by weight, and yet alternatively from about 0.5 to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavours and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavouring agent such as peppermint, methyl salicylate, or orange flavouring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or Formulation. All such known transdermal formulations and ingredients are included within the scope provided herein.

The compounds provided herein can also be administered by a transdermal device. Accordingly, transdermal administration may be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

The compounds disclosed herein can also be administered in sustained release forms or from sustained release drug delivery systems. A description of representative sustained release materials may be found in Remington's Pharmaceutical Sciences.

The present disclosure also relates to the pharmaceutically acceptable formulations of a compound disclosed herein. In one embodiment, the formulation comprises water. In another embodiment, the formulation comprises a cyclodextrin derivative. The most common cyclodextrins are α-, β- and γ- cyclodextrins consisting of 6, 7 and 8 α-1,4-linked glucose units, respectively, optionally comprising one or more substituents on the linked sugar moieties, which

include, but are not limited to, methylated, hydroxyalkylated, acylated, and sulfoalkylether substitution. In certain embodiments, the cyclodextrin is a sulfoalkyl ether β-cyclodextrin, *e.g.*, for example, sulfobutyl ether β-cyclodextrin, also known as Captisol. See, *e.g.,* U.S. 5,376,645. In certain embodiments, the formulation comprises hexapropyl-β-cyclodextrin (e.g., 10-50% in water).

Indication

Provided herein is a method of treating, preventing or ameliorating diseases or disorders modulated or otherwise affected by one or more of RET, Trk, FLT3, c-Kit, PDGFR, and VEGFR kinases (including one or more of wild-type and/or mutant RET, Trk, FLT3, c-Kit, PDGFR, and VEGFR kinases), or one or more symptoms or causes thereof.

The present disclosure provides a method of treating diseases mediated by protein kinases in a subject, comprising administering to the subject a compound disclosed herein, or a pharmaceutically acceptable salt, hydrate or solvate thereof, or a pharmaceutical composition disclosed herein.

As used herein, the term "wild-type" refers to the most common genes or alleles found in organisms. In some specific embodiments, "wild-type" refers to a gene or allele that does not have a mutation.

As used herein, the term "cancer" refers to the abnormal growth of cells that proliferate in uncontrolled way and metastasize in some circumstances. Types of cancer include, but are not limited to, solid tumors, such as bladder tumor, intestine tumor, brain tumor, breast tumor, endometrial tumor, heart tumor, kidney tumor, lung tumor, lymphoid tissue tumor (lymphoma), ovary tumor, pancreas tumor, or other endocrine organ (thyroid) tumor, prostate tumor, skin tumor (melanoma), or hematological tumor (e.g., leukemia) and so on.

RET

In a specific embodiment, compounds disclosed herein are inhibitors of a RET kinase, and can be used for treating, preventing or ameliorating diseases or disorders that are modulated or otherwise affected by one or more of wild-type RET and RET kinase domain mutants, or one or more symptoms or causes thereof. Such diseases or disorders include, but are not limited to, proliferative conditions (e.g., cancers, including hematological cancers and solid tumors) and gastrointestinal diseases (IBS) that can be treated, prevented or controlled by modulating various activities of kinases (including dimerization, ligand binding, and phosphotransferase activities) or by modulating the expression of kinases.

As used herein, the term "RET kinase domain mutants" refers to one or more mutants of RET kinase domain, or alternatively, refers to RET (protein itself becomes the "RET kinase domain mutant") comprising one or more of the mutations. Mutations in the RET kinase domain can be insertions, deletions, or point mutations. In a specific embodiment, mutations of RET kinase domain comprise at least one point mutation in the RET kinase domain. In another specific embodiment, mutations of RET kinase domain comprise at least one point mutation in the RET kinase domain. In another specific embodiment, the point mutation in the RET kinase domain is selected from S32L, D34S, L40P, P64L, R67H, $R_1$14H, V145G, V292M, G321R, R330Q, T338I, R360W, F393L, $A_5$10V, E511K, C515S, C531R, G533C, G533S, G550E, V591I, G593E, I602V, R600Q, K603Q, K603E, Y606C, C609Y, C609S, C609G, C609R, C609F, C609W, C611R, C611S, C611G, C611Y, C611F, C611W, C618S, C618Y, C618R, C618Y, C618G, C618F, C618W, F619F, C620S, C620W, C620R, C620G, C620L, C620Y, C620F, E623K, D624N, C630A, C630R, C630S, C630Y, C630F, D631N, D631Y, D631A, D631G, D631V, D631E, E632K, E632G, C634W, C634Y, C634S, C634R, C634F, C634G, C634L, C634A, C634T, R635G, T636P, T636M, $A_6$40G, $A_6$41S, $A_6$41T, V648I, S649L, $A_6$64D, H665Q, K666E, K666M, K666N, S686N, G691S, R694Q, M700L, V706M, V706A, E713K, G736R, G748C, $A_7$50P, S765P, P766S, P766M, E768Q, E768D, L769L, R770Q, D771N, N777S, V778I, Q781R, L790F, Y791F, V804L, V804M, V804E, E805K, Y806E, Y806F, Y806S, Y806G, Y806C, E818K, S819I, G823E, Y826M, R833C, P841L, P841P, E843D, R844W, R844Q, R844L, M848T, I852M, A866W, R873W, A876V, L881V, A883F, A883S, A883T, E884K, R886W, S891A, R897Q, D898V, E901K, S904F, S904C, K907E, K907M, R908K, G911D, R912P, R912Q, M918T, M918V, M918L, A919V, E921K, S922P, S922Y, T930M, F961L, R972G, R982C, M1009V, D1017N, V1041G, and M1064T. In another specific embodiment, the point mutation in RET kinase domain is selected from V804L, V804M, V804E, M918T, E805K, Y806C, Y806E, C634Y, and C634W. In another specific embodiment, RET kinase domain mutations further include RET gene fusion. In another specific embodiment, RET gene fusion is selected from BCR-RET, CLIP1-RET, KIF5B-RET, CCDC6-RET, $NCOA_4$-RET, TRIM33-RET, ERC1-RET, ELKS-RET, RET-ELKS, $FGFR_1$OP-RET, RET-MBD1, RET-RAB61P2, RET-PCM1, RET-$PPKAR_1$A, RET-TRIM24, RET-RFG9, RFP-RET, RET-$GOLGA_5$, HOOK3-RET, KTN1-RET, TRIM27-RET, AKAP13-RET, FKBP15-RET, SPECC1L-RET, $TBL1XR_1$/RET, CEP55-RET, CUX1-RET, $KIAA_1$468-RET, $PPKAR_1$A-RET, RFG8/RET, RET/RFG8, H4-RET, ACBD5-RET, PTCex9-RET, MYH13-RET, PIBF1-RET, $KIAA_1$217-RET, and MPRIP-RET; in another specific embodiment, RET gene fusion is selected from KIF5B-RET and CCDC6-RET; in another specific embodiment, point mutation in KIF5B-RET and CCDC6-RET kinase domains is selected from V804L, V804M, V804E, M918T, E805K, Y806C, Y806E, C634Y, C634W, and G810R.

TRK

In a specific embodiment, compounds disclosed herein are inhibitors of a Trk kinase, and can be used for treating, preventing or ameliorating diseases or disorders that are modulated or otherwise affected by one or more of wild-type Trk and Trk kinase domain mutants, or one or more symptoms or causes thereof. Such diseases or disorders include, but are not limited to, proliferative conditions (e.g., cancers, including hematological cancers and solid tumors), pain, inflammation, and certain infectious diseases that can be treated, prevented or controlled by modulating various activities of kinases (including dimerization, ligand binding, and phosphotransferase activities) or by modulating the expression of kinases. In a specific embodiment, the cancer may be selected from non-small cell lung cancer, papillary thyroid cancer, glioblastoma multiforme, acute myeloid leukemia, colorectal cancer, large cell neuroendocrine cancer, prostate cancer, colon cancer, acute myeloid leukemia, sarcoma, pediatric glioma, intrahepatic cholangiocarcinoma, hairy cell astrocytoma, low grade glioma, lung adenocarcinoma, salivary gland cancer, secretory breast cancer, fibrosarcoma, nephroma and breast cancer.

In a specific embodiment, the Trk kinase is selected from TrkA, TrkB, and TrkC.

Point mutations in NTRK1 gene, NTRK2 gene, and NTRK3 gene have been found in Trk inhibitor-resistant cancer cells. The point mutations in NTRK1/2/3 genes may produce TrkA/B/C proteins, including wild-type TrkA/B/C proteins whose amino acids have been substituted by different amino acids.

The compounds of the present disclosure may be used to treat diseases mediated by at least one point mutation in NTRK genes leading to the expression of Trk protein.

The compounds of the present disclosure may be used in the manufacture of medicaments for the treatment of diseases mediated by at least one point mutation in NTRK genes leading to the expression of Trk protein.

In another specific embodiment, at least one point mutation in NTRK genes that results in the expression of Trk protein (which includes mutations at one or more amino acid positions) may be selected from (i) at least one point mutation in NTRK1 gene, which results in expression of mutant TrkA protein at one or more amino acid positions selected from the group comprising: 517, 542, 568, 573, 589, 595, 599, 600, 602, 646, 656, 657, 667, and 676, and/or (ii) at least one point mutation in NTRK2 gene, which results in expression of mutant TrkB protein at one or more amino acid positions selected from the group comprising: 545, 570, 596, 601, 617, 623, 624, 628, 630, 672, 682, 683, 693, and 702, and/or (iii) at least one point mutation in NTRK3 gene, which results in expression of mutant TrkC protein at one or more amino acid positions selected from the group comprising: 545, 570, 596, 601, 617, 623, 624, 628, 630, 675, 685, 686, 696, and 705. In another specific embodiment, TrkA protein includes one or more amino acid substitutions of: G517R, A542V, V573M, F589L, F589C, G595S, G595R, D596V, D596V, F600L, F646V, C656Y, C656F, L657V, G667S, G667C, and Y676S. In another specific embodiment, TrkB protein includes one or more amino acid substitutions of: G545R, A570V, Q596E, Q596P, V601G, F617L, F617C, F617I, G623S, G623R, D624V, R630K, C682Y, C682F, L683V, G693S, and G713S. In another specific embodiment, TrkC protein includes one or more amino acid substitutions of: G545R, A570V, F617L, G623R, D624V, C685Y, C685F, L686V, and G696A.

FLT3

In a specific embodiment, compounds disclosed herein are inhibitors of a FLT3 kinase, and can be used for treating, preventing or ameliorating diseases or disorders that are modulated or otherwise affected by one or more of wild-type FLT3, FLT3-ITD, and FLT3 kinase domain mutants, or one or more symptoms or causes thereof. Such diseases or disorders include, but are not limited to, hematological cancers, including acute myeloid leukemia (AML), acute lymphoblastic leukemia (ALL), and myelodysplastic syndrome (MDS), that can be treated, prevented or controlled by modulating various activities of kinases (including dimerization, ligand binding, and phosphotransferase activities) or by modulating the expression of kinases, wherein the method comprises administering a therapeutically or prophylactically effective amount of a compound provided herein to a subject, for example human, in need of such treatment, prevention or control.

As used herein, the term "FLT3 kinase domain mutants" refers to one or more mutants of FLT3 kinase domain, or alternatively, refers to FLT3 (protein itself becomes the "FLT3 kinase domain mutant") comprising one or more of the mutations. Mutations in FLT3 kinase domain can be insertions, deletions, or point mutations. In a specific embodiment, mutations of FLT3 kinase domain comprise at least one point mutation in the FLT3 kinase domain. In another specific embodiment, mutations of FLT3 kinase domain comprise at least one point mutation in the FLT3 kinase domain. In another specific embodiment, the point mutation in FLT3 kinase domain is at position E608, N676, F691, C828, D835, D839, N841, Y842, or M855. In another specific embodiment, the point mutation in the FLT3 kinase domain is selected from E608K, N676D, N676I, N676S, F691I, F691L, C828S, D835Y, D835V, D835H, D835F, D835E, D839G, D839H, N841C, Y842C, Y842H, Y842N, Y842S, and M855T. In another specific embodiment, "FLT3 kinase domain mutants" refers to point mutations at position F691, D835, or Y842, or refers to FLT3 comprising at least one point mutation at those positions. In another specific embodiment, "FLT3 kinase domain mutants" refers to one or more point mutation

selected from F691L, D835Y, D835V, D835H, D835F, D835E, Y842C, Y842H, Y842N, and Y842S, or refers to FLT3 comprising at least one of the point mutations. In another specific embodiment, FLT3 kinase domain mutants further comprise one or more additional FLT3-ITD mutants. In another specific embodiment, FLT3 kinase domain mutants further comprise one or more additional FLT3-ITD mutants. However, when FLT3 kinase domain mutants comprise more than one point mutations, additional point mutations or mutations can appears on the same FLT3 receptor, or additional point mutations or mutations can appears on the separate alleles or on different pure lineages of leukemia; in this case, the mutation is polyclonal.

The term "juxtamembrane region" or "juxtamembrane domain" of FLT3 refers to a region of FLT3 that connects transmembrane helices to tyrosine kinase domains.

In another specific embodiment, "wild-type FLT3" refers to FTL3 gene or allele, comprising allelic variants and mutations other than FLT3 kinase domain mutations and FLT3-ITD mutations.

c-KIT

In a specific embodiment, compounds disclosed herein are inhibitors of a c-Kit kinase, and can be used for treating symptoms associated with abnormal c-KIT activities. Activating mutations in c-KIT exist in a variety of indications, including systemic mastocytosis, gastrointestinal stromal tumor, acute myeloid leukemia, melanoma, seminoma, intracranial germ cell tumor, and mediastinal B-cell lymphoma.

In another specific embodiment, compounds disclosed herein can be used for treating one or more c-Kit mutations in exon 17 (e.g., D816V, D816Y, D816F, D816K, D816A, D816G, D820A, D820E, D820G, N822K, N822H, Y823D, and A829P) and have activity on the mutations while having much lower activity on wild-type c-Kit.

In the treatment method of the present disclosure, "effective amount" is intended to refer to an amount or dose sufficient to produce the required therapeutic benefits in individuals in need thereof. The effective amount or dose of the compound of the present disclosure can be determined by conventional methods (e.g., modeling, dose escalation or clinical trials) and conventional factors (e.g.,, mode or way for drug delivery, pharmacokinetics of a formulation, severity and process of infection, health status and weight of an individual, and judgment of a physician). An exemplary dose is in the range of from about 0.1 mg to 1 g per day, or about 1 mg to 50 mg per day, or about 50 mg to 250 mg per day, or about 250 mg to 1 g per day. The total dose can be a single dose unit or separate dose units (e.g., BID, TID, or QID).

After the patient's disease is improved, the dose can be adjusted for prophylactic or maintenance therapy. For example, the dose or frequency of administration or both can be reduced to an amount that maintains the desired therapeutic or preventive effect depending on symptoms. Of course, if symptoms have been reduced to an appropriate extent, the treatment can be stopped. However, patients may require long-term intermittent treatment if any recurrence of symptoms. Patients may also need long-term slow treatment.

Drug combination

The compound of the present disclosure can be used in combination with one or more other active ingredients in pharmaceutical compositions or methods to treat the diseases and conditions described herein. Other additional active ingredients include other therapeutic agents or medicines that mitigate adverse effects of the therapeutic agent on the intended disease target. The combination can be used to increase efficacy, improve other disease symptoms, reduce one or more negative effects, or reduce the required dosage of the compound of the present disclosure. The additional active ingredient may be formulated into a pharmaceutical composition separate from the compound of the present disclosure or may be included in a single pharmaceutical composition with the compound of the present disclosure. The additional active ingredient may be administered simultaneously with, before, or after the administration of the compound of the present disclosure.

Combination agents include those additional active ingredients that known or observed to be effective in the treatment of the diseases and conditions described herein, including those effectively against another target related to the disease. For example, the compositions and formulations of the present disclosure, and treatment methods may further include other drugs or medicines, such as other active agents that can be used to treat or alleviate the target disease or related symptoms or conditions. For cancer indications, other such agents include, but are not limited to, kinase inhibitors, for example, EGFR inhibitors (such as erlotinib, gefitinib); Raf inhibitors (such as vemurafenib), VEGFR inhibitors (such as sunitinib); standard chemotherapeutic agents such as alkylating agents, antimetabolites, antitumor antibiotics, topoisomerase inhibitors, platinum drugs, mitotic inhibitors, antibodies, hormone therapy or corticosteroids. For pain indications, suitable combination agents include anti-inflammatory agents, such as NSAID. The pharmaceutical composition of the present disclosure may additionally include one or more of the active agents, and the method of treatment may additionally include administering an effective amount of one or more of the active agents.

## EXAMPLE

The present disclosure will be further described below in combination with specific examples. It should be understood that these examples are only used to illustrate the present disclosure and not to limit the scope of the present disclosure. The experimental methods without specific conditions in the following examples generally follow the conventional conditions or the conditions recommended by the manufacturer. Unless otherwise stated, parts and percentages are parts by weight and weight percent.

Generally, in the preparation process, each reaction is carried out in an inert solvent at a temperature from room temperature to reflux temperature (e.g., 0 °C to 100 °C, or alternatively 0 °C to 80 °C). The reaction time is usually 0.1-60 hours, or alternatively 0.5-24 hours.

The abbreviations as used herein have the following meanings:

| | |
|---|---|
| $Pd(dppf)Cl_2$: | [1,1'-bis(diphenylphosphino)ferrocene]dichloropalladium(II) |
| $B_2pin_2$: | bis(pinacolato)diboron |
| NBS: | N-bromosuccinimide |
| DMAP: | 4-dimethylaminopyridine |
| HATU: | 2-(7-oxido-benzotriazole)-*N*,*N*,*N*',*N*'-tetramethylurea hexafluorophosphate |
| TEA: | triethylamine |
| DIEA: | N,N-diisopropylethylamine |
| $Na_2CO_3$: | sodium carbonate |
| $K_2CO_3$: | potassium carbonate |
| MeOH: | methanol |
| EtOH: | ethanol |
| DCM: | dichloromethane |
| THF: | tetrahydrofuran |
| ACN: | acetonitrile |
| DME: | 1,2-dimethoxyethane |
| DMF: | N,N-dimethylformamide |
| EDCI: | 1-ethyl-3-(3-dimethylaminopropyl)carbodiimide |
| HATU: | 2-(7-azabenzotriazole)-*N*,*N*,*N*',*N*'-tetramethyluronium hexafluorophosphate |

**Intermediate A-1: Preparation of 3-iodo-1-isopropyl-1H-pyrazolo[3,4-d]pyrimidin-4-amine**

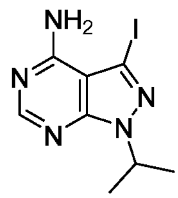

A-1

**The following route was used for the synthesis:**

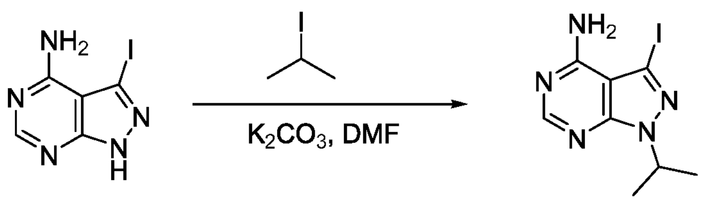


A-1

3-iodo-1H-pyrazolo[3,4-d]pyrimidin-4-amine (4.6 g, 17.6 mmol) and potassium carbonate (4.86 g, 35.2 mmol) were dissolved in 50 mL of DMF, and 2-iodopropane (3.3 g, 19.4 mmol) was slowly added dropwise under an ice bath.

The ice bath was removed after the completion of addition, and the reaction was stirred at room temperature for 2 hours. The reaction solution was diluted with 150 mL of water, and extracted with ethyl acetate (100 mL*3). The organic phase was washed with 50 mL of saturated brine, dried over anhydrous sodium sulfate, and concentrated. The residue was purified by silica gel column to give 3.2 g of a pale-yellow solid in a yield of 60%. ESI-MS:304[$M^+$+1].

**Intermediate A-2: Preparation of 5-iodo-7-isopropyl-7H-pyrrolo[2,3-d]pyrimidin-4-amine**

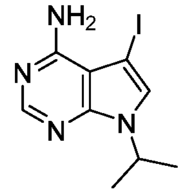

A-2

**The following route was used for the synthesis:**

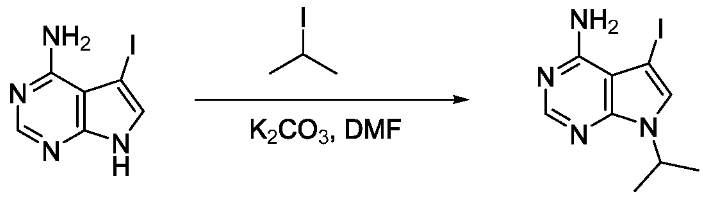


**A-2**

5-iodo-7H-pyrrolo[2,3-d]pyrimidin-4-amine (4.6 g, 17.6 mmol) and potassium carbonate (4.86 g, 35.2 mmol) were dissolved in 50 mL of DMF, and 2-iodopropane (3.3 g, 19.4 mmol) was slowly added dropwise under an ice bath. The ice bath was removed after the completion of addition, and the reaction was stirred at room temperature for 2 hours. The mixture was diluted with 150 mL of water, and extracted with ethyl acetate (100 mL*3). The organic phase was washed with 50 mL of saturated brine, dried over anhydrous sodium sulfate, and concentrated. The residue was purified by silica gel column to give 2.8 g of a pale-yellow solid in a yield of 52%. ESI-MS:303[$M^+$+1].

**Intermediate A-3: Preparation of 1-bromo-3-isopropylimidazo[1,5-alpyrazin-8-amine**

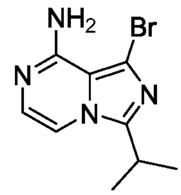

A-3

**The following route was used for the synthesis:**

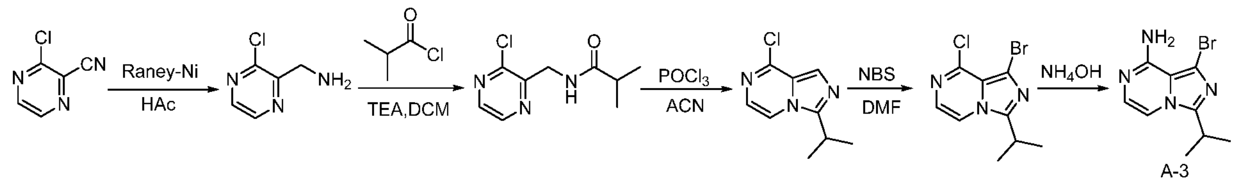


A-3

Step 1. Synthesis of compound (3-chloropyrazin-2-yl)methylamine

2-cyano-3-chloropyrazine (1.5 g, 10.8 mmol) and Raney-Ni (50% slurry in water, 0.5 g) were added to 15 mL of glacial acetic acid under nitrogen gas, and the atmosphere was replaced 3 times with hydrogen gas. The reaction was stirred at 50°C under hydrogen atmosphere of 3 atm overnight. After the completion of the reaction, the reaction was filtered and the filtrate was concentrated. The residue was dissolved in ethyl acetate, washed with saturated solution of sodium bicarbonate, dried over anhydrous sodium sulfate, and concentrated. The residue was purified by silica gel column to give 0.82 g of a light-yellow oil in a yield of 53%. ESI-MS:144[$M^+$+1].

Step 2. Synthesis of compound N-((3-chloropyrazin-2-yl)methyl)isobutyramide

(3-chloropyrazin-2-yl)methylamine (0.82g, 5.7 mmol) and triethylamine (0.86 g, 8.5 mmol) were dissolved in 10 mL of dichloromethane, and isobutyryl chloride (0.72 g, 6.8 mmol) was slowly added dropwise under an ice bath. The ice bath was removed after the completion of addition, and the reaction was stirred at room temperature overnight. The reaction was completed by TLC monitoring. The reaction solution was washed with 1N hydrochloric acid, 5% $NaHCO_3$ solution, and saturated saline, respectively. The organic phase was dried over anhydrous sodium sulfate, and concentrated. The residue was purified by silica gel column to give 0.7 g of a light-yellow oil in a yield of 58%. ESI-MS:214[$M^+$+1].

Step 3. Synthesis of compound 8-chloro-3-isopropylimidazo[1,5-a]pyrazine

N-((3-chloropyrazin-2-yl)methyl)isobutyramide (0.7 g, 3.3 mmol) was dissolved in 15 mL of anhydrous acetonitrile, and phosphorus oxychloride (2.1 g, 14 mmol) was added. The mixture was heated to 60 °C and reacted overnight. After the reaction was completed, the solvent was evaporated. The residue was slowly added to ice water, and the mixture was extracted with ethyl acetate (10 mL*3). The organic phase was washed respectively with saturated sodium bicarbonate solution and saturated brine, dried over anhydrous sodium sulfate, and concentrated. The residue was purified by silica gel column to give 0.51 g of a light-yellow solid in a yield of 80%. ESI-MS:196[$M^+$+1].

Step 4. Synthesis of compound 1-bromo-8-chloro-3-isopropylimidazo[1,5-a]pyrazine

8-chloro-3-isopropylimidazo[1,5-a]pyrazine (0.51 g, 2.6 mmol) was dissolved in 10 mL of DMF, and NBS (0.51 g, 2.9 mmol) was added in batches under an ice bath. The mixture was allowed to naturally warm to room temperature and reacted overnight. After the reaction was completed, 20 mL of water was added to the reaction solution. The mixture was extracted with ethyl acetate (10 mL*3). The organic phase was washed with 10 mL of saturated brine, dried over anhydrous sodium sulfate, and concentrated. The residue was purified by silica gel column to give 0.51 g of a light-yellow solid in a yield of 72%. ESI-MS:276[$M^+$+2].

Step 5. Synthesis of compound 1-bromo-3-isopropylimidazo[1,5-a]pyrazin-8-amine (Intermediate A-3)

To a 50 mL sealed tube were added 1-bromo-8-chloro-3-isopropylimidazo[1,5-a]pyrazine (0.51 g, 1.8 mmol) and 10 mL of concentrated ammonia. The mixture was heated to 120 °C under an oil bath and reacted overnight. After the reaction was completed, the reaction solution was allowed to cool to room temperature. The solvent was removed by rotary evaporation to give 0.4 g of a white powdery solid in a yield of 87%. ESI-MS:257[$M^+$+2].

**Intermediate A-4: Preparation of 5-bromo-7-isopropylpyrrolo[2,1-f][1,2,4]triazin-4-amine**

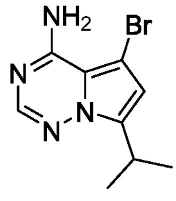

A-4

**The following route was used for the synthesis:**

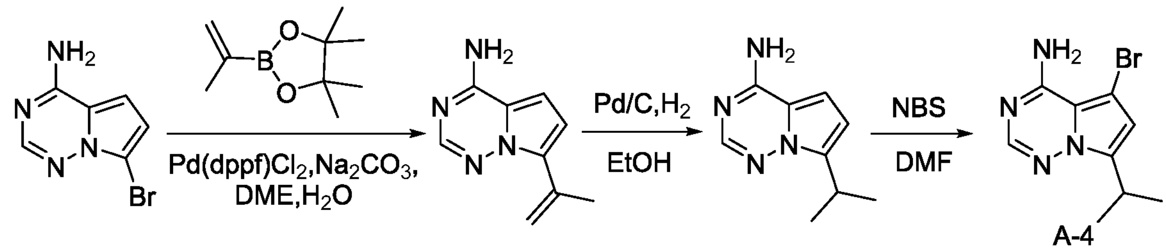


A-4

Step 1. Synthesis of compound 7-(prop-1-en-2-yl)pyrrolo[2,1-f][1,2,4]triazin-4-amine

4-amino-7-bromopyrrolo[2,1-f][1,2,4]triazine (0.94 g, 4.4 mmol), isopropenylboronic acid pinacol ester (0.89 g, 5.3 mmol), Pd(dppf)$Cl_2$ (165 mg, 0.22 mmol) and $Na_2CO_3$ (1.4 g, 13.2 mmol) were added to 20 mL of DME and 5 mL of water, and the atmosphere was replaced 3 times with nitrogen gas. The mixture was heated to 90 °C and reacted overnight. The reaction solution was cooled to room temperature, and 40 mL of water was added. The mixture was extracted with ethyl acetate (30 mL*3). The organic phase was washed with 20 mL of saturated brine, dried over anhydrous sodium sulfate, and concentrated. The residue was purified by silica gel column to give 0.54 g of a light-yellow solid in a yield of 70%. ESI-MS:175[$M^+$+1].

Step 2. Synthesis of compound 7-isopropylpyrrolo[2,1-f][1,2,4]triazin-4-amine

7-(prop-1-en-2-yl)pyrrolo[2,1-f][1,2,4]triazin-4-amine (0.54 g, 3.1 mmol) was dissolved in 20 mL of ethanol, and 100 mg of 10% Palladium/carbon was added. The atmosphere was replaced 3 times with hydrogen gas, and the reaction was stirred under hydrogen atmosphere of 1 atm at room temperature overnight. After the completion of the reaction, the palladium/carbon was filtered off. The filtrate was concentrated and dried to give 0.49 g of a white solid in a yield of 90%. ESI-MS: 177[$M^+$+1].

Step 3. Synthesis of compound 5-bromo-7-isopropylpyrrolo[2,1-f][1,2,4]triazin-4-amine (Intermediate A-4)

7-isopropylpyrrolo[2,1-f][1,2,4]triazin-4-amine (0.49 g, 2.8 mmol) was dissolved in 10 mL of DMF, and NBS (0.55 g, 3.1 mmol) was added in batches under an ice bath. The mixture was allowed to naturally warm to room temperature and reacted overnight. After the reaction was completed, 20 mL of water was added to the reaction solution, and the mixture was extracted with ethyl acetate (10 mL*3). The organic phase was washed with 10 mL of saturated brine, dried over anhydrous sodium sulfate, and concentrated. The residue was purified by silica gel column to give 0.5 g of a light-yellow solid in a yield of 70%. ESI-MS:257[$M^+$+2].

**Intermediate A-5: Preparation of 5-iodo-7-isopropylimidazo[5,1-f][1,2,4]triazin-4-amine**

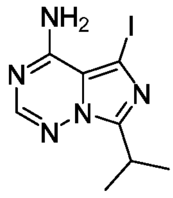

A-5

**The following route was used for the synthesis:**

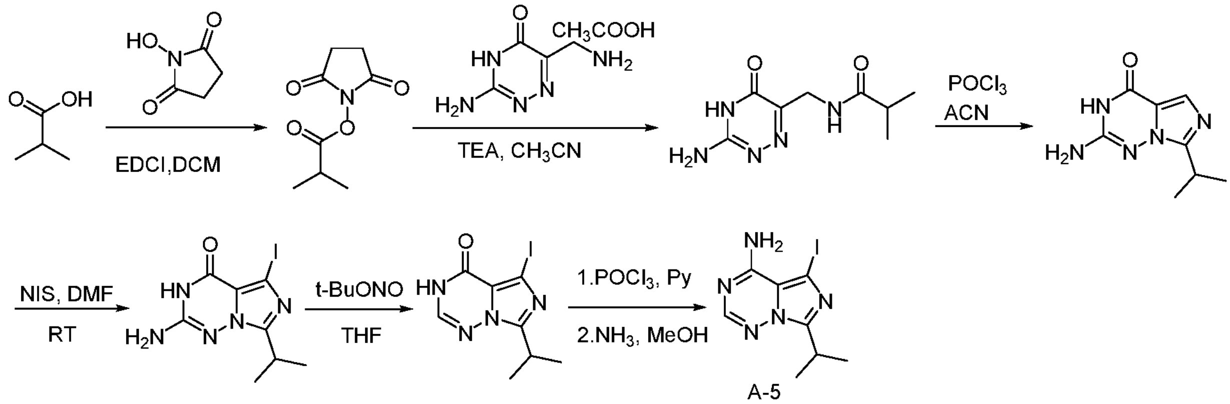


Step 1 Synthesis of compound 2,5-oxopyrrolidin-1-yl isobutyrate

Isobutyric acid (2.67 g, 30.4 mmol), 1-hydroxypyrrolidine-2,5-dione (4.26 g, 37 mmol) and triethylamine (6.14 g, 60.8 mmol) were dissolved in dichloromethane (50 mL), and EDCI (8.68 g, 45.3 mmol) was added under an ice bath. The mixture was reacted at room temperature overnight. The reaction solution was diluted with DCM (150 mL), and washed with water (200 mLx3). The organic phase was washed with saturated brine, dried over anhydrous sodium sulfate, and concentrated to dryness to give 4.6 g of a pale-yellow oil in a yield of 81.8%. ESI-MS:186.1[$M^+$+1].

Step 2 Synthesis of compound N-((3-amino-5-oxo-4,5-dihydro-1,2,4-triazin-6-yl)methyl)isobutyramide

2,5-oxopyrrolidin-1-yl isobutyrate (1.8 g, 10.0 mmol) and 3-amino-6-(aminomethyl)-1,2,4-triazin-5(4H)-one (2.0 g, 10.0 mmol) were dissolved in acetonitrile (30 mL), and triethylamine (3.0 g, 30 mmol) was added. The mixture was heated to 50 °C and reacted for 2 hours. A large amount of a white solid precipitated out. The reaction mixture was allowed to cool to room temperature, diluted with water (150 mL), and stirred for 10 minutes. The mixture was then filtered, and washed with water (30 mL). The filter cake was dried with baking to give 1.7 g of an off-white solid in a yield of 80.6%. ESI-MS:212.1[$M^+$+1].

Step 3 Synthesis of compound 2-amino-7-isopropylimidazo[5,1-f][1,2,4]triazin-4(3H)-one

N-((3-amino-5-oxo-4,5-dihydro-1,2,4-triazin-6-yl)methyl)isobutyramide (1.7 g, 8.1 mmol) was added to acetonitrile (30 mL), and phosphorus oxychloride (2.45 g, 16.2 mmol) was slowly added dropwise under a nitrogen atmosphere. The mixture was heated to 80 °C, and reacted for 4 hours while maintaining the temperature. The reaction was cooled to room temperature, and quenched by adding water (20 mL). Saturated aqueous sodium bicarbonate solution (30 mL) was added, and the mixture was extracted with ethyl acetate (60 mL*3). The organic phase was washed with saturated brine, dried over anhydrous sodium sulfate, and concentrated. The residue was purified by silica gel column to give 0.6 g of an off-white solid in a yield of 38.6%. ESI-MS:194.1[$M^+$+1].

Step 4 Synthesis of compound 2-amino-5-iodo-7-isopropylimidazo[5,1-f][1,2,4]triazin-4(3H)-one

2-amino-7-isopropylimidazo[5,1-f][1,2,4]triazin-4(3H)-one (0.6 g, 3.1 mmol) was dissolved in DMF (8 mL), and NIS (0.70 g, 3.1 mmol) was added in batches under an ice bath. The mixture was allowed to naturally warm to room temperature and reacted overnight. After the reaction was completed, 50 mL of water was added to the reaction solution,

and the mixture was extracted with ethyl acetate (50 mL*3). The organic phase was washed with saturated brine (150 mLx3), dried over anhydrous sodium sulfate, and concentrated. The residue was purified by silica gel column to give 0.62 g of a light-yellow solid in a yield of 62.5%. ESI-MS:320.0[$M^{+}$+2].

Step 5 Synthesis of compound 5-iodo-7-isopropylimidazo[5,1-f][1,2,4]triazin-4(3H)-one

2-amino-5-iodo-7-isopropylimidazo[5,1-f][1,2,4]triazin-4(3H)-one (0.62 g, 1.94 mmol) was dissolved in tetrahydrofuran (8 mL) and DMF (2 mL), and tert-butyl nitrite (1.0 g, 9.7 mmol) was slowly added dropwise under a nitrogen atmosphere. The mixture was reacted under a nitrogen atmosphere at room temperature for 4 hours. After the reaction was completed, the reaction solution was diluted with water (50 mL) and extracted with ethyl acetate (30 mL*3). The organic phase was washed with saturated brine (50 mLx2), dried over anhydrous sodium sulfate, and concentrated. The residue was purified by silica gel column to give 0.46 g of a light-yellow solid in a yield of 77.8%. ESI-MS:305[$M^{+}$+1].

Step 6 Synthesis of compound 5-iodo-7-isopropylimidazo[5,1-f][1,2,4]triazin-4-amine

Under a nitrogen atmosphere, phosphorus oxychloride (0.67 g, 4.5 mmol) was slowly added dropwise to a solution of 1,2,4-triazole (0.93 g, 13.5 mmol) in pyridine (6 mL) cooled in an ice-water bath, and a white solid was formed. The reaction was stirred at room temperature for 20 minutes. 5-iodo-7-isopropylimidazo[5,1-f][1,2,4]triazin-4(3H)-one (0.45 g, 1.5 mmol) was dissolved in pyridine (3 mL), and the resulting solution was slowly added dropwise to the above-mentioned mixture solution of phosphorus oxychloride. After the addition was completed, the reaction mixture was reacted with stirring at room temperature for 2 hours. After the reaction was completed, the reaction solution was added dropwise to ammonia water (40mL) in an ice-water mixture (150 g), and the mixture was reacted for half an hour. The reaction mixture was extracted with ethyl acetate (60 mLx3), and the organic phases were combined. The combined organic phase was washed with saturated brine (30mL), dried with anhydrous sodium sulfate, and concentrated. The residue was purified by silica gel column to give 0.32 g of a light-yellow solid in a yield of 70.2%. ESI-MS:304.1[$M^{+}$+2].

**Intermediate B-1: Preparation of 2-(4-(4,4,5,5-tetramethyl-1,3,2-dioxaborolan-2-yl)phenyl)-N-(5-(1,1,1-trifluoro-2-methylprop-2-yl)isoxazol-3-yl)acetamide**

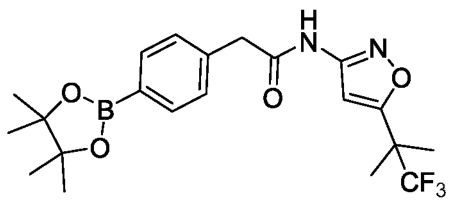

B-1

**The following route was used for the synthesis:**

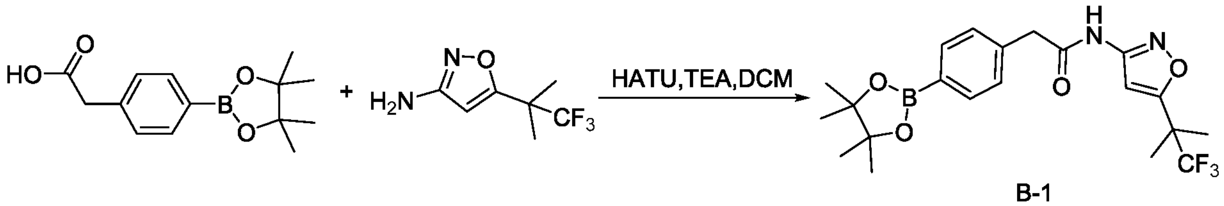


B-1

4-(carboxymethyl)phenylboronic acid pinacol ester (3.85 g, 14.7 mmol), 5-(1,1,1-trifluoro-2-methylprop-2-yl)isoxazol-3-amine (2.85 g, 14.7 mmol) and triethylamine (3.0 g, 29.4 mmol) were dissolved in 50 mL of dichloromethane, and HATU (8.4 g, 22 mmol) was added under an ice bath. The mixture was reacted at room temperature overnight. The reaction solution was diluted with 50 mL of dichloromethane, and washed with water. The organic phase was washed with 30 mL of saturated brine, dried over anhydrous sodium sulfate, and concentrated. The residue was purified by silica gel column to give 4.5 g of a light-yellow solid in a yield of 70%. ESI-MS:439[$M^{+}$+1].

**Intermediate B-2: Preparation of 1-(4-(4,4,5,5-tetramethyl-1,3,2-dioxaborolan-2-yl)phenyl)-3-(5-(1,1,1-trifluoro-2-methylprop-2-yl)isoxazol-3-yl)urea**

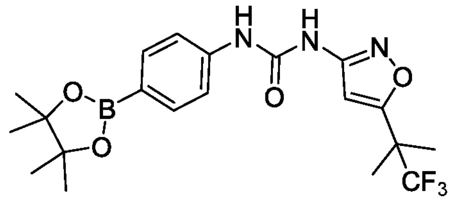

B-2

**The following route was used for the synthesis:**

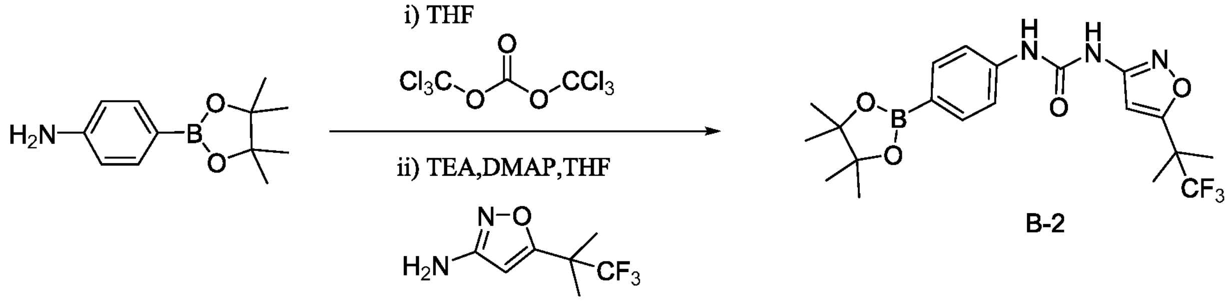


B-2

4-aminophenylboronic acid pinacol ester (0.79 g, 3.6 mmol) was dissolved in 15 mL of tetrahydrofuran, and bis(trichloromethyl) carbonate (0.38 g, 1.3 mmol) was slowly added. The mixture was heated to reflux and reacted for 1 hour. The solvent was removed by rotary evaporation. The residue was dissolved in 20 mL of tetrahydrofuran, and DMAP (49 mg, 0.4 mmol), triethylamine (0.73 g, 7.2 mmol), and then 5-(1,1,1-trifluoro-2-methylprop-2-yl)isoxazol-3-amine (0.7 g, 3.6 mmol) were added. The mixture was reacted under reflux overnight. The reaction solution was cooled to room temperature, and 40 mL of water was added. The mixture was extracted with ethyl acetate (30 mL*3). The organic phase was washed with 10 mL of saturated brine, dried over anhydrous sodium sulfate, and concentrated. The residue was purified by silica gel column to give 1.2 g of a light-yellow solid in a yield of 76%. ESI-MS:440[$M^{+}$+1].

**Intermediate B-3: Preparation of 2-(6-(4,4,5,5-tetramethyl-1,3,2-dioxaborolan-2-yl)pyridin-3-yl)-N-(5-(1,1,1-trifluoro-2-methylprop-2-yl)isoxazol-3-yl)acetamide**

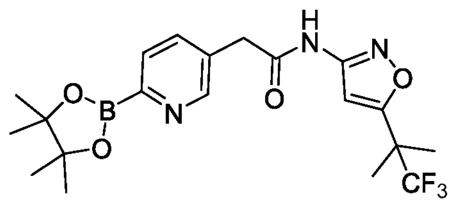

B-3

**The following route was used for the synthesis:**

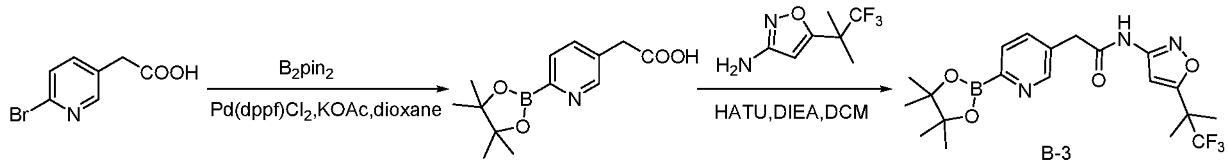


B-3

Step 1: Synthesis of compound 2-(6-(4,4,5,5-tetramethyl-1,3,2-dioxaborolan-2-yl)pyridin-3-yl)acetic acid

2-(6-bromopyridin-3-yl)acetic acid (4.23 g, 19.6 mmol), bis(pinacolato)diboron (5.97 g, 23.5 mmol), Pd(dppf)$Cl_2$ (0.44 g, 0.6 mmol), and potassium acetate (5.88 g, 60 mmol) were added to 80 mL of dioxane, and the atmosphere was replaced 3 times with nitrogen gas. The mixture was heated to 90 °C and reacted for 3 hours. The reaction solution was cooled to room temperature, and 150 mL of water was added. The mixture was extracted with ethyl acetate (100 mL*3). The organic phase was washed with 50 mL of saturated brine, dried over anhydrous sodium sulfate, and concentrated. The residue was purified by silica gel column to give 3.87 g of a light-yellow solid in a yield of 75%. ESI-MS:264[$M^+$+1].

Step 2: Synthesis of compound 2-(6-(4,4,5,5-tetramethyl-1,3,2-dioxaborolan-2-yl)pyridin-3-yl)-N-(5-(1,1,1-trifluoro-2-methylprop-2-yl)isoxazol-3-yl)acetamide (Intermediate B-3)

2-(6-(4,4,5,5-tetramethyl-1,3,2-dioxaborolan-2-yl)pyridin-3-yl)acetic acid (3.87 g, 14.7 mmol), 5-(1,1,1-trifluoro-2-methylprop-2-yl)isoxazol-3-amine (2.85 g, 14.7 mmol) and triethylamine (3.0 g, 29.4 mmol) were dissolved in 20 mL of dichloromethane, and HATU (8.4 g, 22 mmol) was added under an ice bath. The mixture was reacted at room temperature overnight. The reaction solution was diluted with 20 mL of dichloromethane, and washed with water. The organic phase was washed with 10 mL of saturated brine, dried over anhydrous sodium sulfate, and concentrated. The residue was purified by silica gel column to give 3.55 g of a light-yellow solid in a yield of 55%. ESI-MS:440[$M^+$+1].

**Intermediate B-4: Preparation of 2-(4-(4,4,5,5-tetramethyl-1,3,2-dioxaborolan-2-yl)phenyl)-N-(3-(1,1,1-trifluoro-2-methylprop-2-yl)isoxazol-5-yl)acetamide**

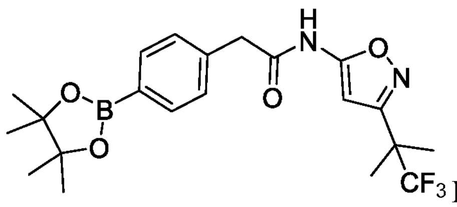

B-4

**The following route was used for the synthesis:**

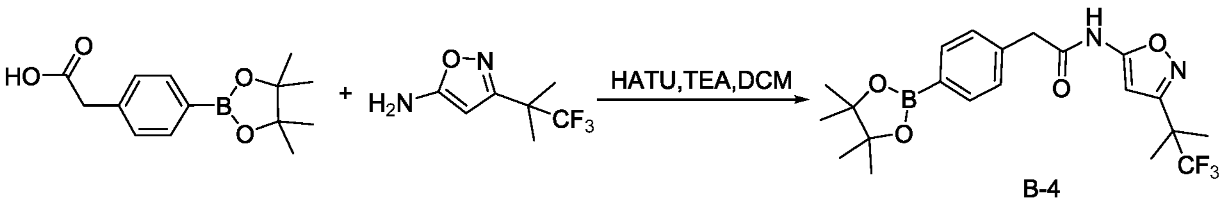


B-4

4-(carboxymethyl)phenylboronic acid pinacol ester (3.85 g, 14.7 mmol), 3-(1,1,1-trifluoro-2-methylprop-2-yl)isoxazol-5-amine (2.85 g, 14.7 mmol) and triethylamine (3.0 g, 29.4 mmol) were dissolved in 50 mL of dichloromethane, and HATU (8.4 g, 22 mmol) was added under an ice bath. The mixture was reacted at room temperature overnight. The reaction solution was diluted with 50 mL of dichloromethane, and washed with water. The organic phase was washed with 30 mL of saturated brine, dried over anhydrous sodium sulfate, and concentrated. The residue was purified by silica gel column to give 4.5 g of a light-yellow solid in a yield of 70%. ESI-MS:439[$M^+$+1].

**Intermediate B-5: Preparation of 2-(6-(4,4,5,5-tetramethyl-1,3,2-dioxaborolan-2-yl)pyridin-3-yl)-N-(3-(1,1,1-trifluoro-2-methylprop-2-yl)isoxazol-5-yl)acetamide**

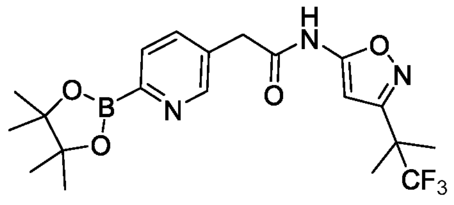

B-5

**The following route was used for the synthesis:**

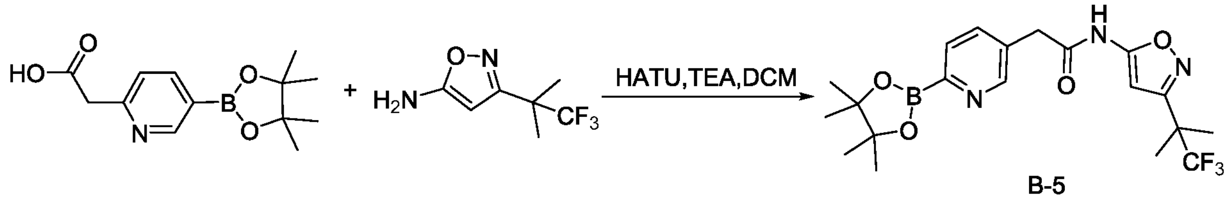


B-5

2-(6-(4,4,5,5-tetramethyl-1,3,2-dioxaborolan-2-yl)pyridin-3-yl)acetic acid (3.87 g, 14.7 mmol), 3-(1,1,1-trifluoro-2-methylprop-2-yl)isoxazol-5-amine (2.85 g, 14.7 mmol) and triethylamine (3.0 g, 29.4 mmol) were dissolved in 20 mL of dichloromethane, and HATU (8.4 g, 22 mmol) was added under an ice bath. The mixture was reacted at room temperature overnight. The reaction solution was diluted with 20 mL of dichloromethane, and washed with water. The organic phase was washed with 10 mL of saturated brine, dried over anhydrous sodium sulfate, and concentrated. The residue was purified by silica gel column to give 3.55 g of a light-yellow solid in a yield of 55%. ESI-MS:440[$M^{+}$+1].

**Example 1. Preparation of 2-(4-(4-amino-1-isopropyl-1H-pyrazolo[3,4-d]pyrimidin-3-yl)phenyl)-N-(5-(1,1,1-trifluoro-2-methylprop-2-yl)isoxazol-3-yl)acetamide (Compound** T-1).

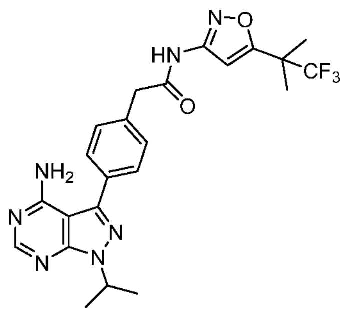

T-1

**The following route was used for the synthesis:**

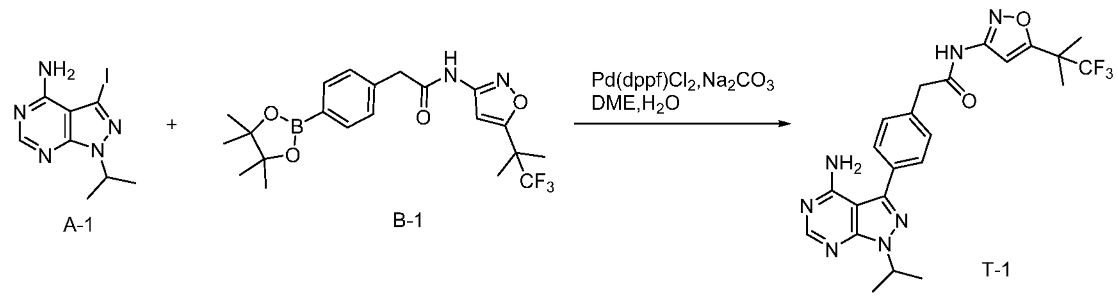


A-1 B-1 T-1

Intermediate A-1 (96 mg, 0.32 mmol), Intermediate B-1 (166 mg, 0.38 mmol), Pd(dppf)$Cl_2$ (15 mg, 0.02 mmol), and $Na_2CO_3$ (102 mg, 0.96 mmol) were added to 12 mL of DME and 4 mL of water, and the atmosphere was replaced 3 times with nitrogen gas. The mixture was heated to 90 °C and reacted overnight. The reaction solution was cooled to room temperature, and 30 mL of water was added. The mixture was extracted with ethyl acetate (20 mL*3). The organic phase was washed with 10 mL of saturated brine, dried over anhydrous sodium sulfate, and concentrated. The residue was purified by silica gel column to give 100 mg of a light-yellow solid in a yield of 65%. ESI-MS:488[$M^+$+1]. $^1H$ NMR (300 MHz, DMSO-$d_6$) δ 11.45 (s, 1H), 8.24 (s, 1H), 7.63 (d, J = 8.1 Hz, 2H), 7.49 (d, J = 7.9 Hz, 2H), 6.97 (s, 1H), 5.15 - 4.96 (m, 1H), 3.78 (s, 2H), 1.53 (s, 6H), 1.48 (d, J = 6.7 Hz, 6H).

**Example 2. Preparation of 1-(4-(4-amino-1-isopropyl-1H-pyrazolo[3,4-d]pyrimidin-3-yl)phenyl)-3-(5-(1,1,1-trifluoro-2-methylprop-2-yl)isoxazol-3-yl)urea (Compound** T-2).

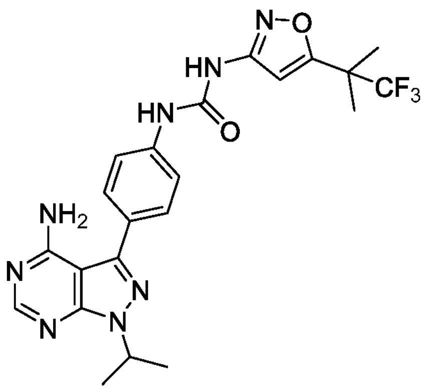

T-2

**The following route was used for the synthesis:**

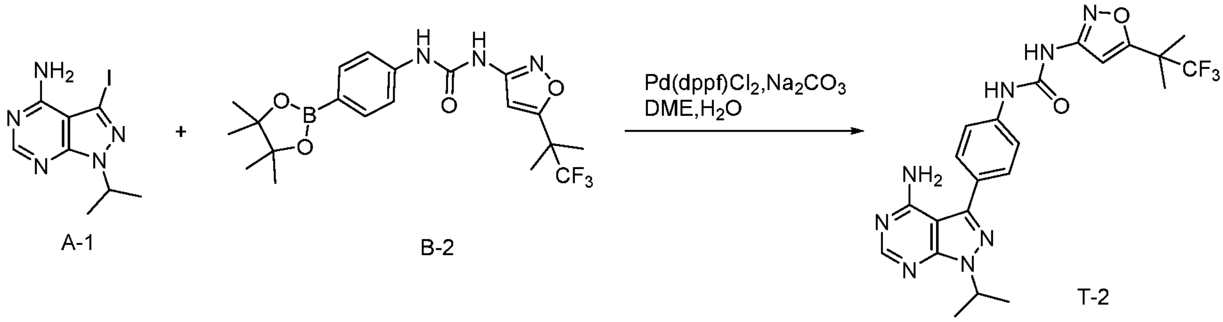


A-1 B-2 T-2

Intermediate A-1 (106 mg, 0.35 mmol), Intermediate B-2 (184 mg, 0.42 mmol), Pd(dppf)$Cl_2$ (15 mg, 0.02 mmol), and $Na_2CO_3$ (110 mg, 1.05 mmol) were added to 12 mL of DME and 4 mL of water, and the atmosphere was replaced 3 times with nitrogen gas. The mixture was heated to 90 °C and reacted overnight. The reaction solution was cooled to

room temperature, and 30 mL of water was added. The mixture was extracted with ethyl acetate (20 mL*3). The organic phase was washed with 10 mL of saturated brine, dried over anhydrous sodium sulfate, and concentrated. The residue was purified by silica gel column to give 110 mg of a light-yellow solid in a yield of 64%. ESI-MS:489[$M^{+}$+1]. $^{1}H$ NMR (400 MHz, DMSO-$d_6$) δ 9.75 (s, 1H), 9.06 (s, 1H), 8.23 (s, 1H), 7.71 - 7.56 (m, 4H), 6.93 (s, 1H), 5.11 - 5.00 (m, 1H), 1.56 (s, 6H), 1.48 (d, J = 6.7 Hz, 6H).

**Example 3. Preparation of 2-(4-(4-amino-7-isopropyl-7H-pyrrolo[2,3-d]pyrimidin-5-yl)phenyl)-N-(5-(1,1,1-trifluoro-2-methylprop-2-yl)isoxazol-3-yl)acetamide (Compound T-3).**

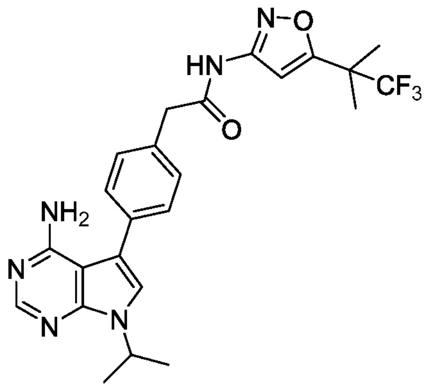

T-3

**The following route was used for the synthesis:**

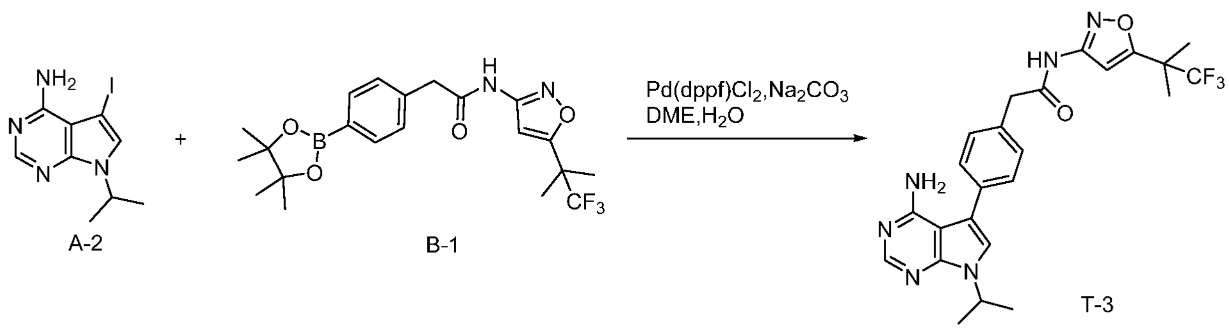


Intermediate A-2 (94 mg, 0.31 mmol), Intermediate B-1 (162 mg, 0.37 mmol), Pd(dppf)$Cl_2$ (15 mg, 0.02 mmol), and $Na_2CO_3$ (131 mg, 1.24 mmol) were added to 12 mL of DME and 4 mL of water, and the atmosphere was replaced 3 times with nitrogen gas. The mixture was heated to 90 °C and reacted overnight. The reaction solution was cooled to room temperature, and 30 mL of water was added. The mixture was extracted with ethyl acetate (20 mL*3). The organic phase was washed with 10 mL of saturated brine, dried over anhydrous sodium sulfate, and concentrated. The residue was purified by silica gel column to give 90 mg of a light-yellow solid in a yield of 60%. ESI-MS:487[$M^{+}$+1]. $^{1}H$ NMR (500 MHz, DMSO-$d_6$) δ 11.39 (s, 1H), 8.14 (s, 1H), 7.46 - 7.39 (m, 5H), 6.97 (s, 1H), 6.04 (s, 2H), 4.97 (p, J = 6.8 Hz, 1H), 3.74 (s, 2H), 1.54 (s, 6H), 1.46 (d, J = 6.8 Hz, 6H).

**Example 4. Preparation of 2-(4-(8-amino-3-isopropylimidazo[1,5-a]pyrazin-1-yl)phenyl)-N-(5-(1,1,1-trifluoro-2-methylprop-2-yl)isoxazol-3-yl)acetamide (Compound** T-4).

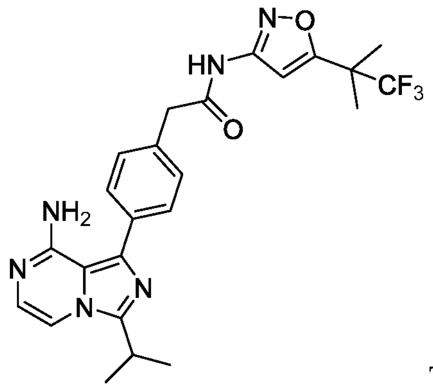

T-4

**The following route was used for the synthesis:**

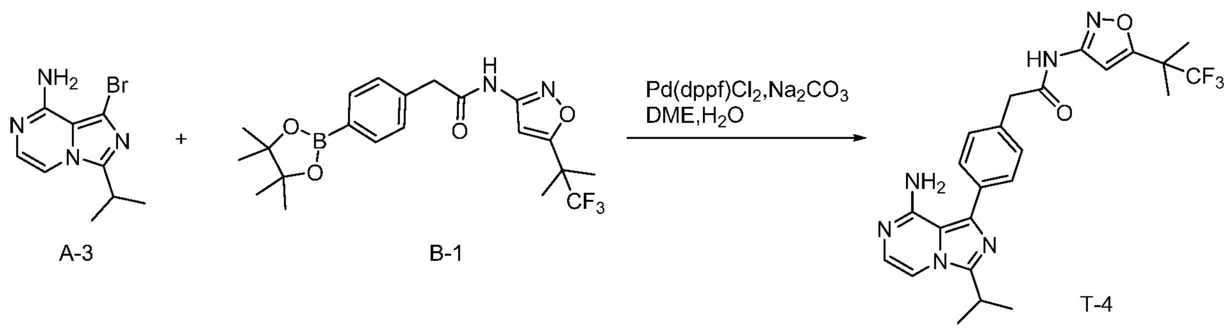


A-3

B-1

T-4

Intermediate A-3 (80 mg, 0.31 mmol), Intermediate B-1 (162 mg, 0.37 mmol), Pd(dppf)$Cl_2$ (15 mg, 0.02 mmol), and $Na_2CO_3$ (131 mg, 1.24 mmol) were added to 12 mL of DME and 4 mL of water, and the atmosphere was replaced 3 times with nitrogen gas. The mixture was heated to 90 °C and reacted overnight. The reaction solution was cooled to room temperature, and 30 mL of water was added. The mixture was extracted with ethyl acetate (20 mL*3). The organic phase was washed with 10 mL of saturated brine, dried over anhydrous sodium sulfate, and concentrated. The residue was purified by silica gel column to give 93 mg of a light-yellow solid in a yield of 62%. ESI-MS:487[$M^+$+1]. $^1$H NMR (500 MHz, DMSO-$d_6$) δ 11.41 (s, 1H), 7.64 - 7.53 (m, 3H), 7.44 (d, J = 7.8 Hz, 2H), 7.02 (d, J = 5.0 Hz, 1H), 6.97 (s, 1H), 5.98 (s, 2H), 3.76 (s, 2H), 3.48 - 3.38 (m, 1H), 1.53 (s, 6H), 1.32 (d, J = 6.8 Hz, 6H).

**Example 5. Preparation of 2-(4-(4-amino-7-isopropylpyrrolo[2,1-f][1,2,4]triazin-5-yl)phenyl)-N-(5-(1,1,1-trifluoro-2-methylprop-2-yl)isoxazol-3-yl)acetamide (Compound T-5).**

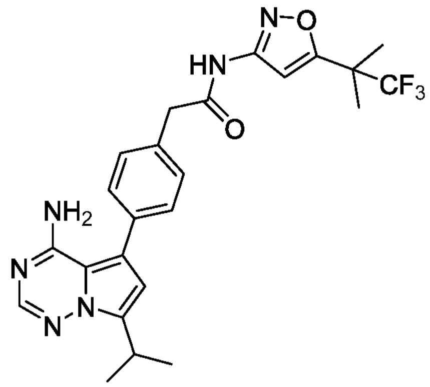

T-5

**The following route was used for the synthesis:**

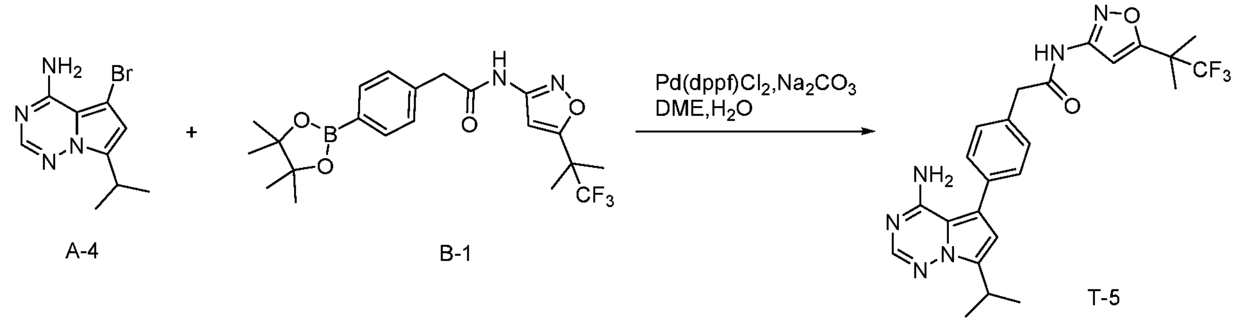


A-4 B-1 T-5

Intermediate A-4 (76 mg, 0.3 mmol), Intermediate B-1 (158 mg, 0.36 mmol), Pd(dppf)$Cl_2$ (15 mg, 0.02 mmol), and $Na_2CO_3$ (127 mg, 1.2 mmol) were added to 10 mL of DME and 2 mL of water, and the atmosphere was replaced 3 times with nitrogen gas. The mixture was heated to 90 °C and reacted overnight. The reaction solution was cooled to room temperature, and 30 mL of water was added. The mixture was extracted with ethyl acetate (20 mL*3). The organic phase was washed with 10 mL of saturated brine, dried over anhydrous sodium sulfate, and concentrated. The residue was purified by silica gel column to give 89 mg of a light-yellow solid in a yield of 61%. ESI-MS:487[$M^+$+1]. $^1$H NMR (500 MHz, $CDCl_3$) $\delta$ 9.01 (s, 1H), 7.94 (s, 1H), 7.48 (d, J = 8.0 Hz, 2H), 7.40 (d, J = 7.8 Hz, 2H), 7.04 (s, 1H), 6.49 (d, J = 3.5 Hz, 1H), 5.47 (s, 1H), 3.82 (s, 2H), 3.58 - 3.46 (m, 1H), 1.57 (s, 6H), 1.38 (d, J = 6.9 Hz, 6H).

**Example 6. Preparation of 2-(4-(4-amino-1-isopropyl-1H-pyrazolo[3,4-d]pyrimidin-3-yl)-2-fluorophenyl)-N-(5-(1,1,1-trifluoro-2-methylprop-2-yl)isoxazol-3-yl)acetamide** (Compound T-6).

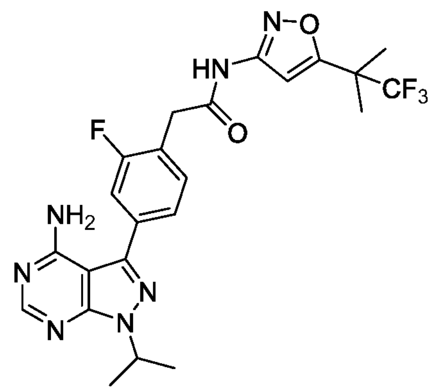

T-6

**The following route was used for the synthesis:**

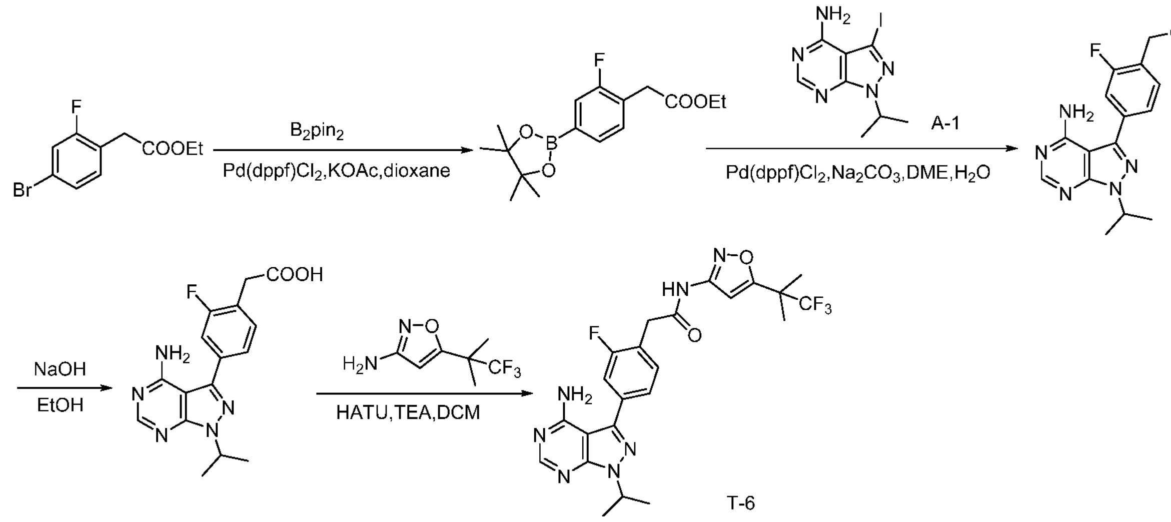


T-6

Step 1. Synthesis of compound ethyl 2-(2-fluoro-4-(4,4,5,5-tetramethyl-1,3,2-dioxaborolan-2-yl)phenyl)acetate

Ethyl 2-(4-bromo-2-fluorophenyl)acetate (2.7 g, 10.3 mmol), bis(pinacolato)diboron (3.15 g, 12.4 mmol), $Pd(dppf)Cl_2$ (0.23 g, 0.3 mmol), and potassium acetate (3.04 g, 31 mmol) were added to 50 mL of dioxane, and the atmosphere was replaced 3 times with nitrogen gas. The mixture was heated to 90 °C and reacted for 3 hours. The reaction solution was cooled to room temperature, and 100 mL of water was added. The mixture was extracted with ethyl acetate (50 mL*3). The organic phase was washed with 40 mL of saturated brine, dried over anhydrous sodium sulfate, and concentrated. The residue was purified by silica gel column to give 2.21 g of a light-yellow solid in a yield of 70%. ESI-MS:309[$M^+$+1].

Step 2. Synthesis of compound ethyl 2-(4-(4-amino-1-isopropyl-1H-pyrazolo[3,4-d]pyrimidin-3-yl)-2-fluorophenyl)acetate

Intermediate A-1 (2.21 g, 7.3 mmol), ethyl 2-(2-fluoro-4-(4,4,5,5-tetramethyl-1,3,2-dioxaborolan-2-yl)phenyl)acetate (2.71 g, 8.8 mmol), $Pd(dppf)Cl_2$ (0.27 g, 0.36 mmol), and $Na_2CO_3$ (2.32 g, 21.9 mmol) were added to 30 mL of DME and 6 mL of water, and the atmosphere was replaced 3 times with nitrogen gas. The mixture was heated to 90 °C and reacted overnight. The reaction solution was cooled to room temperature, and 50 mL of water was added. The mixture was extracted with ethyl acetate (20 mL*3). The organic phase was washed with 10 mL of saturated brine, dried over anhydrous sodium sulfate, and concentrated. The residue was purified by silica gel column to give 1.43 g of a light-yellow solid in a yield of 55%. ESI-MS:358[$M^+$+1].

Step 3. Synthesis of compound 2-(4-(4-amino-1-isopropyl-1H-pyrazolo[3,4-d]pyrimidin-3-yl)-2-fluorophenyl)acetic acid

Ethyl 2-(4-(4-amino-1-isopropyl-1H-pyrazolo[3,4-d]pyrimidin-3-yl)-2-fluorophenyl)acetate (1.43g, 4.0mmol) was dissolved in 20 mL of ethanol and 10 mL of water, and sodium hydroxide (0.4 g, 10 mmol) was added. The mixture was reacted at room temperature overnight. The reaction solution was diluted with 30 mL of water, and adjusted to a pH of 3 to 4 with 1N hydrochloric acid. The mixture was extracted with ethyl acetate (30 mL*3). The organic phase was washed with 20 mL of saturated brine, dried over anhydrous sodium sulfate, and concentrated. The residue was purified by silica gel column to give 1.2 g of a light-yellow solid in a yield of 90%. ESI-MS:330[$M^+$+1].

Step 5. Synthesis of compound 2-(4-(4-amino-1-isopropyl-1H-pyrazolo[3,4-dlpyrimidin-3-yl)-2-fluorophenyl)-N-(5-(1,1,1-trifluoro-2-methylprop-2-yl)isoxazol-3-yl)acetamide (Compound T-6)

2-(4-(4-amino-1-isopropyl-1H-pyrazolo[3,4-d]pyrimidin-3-yl)-2-fluorophenyl)acetic acid (112 mg, 0.34 mmol), (1,1,1-trifluoro-2-methylprop-2-yl)isoxazol-3-amine (66 mg, 0.34 mmol) and triethylamine (70 mg, 0.68 mmol) were dissolved in 10 mL of dichloromethane, and HATU (194 mg, 0.51 mmol) was added under an ice bath. The mixture was reacted at room temperature overnight. The reaction solution was diluted with 20 mL of dichloromethane, and washed with water. The organic phase was washed with 10 mL of saturated brine, dried over anhydrous sodium sulfate, and concentrated. The residue was purified by silica gel column to give 120 mg of a light-yellow solid in a yield of 70%. ESI-MS:506[$M^+$+1]. $^1H$ NMR (300 MHz, DMSO-d6) 8 11.47 (s, 1H), 8.25 (s, 1H), 7.63 - 7.35 (m, 3H), 6.96 (s, 1H), 5.17 -

4.92 (m, 1H), 3.86 (s, 2H), 1.54 (s, 6H), 1.48 (d, J = 6.7 Hz, 6H).

**Example 7. Preparation of 2-(6-(4-amino-1-isopropyl-1H-pyrazolo[3,4-d]pyrimidin-3-yl)pyridin-3-yl)-N-(5-(1,1,1-trifluoro-2-methylprop-2-yl)isoxazol-3-yl)acetamide (Compound T-7).**

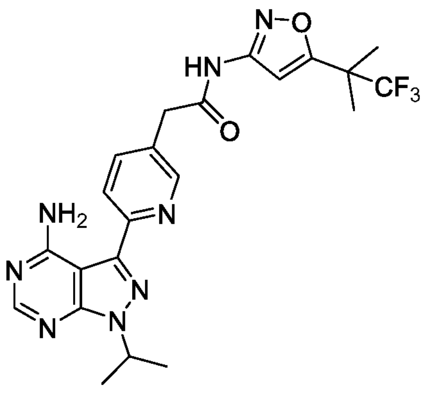

T-7

**The following route was used for the synthesis:**

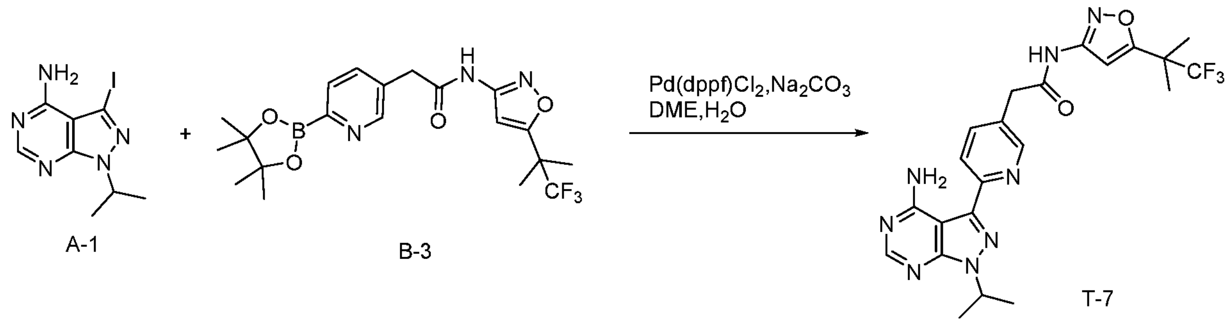


A-1 B-3 T-7

Intermediate A-1 (121 mg, 0.4 mmol), Intermediate B-3 (210 mg, 0.48 mmol), Pd(dppf)$Cl_2$ (15 mg, 0.02 mmol), and $Na_2CO_3$ (127 mg, 1.2 mmol) were added to 10 mL of DME and 2 mL of water, and the atmosphere was replaced 3 times with nitrogen gas. The mixture was heated to 90 °C and reacted overnight. The reaction solution was cooled to room temperature, and 30 mL of water was added. The mixture was extracted with ethyl acetate (20 mL*3). The organic phase was washed with 10 mL of saturated brine, dried over anhydrous sodium sulfate, and concentrated. The residue was purified by silica gel column to give 110 mg of a light-yellow solid in a yield of 56%. ESI-MS:489[$M^{+}$+1]. $^1H$ NMR (300 MHz, DMSO-$d_6$) δ 11.51 (s, 1H), 8.26 (s, 1H), 7.95 (s, 1H), 7.52 (d, J = 7.9 Hz, 2H), 6.93 (s, 1H), 5.12 - 4.94 (m, 1H), 3.76 (s, 2H), 1.54 (s, 6H), 1.48 (d, J = 6.7 Hz, 6H).

**Example 8. Preparation of 2-(4-(4-amino-1-isopropyl-1H-pyrazolo[3,4-d]pyrimidin-3-yl)phenyl)-N-(3-(1,1,1-trifluoro-2-methylprop-2-yl)isoxazol-5-yl)acetamide (Compound T-8).**

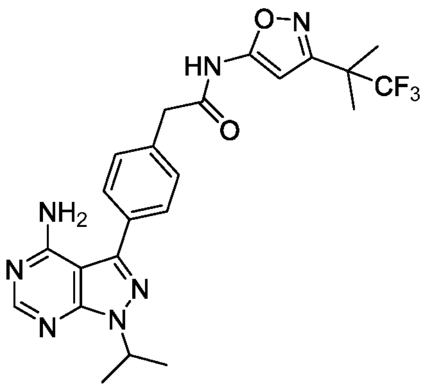

T-8

**The following route was used for the synthesis:**

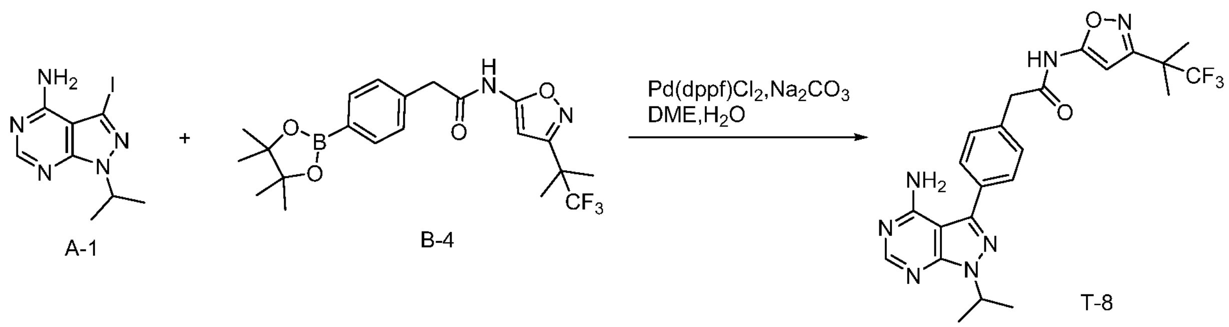


Intermediate A-1 (96 mg, 0.32 mmol), Intermediate B-4 (166 mg, 0.38 mmol), Pd(dppf)$Cl_2$ (15 mg, 0.02 mmol), and $Na_2CO_3$ (102 mg, 0.96 mmol) were added to 12 mL of DME and 4 mL of water, and the atmosphere was replaced 3 times with nitrogen gas. The mixture was heated to 90 °C and reacted overnight. The reaction solution was cooled to room temperature, and 30 mL of water was added. The mixture was extracted with ethyl acetate (20 mL*3). The organic phase was washed with 10 mL of saturated brine, dried over anhydrous sodium sulfate, and concentrated. The residue was purified by silica gel column to give 100 mg of a light-yellow solid in a yield of 65%. ESI-MS:488[$M^+$+1]. $^1H$ NMR (300 MHz, DMSO-$d_6$) $\delta$ 10.65 (s, 1H), 8.27 (s, 1H), 7.68 (d, J = 8.1 Hz, 2H), 7.42 (d, J = 7.9 Hz, 2H), 5.97 (s, 1H), 5.17 - 4.94 (m, 1H), 3.78 (s, 2H), 1.54 (s, 6H), 1.48 (d, J = 6.7 Hz, 6H).

**Example 9. Preparation of 2-(6-(4-amino-1-isopropyl-1H-pyrazolo[3,4-d]pyrimidin-3-yl)pyridin-3-yl)-N-(3-(1,1,1-trifluoro-2-methylprop-2-yl)isoxazol-5-yl)acetamide (Compound T-9).**

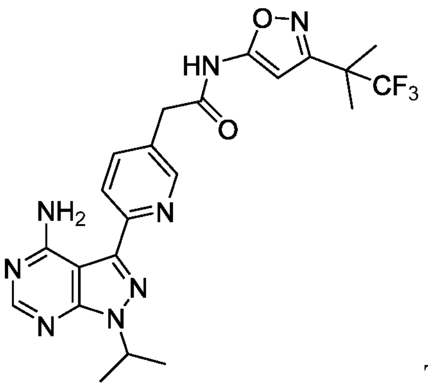

T-9

**The following route was used for the synthesis:**

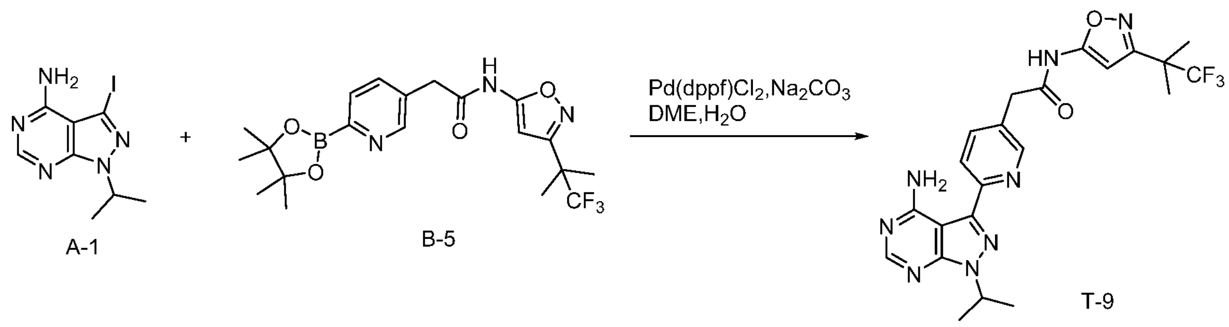


A-1

B-5

T-9

Intermediate A-1 (121 mg, 0.4 mmol), Intermediate B-5 (210 mg, 0.48 mmol), Pd(dppf)$Cl_2$ (15 mg, 0.02 mmol), and $Na_2CO_3$ (127 mg, 1.2 mmol) were added to 10 mL of DME and 2 mL of water, and the atmosphere was replaced 3 times with nitrogen gas. The mixture was heated to 90 °C and reacted overnight. The reaction solution was cooled to room temperature, and 30 mL of water was added. The mixture was extracted with ethyl acetate (20 mL*3). The organic phase was washed with 10 mL of saturated brine, dried over anhydrous sodium sulfate, and concentrated. The residue was purified by silica gel column to give 110 mg of a light-yellow solid in a yield of 56%. ESI-MS:489[$M^+$+1]. $^1$H NMR (300 MHz, DMSO-$d_6$) $\delta$ 10.64 (s, 1H), 8.29 (s, 1H), 7.93 (s, 1H), 7.55 (d, J = 7.9 Hz, 2H), 5.98 (s, 1H), 5.14 - 4.92 (m, 1H), 3.75 (s, 2H), 1.54 (s, 6H), 1.46 (d, J = 6.7 Hz, 6H).

**Example 10. Preparation of 2-(4-(4-amino-7-isopropylimidazo[5,1-f][1,2,4]triazin-5-yl)phenyl)-N-(5-(1,1,1-trifluoro-2-methylprop-2-yl)isoxazol-3-yl)acetamide (Compound T-10).**

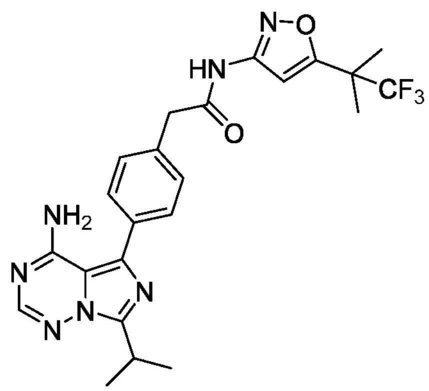

T-10

**The following route was used for the synthesis:**

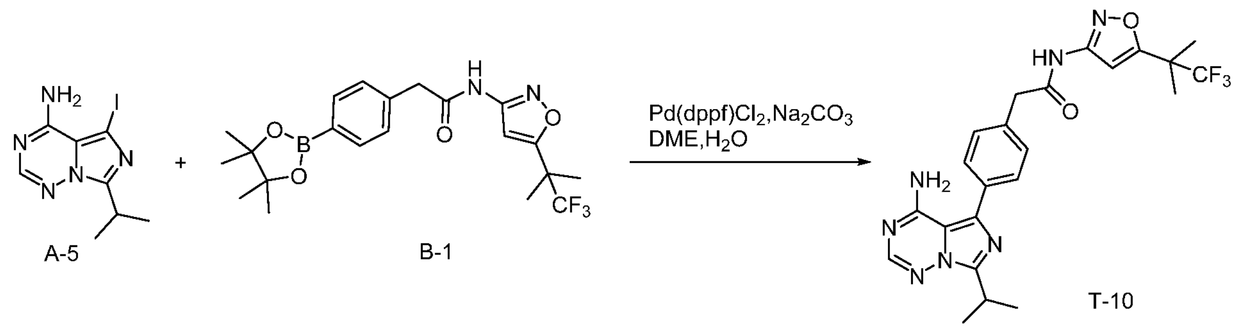


Intermediate A-5 (96 mg, 0.32 mmol), Intermediate B-1 (166 mg, 0.38 mmol), Pd(dppf)$Cl_2$ (15 mg, 0.02 mmol), and $Na_2CO_3$ (102 mg, 0.96 mmol) were added to DME (12 mL) and water (4 mL), and the atmosphere was replaced 3 times with nitrogen gas. The mixture was heated to 90 °C and reacted overnight. The reaction solution was cooled to room temperature, and 30 mL of water was added. The mixture was extracted with ethyl acetate (20 mL*3). The organic phase was washed with 10 mL of saturated brine, dried over anhydrous sodium sulfate, and concentrated. The residue was purified by silica gel column to give 80 mg of a light-yellow solid in a yield of 51.2%. ESI-MS:488.1[$M^+$+1]. $^1H$ NMR(400 MHz, DMSO-$D_6$)δ(ppm):11.38(s, 1H), 7.90(s, 1H), 7.58(d, J=8.4 Hz, 2H), 7.44(d, J=8.4 Hz, 2H), 6.95(s, 1H), 3.75(s, 2H), 3.51(t, J=6.8 Hz, 1H), 1.53(s, 6H), 1.33(d, J=6.8 Hz, 6H).

**Biological Activity Assay**

**Biological Example 1: Inhibition of Kinase**

Reagents and materials:
Ret wt (Carna, Cat. No. 08-159-10ug), RET (V804M), Active (Signalchem, Cat. No. R02-12GG), HTRF KinEASE-TK kit (Cisbio, Cat. No. 62TK0PEC), CEP-32496(MCE, Cat. No. HY-15200), ATP (Sigma, Cat. No. A7699), DMSO (Sigma, Cat. No. D8418-1L), DTT (Sigma, Cat. No. D0632), $MgCl_2$ (Sigma, Cat. No. M1028), 384-well plate (Labcyte, Cat. No. P-05525-BC).

Specific Assay Procedure:

Formulation of compound: The test compounds were dissolved in DMSO to obtain 10 mM stock solutions, which were then serially diluted 3-fold in DMSO to obtain 10 concentrations. At the time of addition, the compound was further diluted 10-fold with buffer.

Ret wt and RET V804M kinase detection:
In 5 x kinase buffer A, Ret wt or RET V804M kinase was mixed with different concentrations of compounds prepared by pre-dilution for 10 minutes. Each concentration was tested in duplicate. The corresponding substrate and ATP were added, and reaction was performed at room temperature for 20 minutes (negative and positive controls were provided: the negative control was a blank control, and the positive control was CEP-32496 (Agerafenib)). After completion of the reaction, detection reagents (reagents in the HTRF KinEASE-TK kit) were added. After incubating for 30 minutes at room temperature, enzyme activity in the presence of various concentrations of the compounds of the present disclosure was determined by Envision microplate reader, and inhibitory activity of different concentrations of the compounds on enzyme activity was calculated. The inhibitory activity of different concentrations of the compounds on enzyme activity was fitted by Graphpad 5.0 software, and the $IC_{50}$ value was calculated.

The compounds disclosed herein were tested in the above assay of inhibition of kinase, and were found to have potent activity against Ret wt and RET V804M. Results of representative example compounds were summarized in Table 1 below.

Table 1:

| Example compound | Ret wt $IC_{50}$ (nM) | RET V804M $IC_{50}$ (nM) |
|---|---|---|
| T-1 | A | A |
| T-2 | A | C |
| T-3 | A | C |

(continued)

| Example compound | Ret wt $IC_{50}$ (nM) | RET V804M $IC_{50}$ (nM) |
|---|---|---|
| T-4 | A | C |
| T-6 | A | B |
| T-7 | C | C |
| wherein A represents $IC_{50} \leq 0.5$ nM, B represents 0.5 nM < $IC_{50} \leq 1$ nM, C represents 1 nM < $IC_{50} \leq 10$ nM, D represents 10 nM < $IC_{50} \leq 50$ nM, and E represents $IC_{50}$ > 50 nM | | |

**Biological Example 2: Cytotoxicity Assay**

Inhibitory effects of example compounds on the activity of Ba/$F_3$ parental generation, Ba/$F_3$ KIF5B-RET, Ba/$F_3$ KIF5B-$RET^{V804M}$, Ba/$F_3$ KIF5B-$RET^{V804L}$, Ba/$F_3$ KIF5B-$RET^{G810R}$, Ba/$F_3$ FLT3-ITD, Ba/$F_3$ FLT3-$ITD^{D835Y}$, Ba/$F_3$ FLT3-$ITD^{F691L}$ cells were determined.

Materials and reagents: fetal bovine serum FBS (GIBCO, Cat# 10099141), CellTiter-Glo® Luminescent Cell Viability Assay kit (Promega, Cat#G7572), 96-well black-wall clear flat-bottom plate (Corning®, Cat# 3603).

Assay Procedure:

**Culture and seeding of cells:**

1. Cells in the logarithmic growth phase were harvested and counted with a platelet counter. Cell viability was detected by a trypan blue exclusion method, ensuring that cell viability was 90% or more;
2. Cell concentration was adjusted to about 3000 cells/well; 90 μl of cell suspension was added to each well of the 96-well plate;
3. The cells in the 96-well plate were incubated overnight under the condition of 37°C, 5% $CO_2$, and 95% humidity.

**Dilution and addition of compounds:**

1. A series of compound solutions were formulated by 3.16-fold serial dilution starting from a concentration of 10 μM, resulting in 9 concentrations. 10 μL of the compound solutions were added in each well of the 96-well plate seeded with cells in triplicate for each compound concentration;
2. The cells in the 96-well plate added with compounds were further incubated under the condition of 37°C, 5% $CO_2$, and 95% humidity for 72 hrs, and then CTG analysis was carried out.

**Plate reading at the end:**

1. CTG reagent was thawed, and the cell plate was equilibrated at room temperature for 30 minutes;
2. An equal volume (10μL) of CTG solution was added to each well;
3. The cell plate was shaken on an orbital shaker for 5 minutes to lyse the cells;
4. The cell plate was placed at RT for 20 minutes to stabilize the luminescence signal;
5. Luminescence value was read.

**Processing of data**

Data were analyzed by GraphPad Prism 5.0 software, and fitted by nonlinear S-curve regression to obtain a dose-response curve, from which $IC_{50}$ values were calculated.

Cell survival rate (%) = (luminescence value of drug to be tested - luminescence value of culture medium control) / (luminescence value of cell control - luminescence value of culture medium control) × 100%.

The compounds disclosed herein were tested in the above cytotoxicity assay. The compounds disclosed herein were found to have potent activities and selectivity on $Ba/F_3$ KIF5B-RET, $Ba/F_3$ KIF5B-$RET^{V804M}$, $Ba/F_3$ KIF5B-$RET^{V804L}$, $Ba/F_3$ KIF5B-$RET^{G810R}$, $Ba/F_3$ FLT3-ITD, and $Ba/F_3$ FLT3-$ITD^{D83SY}$ cell lines. The results of representative example compounds were summarized in Tables 2 and 3 below, where the structure of AD80 is as follows:

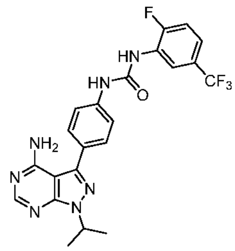

Table 2:

| | $Ba/F_3$ parental generation | Ba/F3 KIF5B-RET | | Ba/F3 KIF5B-$RET^{V804M}$ | | $Ba/F_3$ KIF5B-$RET^{v804L}$ | | Ba/F3 KIF5B-$RET^{G810R}$ | |
|---|---|---|---|---|---|---|---|---|---|
| | | $IC_{50}$ (nM) | Selectivi ty onparental generati on | $IC_{50}$ (nM) | Selectivity on parent al genera tion | $IC_{50}$ (nM) | Selectiv ity on parental generati on | $IC_{50}$ (nM) | Selectiv ity on parental generati on |
| AD80 | 811.94 | 2.19 | 370.7 | 10.79 | 75.2 | 9.05 | 89.7 | 7.88 | 103.0 |
| T-1 | 1620.39 | 0.15 | 10802.6 | 5.65 | 286.8 | 3.71 | 436.8 | 2.64 | 613.8 |
| T-2 | 337.83 | 0.16 | 2111.4 | 2.64 | 128.0 | 5.39 | 62.68 | 2.98 | 113.4 |
| T-3 | 724.44 | 0.58 | 1249.0 | 3.21 | 225.7 | 3.63 | 199.6 | 3.84 | 188.7 |
| T-4 | 701.10 | 1.56 | 449.4 | 9.53 | 73.6 | 31.6 | 22.2 | 25.5 | 27.5 |
| T-5 | 4783 | 2.88 | 1660.8 | 12.9 | 370.8 | 19.7 | 242.8 | 13.6 | 351.7 |
| T-6 | 2095.49 | 0.31 | 6759.6 | 9.90 | 211.7 | 5.8 | 361.3 | 2.92 | 717.6 |
| T-7 | 328.06 | 4.61 | 71.2 | 3.42 | 95.9 | 55.7 | 5.9 | 6.92 | 47.4 |
| T-8 | 3677 | 1.03 | 3569.9 | 3.60 | 1021.4 | 1.53 | 2403.3 | 2.39 | 1538.5 |
| T-9 | 4118 | 10.9 | 377.8 | 29.9 | 137.7 | 49.3 | 83.5 | 47.7 | 86.3 |
| T-10 | 2242 | 3.68 | 609.2 | 11.7 | 191.6 | 18.4 | 121.8 | 2.78 | 806.5 |

Table 3:

| | $Ba/F_3$ parental generation | Ba/F3 FLT3-ITD | | Ba/F3 FLT3-$ITD^{D835Y}$ | | Ba/F3 FLT3-$ITD^{F691L}$ | |
|---|---|---|---|---|---|---|---|
| | | $IC_{50}$ (nM) | Selectivity on parental generation | $IC_{50}$ (nM) | Selectivity on parental generation | $IC_{50}$ (nM) | Selectivity on parental generation |
| AD80 | 811.94 | 0.67 | 1211.9 | 30.09 | 21.82 | 11.8 | 68.8 |
| T-1 | 1620.39 | 0.31 | 5227.1 | 14.42 | 137.97 | 7.7 | 210.4 |

(continued)

| | Ba/$F_3$ parental generation | Ba/F3 FLT3-ITD | | Ba/F3 FLT3-ITD$^{D835Y}$ | | Ba/F3 FLT3-ITD$^{F691L}$ | |
|---|---|---|---|---|---|---|---|
| | | $IC_{50}$ (nM) | Selectivity on parental generation | $IC_{50}$ (nM) | Selectivity on parental generation | $IC_{50}$ (nM) | Selectivity on parental generation |
| T-2 | 337.83 | 0.41 | 824.0 | 6.05 | 35.48 | 2.93 | 115.3 |
| T-3 | 724.44 | 0.10 | 7244.4 | 2.92 | 218.67 | 0.97 | 746.8 |
| T-4 | 701.10 | 0.10 | 7011.0 | 23.05 | 32.03 | 16.6 | 42.2 |
| T-5 | 4783 | 1.89 | 2530.7 | 35.8 | 133.6 | 11.4 | 419.6 |
| T-6 | 2095.49 | 0.40 | 5238.7 | 9.60 | 193.58 | 6.26 | 334.7 |
| T-7 | 328.06 | 0.10 | 3280.6 | 5.57 | 32.63 | 3.22 | 101.9 |
| T-8 | 3677 | 0.25 | 14708 | 7.34 | 500.9 | 6.28 | 585.5 |
| T-9 | 4118 | 5.49 | 750.1 | 46.9 | 87.8 | 28.1 | 146.5 |
| T-10 | 2242 | 0.32 | 7006.3 | 46.4 | 48.3 | 39.1 | 57.3 |

**Biological Example 3: Pharmacokinetic assay in rats**

Six male Sprague-Dawley rats (7-8 weeks old, and weighing about 210 g) were divided into 2 groups, 3 rats in each group. A single dose of compounds (1 mg/kg intravenously, 10 mg/kg orally) was administered intravenously or orally to compare their pharmacokinetics.

The rats were fed with standard feed and water. Fasting was started 16 hours before the assay. The drug was dissolved in 5% DMSO, 10% Solutol (polyethylene glycol-15 hydroxystearate) and 85% normal saline. Blood was collected from the orbit. The time points for blood collection were 0.083 hours, 0.25 hours, 0.5 hours, 1 hour, 2 hours, 4 hours, 8 hours, 12 hours and 24 hours after administration.

The rats were briefly anesthetized after inhaling diethyl ether, and 300 μL of blood sample was collected from the orbit into a test tube containing 30 μL of 1% heparin sodium solution. Before use, the test tube was oven dried overnight at 60 °C. After the blood sample was collected at the last time point, the rats were anesthetized with diethyl ether and then sacrificed.

Immediately after the blood sample was collected, the test tube was gently inverted at least 5 times to ensure sufficient mixing, and then placed on ice. The blood sample was centrifuged at 6000 rpm at 4 °C for 8 minutes to separate the plasma from the red blood cells. 100 μL of blood (about 50 μL of plasma can be produced) was aspirated into a micro K2EDTA tube with a pipette, and the name of compound and the time point were marked. The plasma was stored at -20 °C before analysis. The concentration of the compound of the present disclosure in plasma was determined by LC-MS/MS. The pharmacokinetic parameters were calculated based on the blood drug concentration of each animal at different time points.

The assay shows that the compounds of the present disclosure have better pharmacokinetic properties in animals, and thus have better pharmacodynamics and therapeutic effects.

**Biological Example 4: Efficacy in Ba/$F_3$ KIF5B-RET and Ba/$F_3$ KIF5B-RET$^{G810R}$ allograft models of female NPSG mice**

The efficacy of compounds of the present disclosure in female NPSG mouse allograft models of two B-cell lymphoma cell lines Ba/$F_3$ KIF5B-RET and Ba/$F_3$ KIF5B-RET$^{G810R}$ was verified.

**Assay animals:** Female NPSG mice, 6-8 weeks old (age of mice at the time of tumor cell inoculation), weighing 18-20 g, 60 mice, purchased from Shanghai Jihui Laboratory Animal Care Co.,Ltd.

**Environmental conditions of breeding room for assay animals:** The assay animals were all kept in individually wentilate cagesdul (IVC). The breeding room had a temperature of 20-26 °C, a humidity of 30-70%, and 12h/12h day and night. Dry pellet feed that was sterilized by radiation was continuously supplied and freely accessible without limitation. Drinking tap water (sterilized by acidification) was continuously supplied from drinking bottle and freely accessible without limitation. The bedding was corncob bedding, which was changed once a week. There were 4-5 animals in each box. The number, gender, strain, receiving time, treatment method, item number, group number, mouse number, starting time of administration of animals were marked on the cage card. The assay animals were marked with ear tags.

The animal handling and feeding in this assay were carried out in accordance with AAALAC guidelines, version

2011 (International guidelines as reported in the Guide for the Care and Use of Laboratory Animals, National Research Council 2011).

**Assay methods:** 1) Ba/F3 KIF5B-RET and Ba/F3 KIF5B-RET$^{G810R}$ cells were respectively resuscitated and cultured in vitro, and $3\times10^7$ cells of each type were obtained. 2) 60 female NPSG mice aged 6-8 weeks were adaptively bred for 1 week and then weighed. 3) The inoculation conditions were shown in Table 4. 4) After inoculation, tumor volume and body weight were measured once a week. When average tumor volume reached about 75-110 mm$^3$, each type of Ba/$F_3$ cells were randomly divided into 7 groups according to tumor volume and body weight, with 3 in each group. The administration was started immediately after grouping. The date of start of administration was regarded as day 0. Informations on administration and grouping were shown in Tables 5 and 6. 5) After the start of the administration, the body weight and tumor volume of mice were measured twice a week. 6) After the end of the administration, the assay was over.

Table 4. Inoculation information

| Animal strain | Number of animals | Type of inoculated cells | Inoculation position | Number of inoculated cells | Volume of inoculated cell suspension (mL) | Total number of cells required |
|---|---|---|---|---|---|---|
| NPSG | 30 | Ba/F3 KIF5B-RET | Subcutaneous at the right shoulder | $1\times10^6$ | 0.1mL | $3\times10^7$ |
| NPSG | 30 | Ba/F3 KIF5B-RET$^{G810R}$ | Subcutaneous at the right shoulder | $1\times10^6$ | 0.1mL | $3\times10^7$ |

Table 5. Grouping and administration information of Ba/F3 KIF5B-RET cells

| Grou p | Numbe r of animal s | Group of administratio n | Dosage of administratio n (mg/kg) | Route of administratio n | Amount of administratio n (uL/g) | Cycle of administratio n |
|---|---|---|---|---|---|---|
| 1 | 3 | Vehicle control | - | p.o. | 10 | QD×14 |
| 2 | 3 | Positive control AD80 | 10 | p.o. | 10 | QD×14 |
| 3 | 3 | T-1 | 10 | p.o. | 10 | QD×14 |
| 4 | 3 | T-2 | 10 | p.o. | 10 | QD×14 |
| 5 | 3 | T-3 | 10 | p.o. | 10 | QD×14 |
| 6 | 3 | T-6 | 10 | p.o. | 10 | QD×14 |
| 7 | 3 | T-8 | 10 | p.o. | 10 | QD×14 |

Table 6. Grouping and administration information of Ba/F3 KIF5B-RET$^{G810R}$ cells

| Grou p | Numbe r of animal s | Group of administratio n | Dosage of administratio n (mg/kg) | Route of administratio n | Amount of administratio n (uL/g) | Cycle of administratio n |
|---|---|---|---|---|---|---|
| 1 | 3 | Vehicle control | - | p.o. | 10 | QD×13 |
| 2 | 3 | Positive control AD80 | 25 | p.o. | 10 | QD×13 |
| 3 | 3 | T-1 | 25 | p.o. | 10 | QD×13 |
| 4 | 3 | T-2 | 25 | p.o. | 10 | QD×13 |
| 5 | 3 | T-3 | 25 | p.o. | 10 | QD×13 |
| 6 | 3 | T-6 | 25 | p.o. | 10 | QD×13 |

(continued)

| Group | Number of animals | Group of administration | Dosage of administration (mg/kg) | Route of administration | Amount of administration (uL/g) | Cycle of administration |
|---|---|---|---|---|---|---|
| 7 | 3 | T-8 | 25 | p.o. | 10 | QD×13 |

Assay observation and data collection: After tumor inoculation, morbidity and death of animals were observed every working day. Daily observation included tumor growth and effects of drugs on laboratory animals, such as changes in activity, changes in feeding and drinking, weight loss, changes in appearance of hair and eye, death and other clinical symptoms. Formula for calculating tumor volume is: tumor volume ($mm^3$) = 1/2 × (a × $b^2$) (wherein a represents the long diameter and b represents the short diameter)

**Relative tumor inhibition rate TGI (%):** TGI% = (1-T/C) ×100%. T/C % is relative tumor proliferation rate, that is, a percentage value of relative tumor volume between treatment group and control group at a certain point in time. T and C are relative tumor volume (RTV, that is, the difference between tumor volume at this time point and tumor volume at day 0) of treatment group and control group at a certain point in time, respectively.

**Statistical analysis:** For pairwise comparison, a T-Test analysis method was used, and for comparison of three or more groups, a One-Way ANOVA method was used. For comparison of potential synergistic effects, a Two-way ANOVA was used. SPSS 24.0 was used for all data. A p value less than 0.05 was considered a significant difference.

**Assay results:**

**1. Result and discussion of anti-tumor effect assay in Ba/F3 KIF5B-RET cell line tumor model**

Mice in the vehicle control group had an average tumor volume of 1447 $mm^3$ at day 14 of administration. Mice in the positive control AD80 group (10 mg/kg) had an average tumor volume of 821.7 $mm^3$ at day 14 of administration, and the relative tumor inhibition rate TGI (%) was 46.4%. Compared with the positive control AD80 group, the efficacy in the test drug group was significantly increased. Mice in the groups of test drugs T-1, T-2, T-3, T-6 and T-8 had average tumor volumes of 233 $mm^3$, 588.7 $mm^3$, 153.7 $mm^3$, 108.7 $mm^3$ and 163.7 $mm^3$, respectively, at day 14 of administration, and relative tumor inhibition rates TGI (%) were 90.3%, 63.9%, 96.3%, 99.7% and 95.4%, respectively. It can be found that the efficacy in the test drug groups was significantly increased compared with the positive control AD80 group.

The tumor growth in each treatment group and control group was shown in Table 7 and FIG. 1.

Table 7. Table of drug efficacy analysis in each group in Ba/F3 KIF5B-RET cell line tumor model

| **Assay group** | **Day 0 of administration** | **Day 14 of administration** | |
|---|---|---|---|
| | **Tumor volume ($\bar{x}$±S)** | **Tumor volume ($\bar{x}$±S)** | **TGI (%)** |
| Group 1 vehicle control | 100±8.39 | 1447±242 | -- |
| Group 2 positive control AD80 (10 mg/kg) | 100.3±4.33 | 821.7±54.5 | 46.4% |
| Group 3 test drug T-1 (10 mg/kg) | 102.7±6.06 | 233±65.74 | 90.3% |
| Group 4 test drug T-2 (10 mg/kg) | 102±5.51 | 588.7±25.3 | 63.9% |
| Group 5 test drug T-3 (10 mg/kg) | 104±10.44 | 153.7±13.22 | 96.3% |
| Group 6 test drug T-6 (10 mg/kg) | 104±3.61 | 108.7±31.33 | 99.7% |
| Group 7 test drug T-8 (10 mg/kg) | 101.3±4.33 | 163.7±19.74 | 95.4% |

**2. Result and discussion of safety assay in Ba/F3 KIF5B-RET cell line tumor model**

None of the treatment groups including the groups of test drugs T-1, T-2, T-3, T-6 and T-8 and the group of positive control AD80 showed significant weight loss, drug withdrawal, or significant drug toxicity. The treatment groups were well tolerated during the treatment.

Changes in body weight of mice in the treatment group and the control group after administration were shown in Table 8, FIG. 2, and FIG. 3.

Table 8. Changes in body weight of mice in each group in Ba/F3 KIF5B-RET cell line tumor model

| Assay group | Number of animals at the beginning/end of assay | Average weight g ($\bar{x}\pm$S) | | Weight change rate (%) |
|---|---|---|---|---|
| | | Day 0 of administration | Day 14 of administration | Day 14 of administration |
| Group 1 vehicle control | 3/2* | 20.1±1.12 | 23.6±0.25 | 11.1% |
| Group 2 positive control AD80 (10 mg/kg) | 3/3 | 20.1±0.81 | 22.6±0.87 | 12.7% |
| Group 3 test drug T-1 (10 mg/kg) | 3/3 | 20.9±1.06 | 21.6±1.05 | 3.4% |
| Group 4 test drug T-2 (10 mg/kg) | 3/3 | 20.1±0.56 | 21.7±0.59 | 8.1% |
| Group 5 test drug T-3 (10 mg/kg) | 3/3 | 19.6±0.84 | 21.5±0.7 | 9.8% |
| Group 6 test drug T-6 (10 mg/kg) | 3/3 | 19.3±0.57 | 21±0.91 | 8.8% |
| Group 7 test drug T-8 (10 mg/kg) | 3/3 | 20.5±1.07 | 21.7±0.82 | 6.0% |
| * One of the mice died. | | | | |

**3. Result and discussion of anti-tumor effect assay in Ba/F3 KIF5B-RET$^{G810R}$ cell line tumor model**

Mice in the vehicle control group had an average tumor volume of 457 mm$^3$ at day 13 of administration. Mice in the positive control AD80 group (25 mg/kg) had an average tumor volume of 185.7 mm$^3$ at day 13 of administration, and relative tumor inhibition rate TGI (%) was 75.2%. Mice in the groups of test drugs T-1, T-2, T-3, T-6 and T-8 had average tumor volumes of 63.7mm3, 150.7 mm$^3$, 132.7 mm$^3$, 89.7 mm$^3$ and 90.7 mm$^3$, respectively, at day 13 of administration, and relative tumor inhibition rates TGI (%) were 109.4%, 84.6%, 91.6%, 100.9% and 101.6%, respectively. It can be found that the efficacy in the test drug groups was significantly increased compared with the positive control AD80 group.

The tumor growth of mice in each treatment group and control group was shown in Table 9 and FIG. 4.

Table 9. Table of drug efficacy analysis in each group in Ba/F3 KIF5B-RET$^{G810R}$ cell line tumor model

| **Assay group** | **Day 0 of administration** | **Day 13 of administration** | |
|---|---|---|---|
| | **Tumor volume** ($\bar{x}\pm$S) | **Tumor volume** ($\bar{x}\pm$S) | **TGI (%)** |
| Group 1 vehicle control | 90.3±6.89 | 457±91.13 | - |
| Group 2 positive control AD80 (25 mg/kg) | 94.7±5.24 | 185.7±33.65 | 75.2% |
| Group 3 test drug T-1 (25 mg/kg) | 98±3.61 | 63.7±16.19 | 109.4%# |
| Group 4 test drug T-2 (25 mg/kg) | 94.3±3.84 | 150.7±4.33 | 84.6% |
| Group 5 test drug T-3 (25 mg/kg) | 102±7.77 | 132.7±11.78 | 91.6% |
| Group 6 test drug T-6 (25 mg/kg) | 93±8.33 | 89.7±4.48 | 100.9%# |
| Group 7 test drug T-8 (25 mg/kg) | 96.7±6.74 | 90.7±8.41 | 101.6%# |
| # TGI more than 100% was manifested as a decrease in tumor volume. | | | |

**4. Result and discussion of safety assay in Ba/F3 KIF5B-RET$^{G810R}$ cell line tumor model**

None of the treatment groups including the groups of test drugs T-1, T-2, T-3, T-6 and T-8 and the group of

positive control AD80 showed significant weight loss, drug withdrawal, or significant drug toxicity. The treatment groups were well tolerated during the treatment.

Changes in body weight of mice in the treatment group and the control group after administration were shown in Table 10, FIG. 5, and FIG. 6.

Table 10. Changes in body weight of mice in each group in Ba/F3 KIF5B-RET$^{G810R}$ cell line tumor model

| Assay group | Number of animals at the beginning/end of assay | Average weight g ($\bar{x}\pm$S) | | Weight change rate (%) |
|---|---|---|---|---|
| | | Day 0 of administration | Day 13 of administration | Day 13 of administration |
| Group 1 vehicle control | 3/3 | 20.4±1.21 | 22.5±0.89 | 10.43% |
| Group 2 positive control AD80 (25 mg/kg) | 3/3 | 19.9±0.44 | 20.7±1.53 | 4.14% |
| Group 3 test drug T-1 (25 mg/kg) | 3/3 | 20.3±1.32 | 21.3±1.19 | 4.90% |
| Group 4 test drug T-2 (25 mg/kg) | 3/3 | 19.6±0.47 | 21.7±0.74 | 11.05% |
| Group 5 test drug T-3 (25 mg/kg) | 3/3 | 19.9±1.18 | 23.1±1.49 | 16.01% |
| Group 6 test drug T-6 (25 mg/kg) | 3/3 | 19.4±0.66 | 22.4±0.46 | 15.75% |
| Group 7 test drug T-8 (25 mg/kg) | 3/3 | 19.6±0.32 | 22.5±0.73 | 14.40% |

The above is a further detailed description of the present disclosure in conjunction with specific alternative embodiments, and it cannot be assumed that the specific embodiments of the present disclosure are limited to these descriptions. For ordinary artisan in the art to which the present disclosure belongs, without deviating from the concept of the present disclosure, various simple deductions or replacements may be made, which should be regarded as falling within the scope of the present disclosure.

## Claims

**1.** A compound of formula (I):

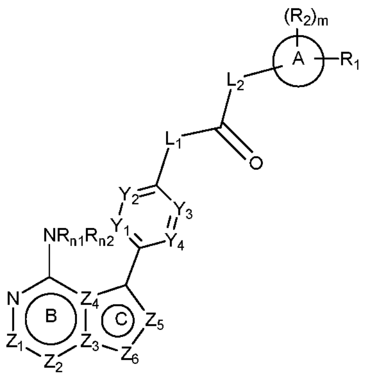

(I)

wherein

• ring A is a 5-membered heteroaryl group containing 1 to 3 heteroatoms selected from N, O and S;

$R_1$ is -C($R_{a1}$)($R_{a2}$)($R_{a3}$), wherein $R_{a1}$, $R_{a2}$ and $R_{a3}$ are each independently selected from H, D, halogen, $C_{1-6}$ alkyl, $C_{1-6}$ haloalkyl, $C_{3-7}$ cycloalkyl, 3- to 7-membered heterocyclyl, $C_{1-6}$ alkoxy, $C_{1-6}$ haloalkoxy, -$OC_{3-7}$ cycloalkyl, and -O-3- to 7-membered heterocyclyl, wherein the $C_{1-6}$ alkyl, $C_{1-6}$ haloalkyl, $C_{3-7}$ cycloalkyl, 3- to 7-membered heterocyclyl, $C_{1-6}$ alkoxy, $C_{1-6}$ haloalkoxy, - $OC_{3-7}$ cycloalkyl, and the -O-3- to 7-membered heterocyclyl are optionally substituted with one or more R;
each instance of $R_2$ is independently H, D, -OH, halogen, -CN, -$NO_2$, -$R_a$, -C(O)$R_a$, -C(O)O$R_a$, -C(O)N$R_b$$R_c$, -N$R_b$$R_c$, -N$R_a$C(O)$R_b$, -N$R_a$C(O)O$R_b$, -N$R_a$C(O)N$R_b$$R_c$, -ORa, -OC(O)$R_a$,-OC(O)O$R_a$, or -OC(O)N$R_b$$R_c$;
m is selected from 0, 1, 2, and 3;

• $L_1$ is selected from bond, O, S, N$R_{L1}$, and C($R_{L1}$)$_2$,
• $L_2$ is selected from bond, O, S, N$R_{L2}$, and C($R_{L2}$)$_2$,
wherein each instance of $R_{L1}$ and $R_{L2}$ is independently selected from H, D, halogen, $C_{1-6}$ alkyl, and $C_{1-6}$ haloalkyl, wherein the $C_{1-6}$ alkyl and $C_{1-6}$ haloalkyl are optionally substituted with one or more R;
• $Y_1$, $Y_2$, $Y_3$ and $Y_4$ are each independently selected from C$R_Y$ and N; wherein $R_Y$ is independently selected from H, D, -OH, halogen, -CN, -$NO_2$, -$R_a$, -C(O)$R_a$,-C(O)O$R_a$, -C(O)N$R_b$$R_c$, -N$R_b$$R_c$, -N$R_a$C(O)$R_b$, -N$R_a$C(O)O$R_b$, -N$R_a$C(O)N$R_b$$R_c$, -ORa,-OC(O)$R_a$, -OC(O)O$R_a$, and -OC(O)N$R_b$$R_c$;
• ring B and ring C form an aromatic fused ring;

$R_{n1}$ and $R_{n2}$ are each independently selected from H, D, $C_{1-6}$ alkyl, $C_{1-6}$ haloalkyl, $C_{2-6}$ alkenyl and $C_{2-6}$ alkynyl;
$Z_1$ is selected from CRzi and N;
$Z_2$ is selected from C$R_{Z2}$ and N;
$Z_3$ and $Z_4$ are each independently selected from N atom and C atom;
$Z_5$ is selected from N atom and C atom, which are optionally substituted with $R_{Z5}$;
$Z_6$ is selected from N atom and C atom, which are optionally substituted with $R_{Z6}$;
wherein Rzi, $R_{Z2}$, $R_{Z5}$ and $R_{Z6}$ are each independently selected from H, D, -OH, halogen, -CN,-NO2, -$R_a$, -C(O)$R_a$, -C(O)O$R_a$, -C(O)N$R_b$$R_c$, -N$R_b$$R_c$, -N$R_a$C(O)$R_b$, -N$R_a$C(O)O$R_b$,-N$R_a$C(O)N$R_b$$R_c$, -O$R_a$, -OC(O)$R_a$, -OC(O)O$R_a$, and -OC(O)N$R_b$$R_c$;

• each of $R_a$, $R_b$ and $R_c$ is independently selected from H, D, $C_{1-6}$ alkyl, $C_{1-6}$ haloalkyl, $C_{2-6}$ alkenyl and $C_{2-6}$ alkynyl, or $R_b$ and $R_c$ together with the nitrogen atom to which they are attached form a 3- to 7-membered heterocyclyl or 5- to 10-membered heteroaryl group, wherein the group is optionally substituted with one or more R;

each R is independently selected from H, D, -OH, -$NH_2$, halogen, -CN, -$R_d$, -C(O)$R_d$, -C(O)O$R_d$, -C(O)N$R_e$$R_f$, -N$R_e$$R_f$, -N$R_a$C(O)$R_e$, -N$R_d$C(O)O$R_e$, -N$R_d$C(O)N$R_e$$R_f$, -O$R_d$, -OC(O)$R_d$,-OC(O)O$R_d$, and -OC(O)N$R_e$$R_f$, or two R groups on the same atom or adjacent atoms can together form a $C_{3-7}$ cycloalkyl, 3- to 7-membered heterocyclyl, $C_{6-10}$ aryl or 5- to 10-membered heteroaryl group, wherein each group in the definition of R is optionally substituted with one or more D until fully deuterated;
each of $R_d$, $R_e$ and $R_f$ is independently selected from $C_{1-6}$ alkyl, $C_{1-6}$ haloalkyl, $C_{2-6}$ alkenyl and $C_{2-6}$ alkynyl, or $R_e$ and $R_f$ together with the nitrogen atom to which they are attached form a 3-to 7-membered heterocyclyl or 5- to 10-membered heteroaryl group, wherein each group in the definition of $R_d$, $R_e$ and $R_f$ is optionally substituted with one or more D until fully deuterated; or a tautomer, stereoisomer, prodrug, crystal form, pharmaceutically acceptable salt, hydrate or solvate thereof.

**2.** The compound, or the tautomer, stereoisomer, prodrug, crystal form, pharmaceutically acceptable salt, hydrate or solvate thereof of claim 1, wherein:

the ring A is selected from pyrrolyl, pyrazolyl, imidazolyl, furyl, oxazolyl, isoxazolyl, thienyl, thiazolyl and isothiazolyl;
alternatively, the ring A is selected from

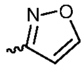, 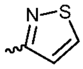, 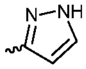, 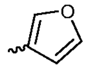, 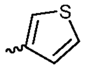 and 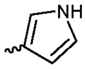;

alternatively selected from

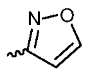, 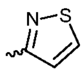 and 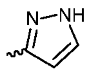;

alternatively

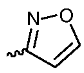.

3. The compound, or the tautomer, stereoisomer, prodrug, crystal form, pharmaceutically acceptable salt, hydrate or solvate thereof of claim 1 or 2, wherein:

$R_1$ is $-C(R_{a1})(R_{a2})(R_{a3})$, wherein $R_{a1}$, $R_{a2}$ and $R_{a3}$ are each independently selected from H, D, halogen, $C_{1-6}$ alkyl, and $C_{1-6}$ haloalkyl, wherein $C_{1-6}$ alkyl and $C_{1-6}$ haloalkyl are optionally substituted with one or more R; alternatively, wherein $R_{a1}$ is $CF_3$;
alternatively, $R_1$ is $C_{1-6}$ alkyl or $C_{1-6}$ haloalkyl, which is optionally substituted with one or more R.

4. The compound, or the tautomer, stereoisomer, prodrug, crystal form, pharmaceutically acceptable salt, hydrate or solvate thereof of any one of claims 1-3, wherein:
$L_1$ is selected from bond, O, S, NH and $CH_2$, and $L_2$ is selected from bond, O, S, NH and $CH_2$; alternatively, $L_1$ is selected from O, S, NH and $CH_2$, and $L_2$ is selected from bond, O, S and NH; alternatively, $L_1$ is selected from NH and $CH_2$, and $L_2$ is NH.

5. The compound, or the tautomer, stereoisomer, prodrug, crystal form, pharmaceutically acceptable salt, hydrate or solvate thereof of any one of claims 1-4, wherein:

$Y_1$, $Y_2$, and $Y_3$ are each independently $CR_Y$, and $Y_4$ is selected from $CR_Y$ and N;
alternatively, $Y_1$ and $Y_3$ are CH; $Y_2$ is $CR_Y$; and $Y_4$ is selected from $CR_Y$ and N.

6. The compound, or the tautomer, stereoisomer, prodrug, crystal form, pharmaceutically acceptable salt, hydrate or solvate thereof of any one of claims 1-5, wherein:

$Z_1$ is $CR_{Z1}$, wherein $RZ_1$ is selected from H, D, -OH, halogen, -CN, $-NO_2$ and $-R_a$;
$Z_4$ is C atom.

7. The compound, or the tautomer, stereoisomer, prodrug, crystal form, pharmaceutically acceptable salt, hydrate or solvate thereof of any one of claims 1-6, wherein:
$R_{Z6}$ is $C_{1-6}$ alkyl, or $C_{1-6}$ haloalkyl, alternatively $C_{1-6}$ alkyl, still alternatively isopropyl.

8. The compound, or the tautomer, stereoisomer, prodrug, crystal form, pharmaceutically acceptable salt, hydrate or solvate thereof of any one of claims 1-7, wherein:
$R_{n1}$ and $R_{n2}$ are each independently selected from H and D.

9. The compound, or the tautomer, stereoisomer, prodrug, crystal form, pharmaceutically acceptable salt, hydrate or solvate thereof of claim 1, which is a compound of formula (II):

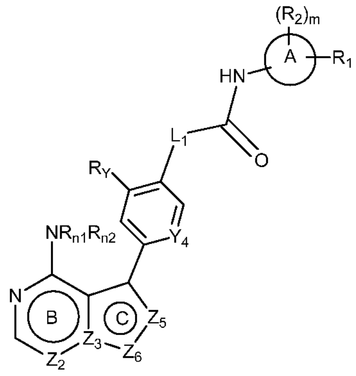

(II)

wherein

ring A is pyrrolyl, pyrazolyl, imidazolyl, furyl, oxazolyl, isoxazolyl, thienyl, thiazolyl or isothiazolyl;
$R_1$ is -C($R_{a1}$)($R_{a2}$)($R_{a3}$), wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
m is selected from 0, 1, 2, and 3;
$L_1$ is O, S, NH, ND, CHD, $CD_2$, or $CH_2$;
$R_Y$ is H, D, or halogen, alternatively fluorine;
$Y_4$ is CH, CD or N;
$Z_2$ is N or $CR_{Z2}$, wherein $R_{Z2}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
$Z_3$ is N or C;
$Z_5$ is N, $NR_{Z5}$, or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
Z6 is $NR_{Z6}$ or CRz6, wherein $R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{Z6}$ is $C_{1-4}$ alkyl, or $C_{1-4}$ haloalkyl;
$R_{n1}$ and $R_{n2}$ are each independently selected from H, D, $C_{1-6}$ alkyl, $C_{1-6}$ haloalkyl, $C_{2-6}$ alkenyl and $C_{2-6}$ alkynyl;
each R is independently selected from H, D, -OH, $-NH_2$, halogen, -CN, $C_{1-6}$ alkyl, and $C_{1-6}$ haloalkyl;
alternatively, wherein at most one of $Z_5$ and $Z_6$ is a substituted or unsubstituted carbon atom.

**10.** The compound, or the tautomer, stereoisomer, prodrug, crystal form, pharmaceutically acceptable salt, hydrate or solvate thereof of claim 9, which is a compound of formula (II-1):

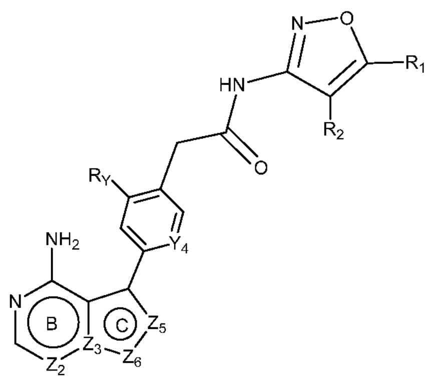

(II-1)

wherein

$R_1$ is -C($R_{a1}$)($R_{a2}$)($R_{a3}$), wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
$R_Y$ is H, D, or halogen, alternatively fluorine;
$Y_4$ is CH, CD or N;
$Z_2$ is N or $CR_{Z2}$, wherein $R_{Z2}$ is H, D, halogen, $C_{1-4}$ alkyl, or $C_{1-4}$ haloalkyl, wherein the $C_{1-4}$ alkyl and $C_{1-4}$ haloalkyl are optionally substituted with one or more R;
$Z_3$ is N or C;
$Z_5$ is N, $NR_{Z5}$, or $CR_{Z5}$, wherein $R_{Z5}$ is H, D, halogen, $C_{1-4}$ alkyl, or $C_{1-4}$ haloalkyl, wherein the $C_{1-4}$ alkyl and $C_{1-4}$ haloalkyl are optionally substituted with one or more R;
Z6 is $NR_{Z6}$ or $CR_{Z6}$, wherein $R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{Z6}$ is $C_{1-4}$ alkyl, or $C_{1-4}$ haloalkyl;
each R is independently selected from H, D, -OH, $-NH_2$, halogen, -CN, $C_{1-6}$ alkyl, and $C_{1-6}$ haloalkyl;
alternatively, wherein at most one of $Z_5$ and $Z_6$ is a substituted or unsubstituted carbon atom.

**11.** The compound, or the tautomer, stereoisomer, prodrug, crystal form, pharmaceutically acceptable salt, hydrate or solvate thereof of claim 9, which is a compound of formula (II-2):

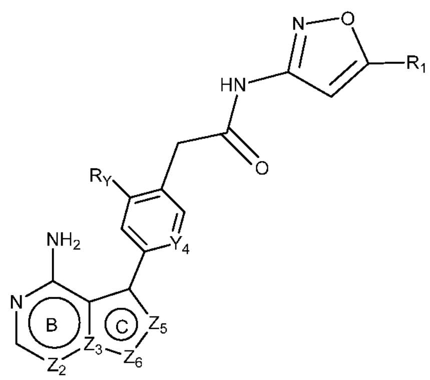

(II-2)

wherein

$R_1$ is -C($R_{a1}$)($R_{a2}$)($R_{a3}$), wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and Ra3 is haloalkyl;
$R_Y$ is H, D, or halogen, alternatively fluorine;
$Y_4$ is CH, CD or N;
$Z_2$ is N, CH or CD;
$Z_3$ is N or C;
$Z_5$ is N, NH, CH or CD;
Z6 is $NR_{Z6}$ or CRz6, wherein $R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{Z6}$ is $C_{1-4}$ alkyl, or $C_{1-4}$ haloalkyl;
each R is independently selected from H, D, -OH, $-NH_2$, halogen, -CN, $C_{1-6}$ alkyl, and $C_{1-6}$ haloalkyl;
alternatively, wherein at most one of $Z_5$ and $Z_6$ is a substituted or unsubstituted carbon atom.

**12.** The compound, or the tautomer, stereoisomer, prodrug, crystal form, pharmaceutically acceptable salt, hydrate or solvate thereof of claim 9, which is a compound of formula (II-3):

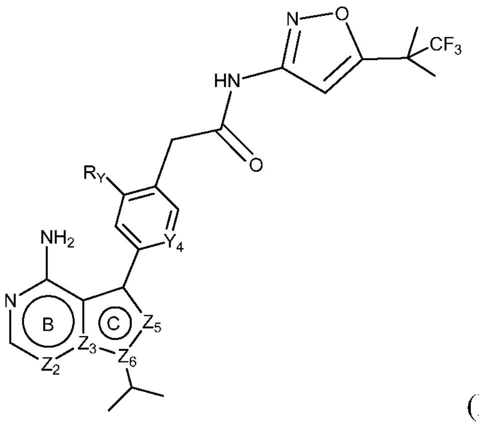

(II-3)

wherein

$R_Y$ is H, D, or halogen, alternatively fluorine;
$Y_4$ is CH, CD or N;
$Z_2$ is N, CH or CD;
$Z_3$ is N or C;
$Z_5$ is N, CH or CD;
$Z_6$ is N or C;
alternatively, wherein at most one of $Z_5$ and $Z_6$ is a substituted or unsubstituted carbon atom.

**13.** The compound, or the tautomer, stereoisomer, prodrug, crystal form, pharmaceutically acceptable salt, hydrate or solvate thereof of claim 9, which is a compound of formula (II-4-1) or formula (II-4-2):

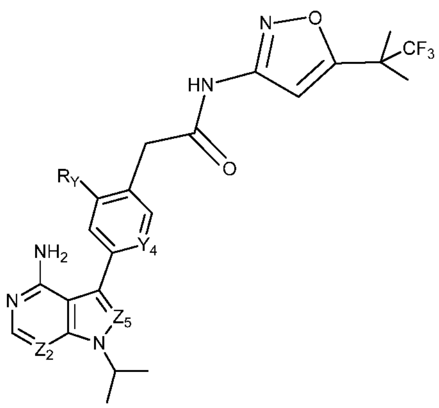

(II-4-1) or

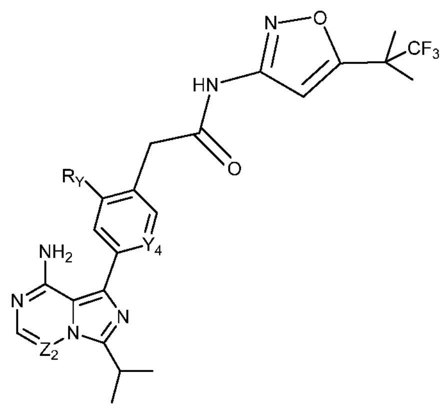

(II-4-2)

wherein

$R_Y$ is H or halogen, alternatively fluorine;
$Y_4$ is CH or N;
$Z_2$ is CH or N;
$Z_5$ is CH or N.

**14.** The compound, or the tautomer, stereoisomer, prodrug, crystal form, pharmaceutically acceptable salt, hydrate or solvate thereof of claim 1, which is a compound of formula (III):

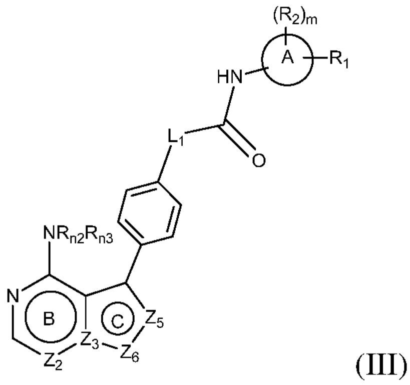

(III)

wherein

ring A is pyrrolyl, pyrazolyl, imidazolyl, furyl, oxazolyl, isoxazolyl, thienyl, thiazolyl or isothiazolyl;

$R_1$ is -C($R_{a1}$)($R_{a2}$)($R_{a3}$), wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and Ra3 is haloalkyl;

$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;

m is selected from 0, 1, 2, and 3;

$L_1$ is O, S, NH, ND, CHD, $CD_2$, or $CH_2$;

$Z_2$ is N or $CR_{Z2}$, wherein $R_{Z2}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;

$Z_3$ is N or C;

$Z_5$ is N, $NR_{Z5}$, or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;

$Z_6$ is $NR_{Z6}$ or $CR_{Z6}$, wherein $R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{Z6}$ is $C_{1-4}$ alkyl, or $C_{1-4}$ haloalkyl;

$R_{n1}$ and $R_{n2}$ are each independently selected from H, D, $C_{1-6}$ alkyl, $C_{1-6}$ haloalkyl, $C_{2-6}$ alkenyl and $C_{2-6}$ alkynyl;

each R is independently selected from H, D, -OH, $-NH_2$, halogen, -CN, $C_{1-6}$ alkyl, and $C_{1-6}$ haloalkyl;

wherein at most one of $Z_5$ and $Z_6$ is a substituted or unsubstituted nitrogen atom.

**15.** The compound, or the tautomer, stereoisomer, prodrug, crystal form, pharmaceutically acceptable salt, hydrate or solvate thereof of claim 14, which is a compound of formula (III-1):

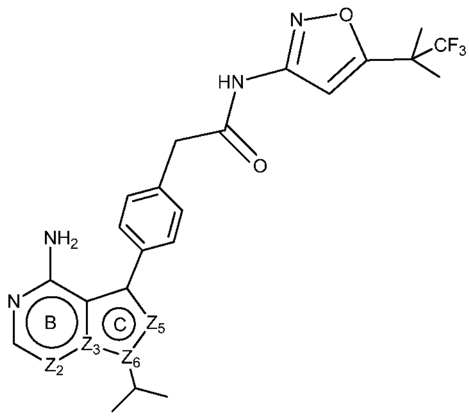

(III-1)

wherein

$Z_2$ is N or CH;

$Z_3$ is N or C;
$Z_5$ is N, NH, or CH;
$Z_6$ is N or C;
wherein at most one of $Z_5$ and $Z_6$ is a nitrogen atom.

**16.** The compound, or the tautomer, stereoisomer, prodrug, crystal form, pharmaceutically acceptable salt, hydrate or solvate thereof of claim 1, which is a compound of formula (IV):

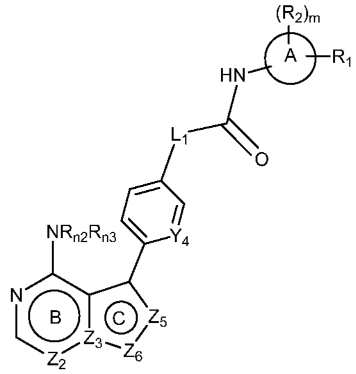

(IV)

wherein

ring A is pyrrolyl, pyrazolyl, imidazolyl, furyl, oxazolyl, isoxazolyl, thienyl, thiazolyl or isothiazolyl;
$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and
Ra3 is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
m is selected from 0, 1, 2, and 3;
$L_1$ is O, S, NH, ND, CHD, $CD_2$, or $CH_2$;
$Y_4$ is CH, CD or N;
$Z_2$ is N or $CR_{Z2}$, wherein $R_{Z2}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
$Z_3$ is N or C;
$Z_5$ is N, $NR_{Z5}$, or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
Z6 is $NR_{Z6}$ or CRz6, wherein $R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{Z6}$ is $C_{1-4}$ alkyl, or $C_{1-4}$ haloalkyl;
$R_{n1}$ and $R_{n2}$ are each independently selected from H, D, $C_{1-6}$ alkyl, $C_{1-6}$ haloalkyl, $C_{2-6}$ alkenyl and $C_{2-6}$ alkynyl;
each R is independently selected from H, D, -OH, $-NH_2$, halogen, -CN, $C_{1-6}$ alkyl, and $C_{1-6}$ haloalkyl;
wherein at most one of $Z_5$ and $Z_6$ is a substituted or unsubstituted carbon atom; and when both of $Z_5$ and $Z_6$ are a substituted or unsubstituted nitrogen atom, $Y_4$ is N.

**17.** The compound, or the tautomer, stereoisomer, prodrug, crystal form, pharmaceutically acceptable salt, hydrate or solvate thereof of claim 16, which is a compound of formula (IV-1):

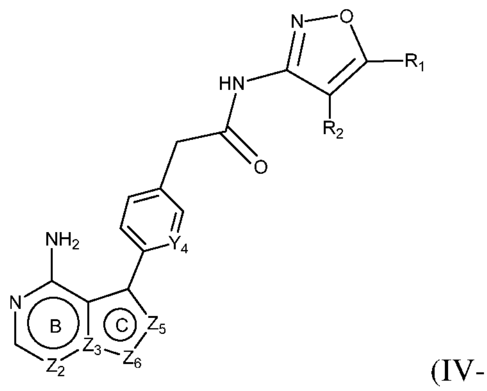

(IV-1)

wherein

$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and Ra3 is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
$Y_4$ is CH, CD, or N;
$Z_2$ is N or $CR_{Z2}$, wherein $R_{Z2}$ is H, D, halogen, $C_{1-4}$ alkyl, or $C_{1-4}$ haloalkyl, wherein the $C_{1-4}$ alkyl and $C_{1-4}$ haloalkyl are optionally substituted with one or more R;
$Z_3$ is N or C;
$Z_5$ is N, $NR_{Z5}$, or $CR_{Z5}$, wherein $R_{Z5}$ is H, D, halogen, $C_{1-4}$ alkyl, or $C_{1-4}$ haloalkyl, wherein the $C_{1-4}$ alkyl and $C_{1-4}$ haloalkyl are optionally substituted with one or more R;
$Z_6$ is $NR_{Z6}$ or $CR_{Z6}$, wherein $R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{Z6}$ is $C_{1-4}$ alkyl, or $C_{1-4}$ haloalkyl;
each R is independently selected from H, D, -OH, $-NH_2$, halogen, -CN, $C_{1-6}$ alkyl, and $C_{1-6}$ haloalkyl;
wherein at most one of $Z_5$ and $Z_6$ is a substituted or unsubstituted carbon atom; and when both of $Z_5$ and $Z_6$ are a substituted or unsubstituted nitrogen atom, $Y_4$ is N.

**18.** The compound, or the tautomer, stereoisomer, prodrug, crystal form, pharmaceutically acceptable salt, hydrate or solvate thereof of claim 16, which is a compound of formula (IV-2):

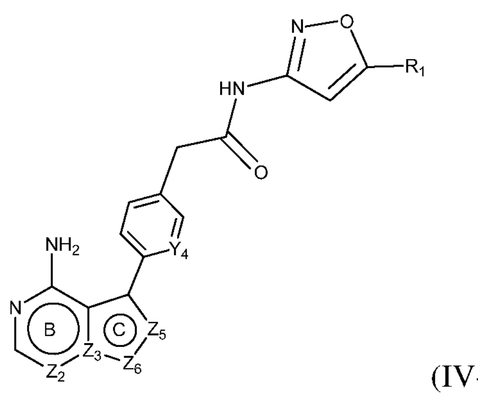

(IV-2)

wherein

$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and Ra3 is haloalkyl;
$Y_4$ is CH, CD, or N;
$Z_2$ is N or CH;
$Z_3$ is N or C;
$Z_5$ is N, NH, or CH;
$Z_6$ is $NR_{Z6}$ or $CR_{Z6}$, wherein $R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{Z6}$ is $C_{1-4}$ alkyl, or $C_{1-4}$ haloalkyl;
each R is independently selected from H, D, -OH, $-NH_2$, halogen, -CN, $C_{1-6}$ alkyl, and $C_{1-6}$ haloalkyl;
wherein at most one of $Z_5$ and $Z_6$ is a substituted or unsubstituted carbon atom; and when both of $Z_5$ and $Z_6$ are a substituted or unsubstituted nitrogen atom, $Y_4$ is N.

**19.** The compound, or the tautomer, stereoisomer, prodrug, crystal form, pharmaceutically acceptable salt, hydrate or solvate thereof of claim 16, which is a compound of formula (IV-3):

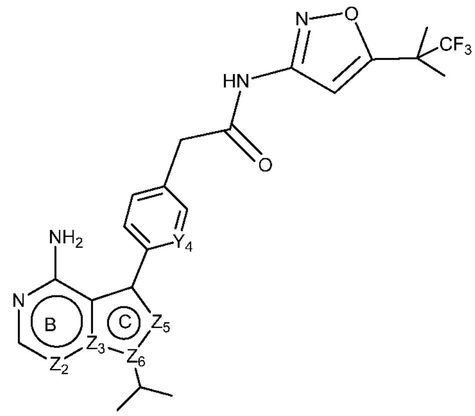

(IV-3)

wherein

$Y_4$ is CH, CD or N;
$Z_2$ is N or CH;
$Z_3$ is N or C;
$Z_5$ is N, NH, or CH;
$Z_6$ is N or C;
wherein at most one of $Z_5$ and $Z_6$ is a substituted or unsubstituted carbon atom; and when both of $Z_5$ and $Z_6$ are a substituted or unsubstituted nitrogen atom, $Y_4$ is N.

**20.** The compound, or the tautomer, stereoisomer, prodrug, crystal form, pharmaceutically acceptable salt, hydrate or solvate thereof of any one of claims 16-19, wherein only one of $Z_5$ and $Z_6$ is a substituted or unsubstituted carbon atom.

**21.** The compound, or the tautomer, stereoisomer, prodrug, crystal form, pharmaceutically acceptable salt, hydrate or solvate thereof of claim 1, which is a compound of formula (V):

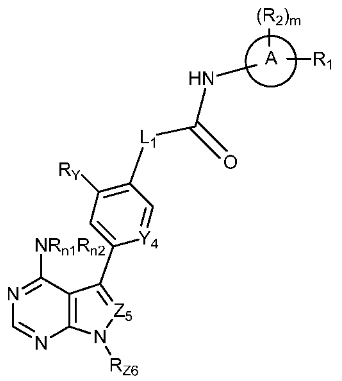

(V)

wherein

ring A is pyrrolyl, pyrazolyl, imidazolyl, furyl, oxazolyl, isoxazolyl, thienyl, thiazolyl or isothiazolyl;
$R_1$ is -C($R_{a1}$)($R_{a2}$)($R_{a3}$), wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
m is selected from 0, 1, 2, and 3;
$L_1$ is O, S, NH, ND, CHD, $CD_2$, or $CH_2$;
$Y_4$ is N or $CR_Y$,
$R_Y$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_Y$ is H or halogen; still alternatively fluorine;
$R_{n1}$ and $R_{n2}$ are each independently selected from H, D, $C_{1-6}$ alkyl, $C_{1-6}$ haloalkyl, $C_{2-6}$ alkenyl and $C_{2-6}$ alkynyl;
$R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{Z6}$ is $C_{1-4}$ alkyl, or $C_{1-4}$ haloalkyl;
$Z_5$ is N or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
each R is independently selected from H, D, -OH, $-NH_2$, halogen, -CN, $C_{1-6}$ alkyl, and $C_{1-6}$ haloalkyl.

**22.** The compound, or the tautomer, stereoisomer, prodrug, crystal form, pharmaceutically acceptable salt, hydrate or solvate thereof of claim 21, which is a compound of formula (V-1) or (V-1'):

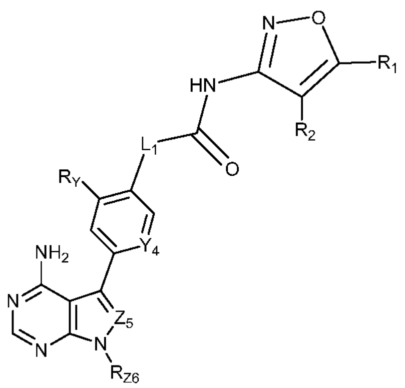

(V-1) or

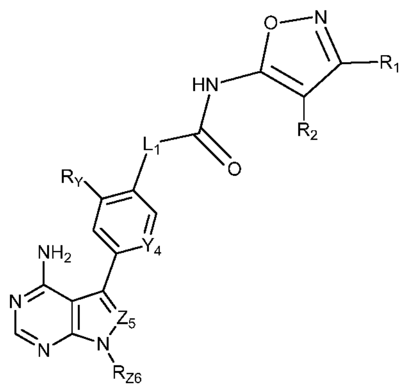

(V-1')

wherein

$R_1$ is -C($R_{a1}$)($R_{a2}$)($R_{a3}$), wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally

substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
$L_1$ is O, S, NH, ND, CHD, $CD_2$, or $CH_2$;
$Y_4$ is N or $CR_Y$,
$R_Y$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_Y$ is H or halogen; still alternatively fluorine;
$R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R, alternatively, $R_{Z6}$ is $C_{1-4}$ alkyl, or $C_{1-4}$ haloalkyl;
$Z_5$ is N or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
each R is independently selected from H, D, -OH, $-NH_2$, halogen, -CN, $C_{1-6}$ alkyl, and $C_{1-6}$ haloalkyl.

**23.** The compound, or the tautomer, stereoisomer, prodrug, crystal form, pharmaceutically acceptable salt, hydrate or solvate thereof of claim 21, which is a compound of formula (V-2) or (V-2'):

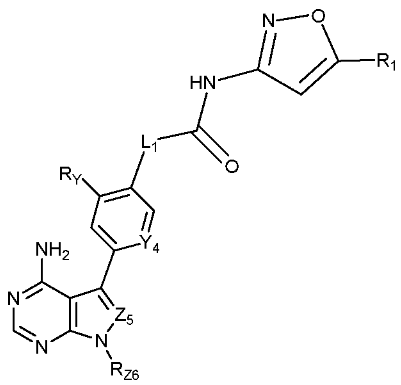

(V-2) or

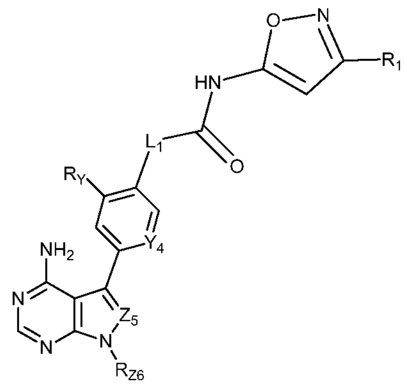

(V-2')

wherein

$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$L_1$ is NH, ND, CHD, $CD_2$, or $CH_2$;
$Y_4$ is N, CD, or CH;
$R_Y$ is H, D, or halogen, alternatively fluorine;
$R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{Z6}$ is $C_{1-4}$ alkyl, or $C_{1-4}$ haloalkyl;
$Z_5$ is N, CD, or CH;
each R is independently selected from H, D, -OH, $-NH_2$, halogen, -CN, $C_{1-6}$ alkyl, and $C_{1-6}$ haloalkyl.

**24.** The compound, or the tautomer, stereoisomer, prodrug, crystal form, pharmaceutically acceptable salt, hydrate or solvate thereof of claim 21, which is a compound of formula (V-3) or (V-3'):

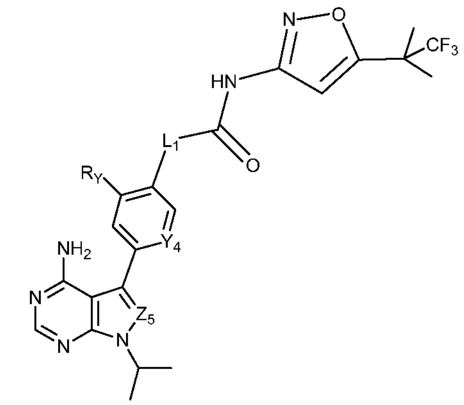

(V-3) or

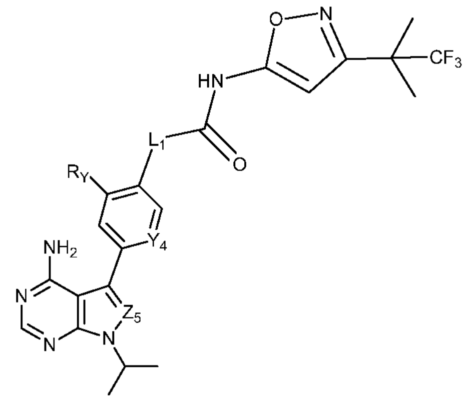

(V-3')

wherein

$L_1$ is NH or $CH_2$;
$Y_4$ is N or CH;
$R_Y$ is H or halogen, alternatively fluorine;
$Z_5$ is N or CH.

**25.** The compound, or the tautomer, stereoisomer, prodrug, crystal form, pharmaceutically acceptable salt, hydrate or solvate thereof of any one of claims 21-24, wherein $Z_5$ is N.

**26.** The compound, or the tautomer, stereoisomer, prodrug, crystal form, pharmaceutically acceptable salt, hydrate or solvate thereof of claim 1, which is a compound of formula (VI):

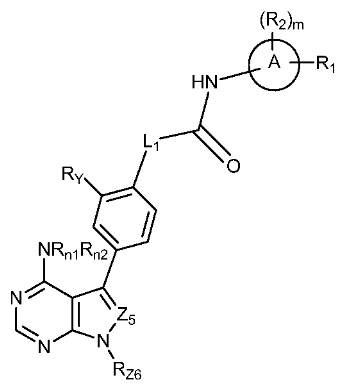

(VI)

wherein

ring A is pyrrolyl, pyrazolyl, imidazolyl, furyl, oxazolyl, isoxazolyl, thienyl, thiazolyl or isothiazolyl;
$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
m is selected from 0, 1, 2, and 3;
$L_1$ is O, S, NH, ND, CHD, $CD_2$, or $CH_2$;
$R_Y$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_Y$ is H or halogen; still alternatively fluorine;
$R_{n1}$ and $R_{n2}$ are each independently selected from H, D, $C_{1-6}$ alkyl, $C_{1-6}$ haloalkyl, $C_{2-6}$ alkenyl and $C_{2-6}$ alkynyl;
$R_{Z6}$ is $C_{1-6}$ alkyl, or $C_{1-6}$ haloalkyl, wherein the $C_{1-6}$ alkyl and $C_{1-6}$ haloalkyl are optionally substituted with one

or more R; alternatively, $R_{Z6}$ is $C_{1-4}$ alkyl, or $C_{1-4}$ haloalkyl;
$Z_5$ is N or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
each R is independently selected from H, D, -OH, $-NH_2$, halogen, -CN, $C_{1-6}$ alkyl, and $C_{1-6}$ haloalkyl.

**27.** The compound, or the tautomer, stereoisomer, prodrug, crystal form, pharmaceutically acceptable salt, hydrate or solvate thereof of claim 26, which is a compound of formula (VI-1):

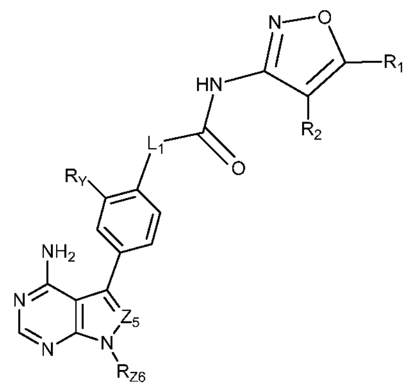

(VI-1)

wherein

$R_1$ is $-C(R_{a1})(R_{a2})(R_{a3})$, wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and $R_{a3}$ is haloalkyl;
$R_2$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
$L_1$ is O, S, NH, ND, CHD, $CD_2$, or $CH_2$;
$R_Y$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_Y$ is H or halogen; still alternatively fluorine;
$R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{Z6}$ is $C_{1-4}$ alkyl, or $C_{1-4}$ haloalkyl;
$Z_5$ is N or $CR_{Z5}$, wherein $R_{Z5}$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R;
each R is independently selected from H, D, -OH, $-NH_2$, halogen, -CN, $C_{1-6}$ alkyl, and $C_{1-6}$ haloalkyl.

**28.** The compound, or the tautomer, stereoisomer, prodrug, crystal form, pharmaceutically acceptable salt, hydrate or solvate thereof of claim 26, which is a compound of formula (VI-2):

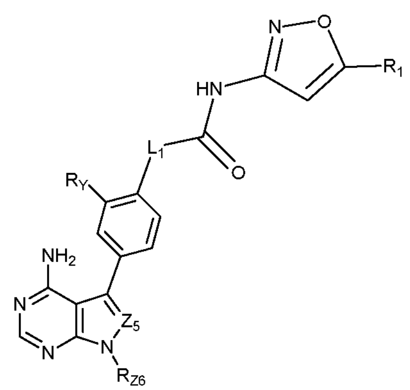

(VI-2)

wherein

$R_1$ is -C($R_{a1}$)($R_{a2}$)($R_{a3}$), wherein $R_{a1}$, $R_{a2}$ and $R_{a3}$ are independently H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_{a1}$, $R_{a2}$ and $R_{a3}$ are independently $C_{1-4}$ alkyl, or $C_{1-4}$ haloalkyl, wherein the $C_{1-4}$ alkyl and $C_{1-4}$ haloalkyl are optionally substituted with one or more R; alternatively, $R_{a1}$, $R_{a2}$ and $R_{a3}$ are independently methyl or halomethyl; alternatively, at least one of $R_{a1}$, $R_{a2}$ and Ra3 is haloalkyl;
$L_1$ is NH, ND, CHD, $CD_2$, or $CH_2$;
$R_Y$ is H, D, halogen, $C_{1-6}$ alkyl, or $C_{1-6}$ haloalkyl, wherein the $C_{1-6}$ alkyl and $C_{1-6}$ haloalkyl are optionally substituted with one or more R; alternatively, $R_Y$ is H or halogen; still alternatively fluorine;
$R_{Z6}$ is $C_{1-6}$ alkyl, or $C_{1-6}$ haloalkyl, wherein the $C_{1-6}$ alkyl and $C_{1-6}$ haloalkyl are optionally substituted with one or more R, alternatively, $R_{Z6}$ is $C_{1-4}$ alkyl, or $C_{1-4}$ haloalkyl;
$Z_5$ is N, CD, or CH;
each R is independently selected from H, D, -OH, $-NH_2$, halogen, -CN, $C_{1-6}$ alkyl, and $C_{1-6}$ haloalkyl.

**29.** The compound, or the tautomer, stereoisomer, prodrug, crystal form, pharmaceutically acceptable salt, hydrate or solvate thereof of claim 26, which is a compound of formula (VI-3):

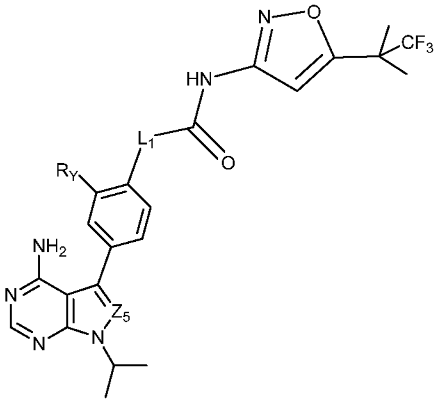

(VI-3)

wherein

$L_1$ is NH or $CH_2$;
$R_Y$ is H or halogen, alternatively fluorine;
$Z_5$ is N or CH.

**30.** The compound, or the tautomer, stereoisomer, prodrug, crystal form, pharmaceutically acceptable salt, hydrate or solvate thereof of any one of claims 26-29, wherein $Z_5$ is N.

**31.** The compound, or the tautomer, stereoisomer, prodrug, crystal form, pharmaceutically acceptable salt, hydrate or solvate thereof of claim 1, wherein the compound is selected from:

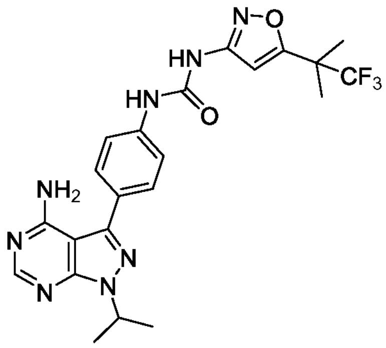
,
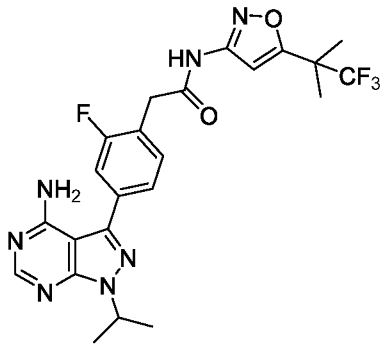
,
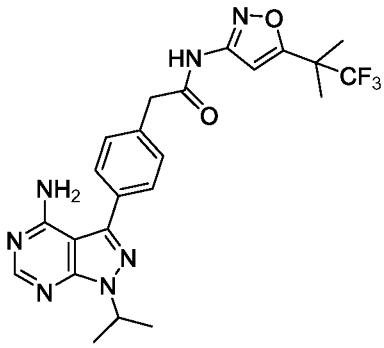
,

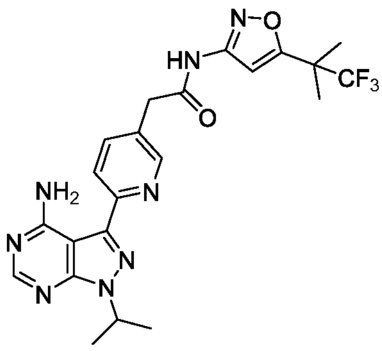,
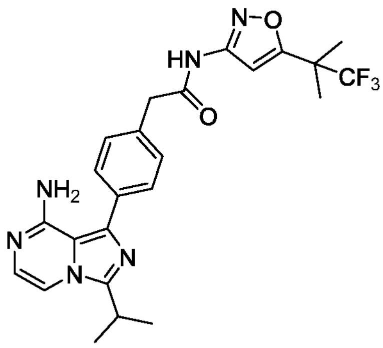,
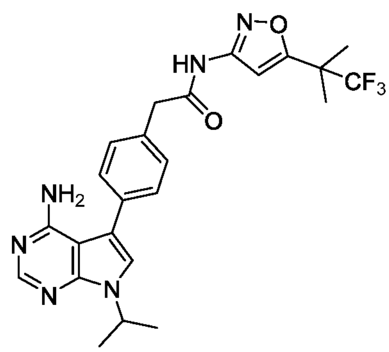,
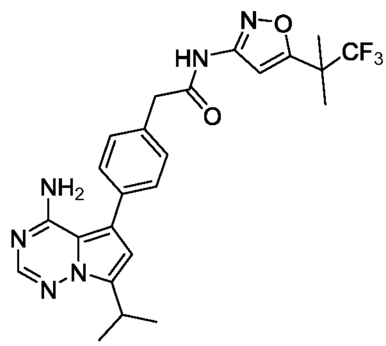,
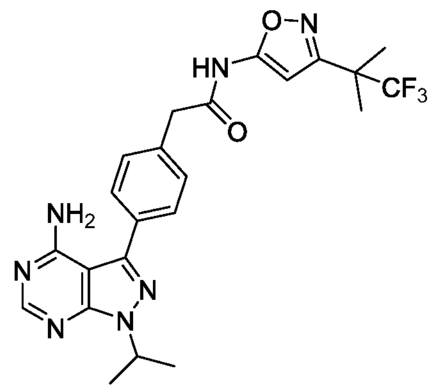,
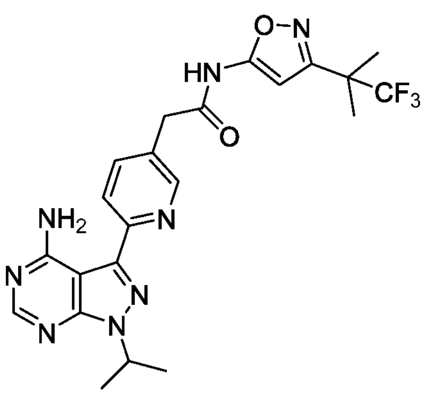 and 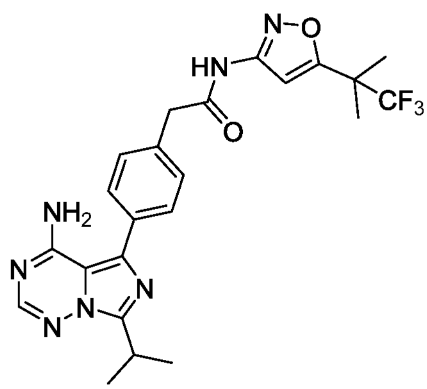.

**32.** A pharmaceutical composition containing the compound or the tautomer, stereoisomer, prodrug, crystal form, pharmaceutically acceptable salt, hydrate or solvate thereof of any one of claims 1-31, and pharmaceutically acceptable excipient (s).

**33.** Use of the compound, or the tautomer, stereoisomer, prodrug, crystal form, pharmaceutically acceptable salt, hydrate or solvate thereof of any one of claims 1-31, or the pharmaceutical composition of claim 32 in the manufacture of a medicament for the treatment and/or prevention of a disease.

**34.** The use of claim 33, wherein the disease is a disease mediated by protein kinases, such as diseases mediated by one or more kinases selected from wild-type and mutant RET, KIF5B-RET, CCDC6-RET, Trk, FLT3, FLT3-ITD, c-Kit, PDGFR and VEGFR kinases.

**35.** The use of claim 34, wherein the mutant RET, KIF5B-RET and CCDC6-RET kinases are selected from V804L, V804M, V804E, M918T, E805K, Y806C, Y806E, C634Y, C634W and G810R.

**36.** The use of claim 34, wherein the mutant Trk kinase is G595R.

**37.** The use of claim 34, wherein the mutant FLT3 and FLT3-ITD kinases are selected from F691L, D835Y, D835V, D835H, D835F, D835E, Y842C, Y842D, Y842H, Y842N and Y842S.

**38.** The use of claim 33, wherein the disease is selected from non-small cell lung cancer, papillary thyroid cancer, glioblastoma multiforme, acute myeloid leukemia, colorectal cancer, large cell neuroendocrine cancer, prostate cancer, colon cancer, acute lymphoblastic leukaemia, sarcoma, pediatric glioma, intrahepatic bile duct carcinoma, pilocytic astrocytoma, low-grade glioma, lung adenocarcinoma, salivary gland carcinoma, secretory breast cancer, fibrosarcoma, kidney cancer, breast cancer, myelodysplastic syndrome, gastrointestinal stromal tumor, melanoma, seminoma, intracranial germ cell tumor, and mediastinal B-cell lymphoma.

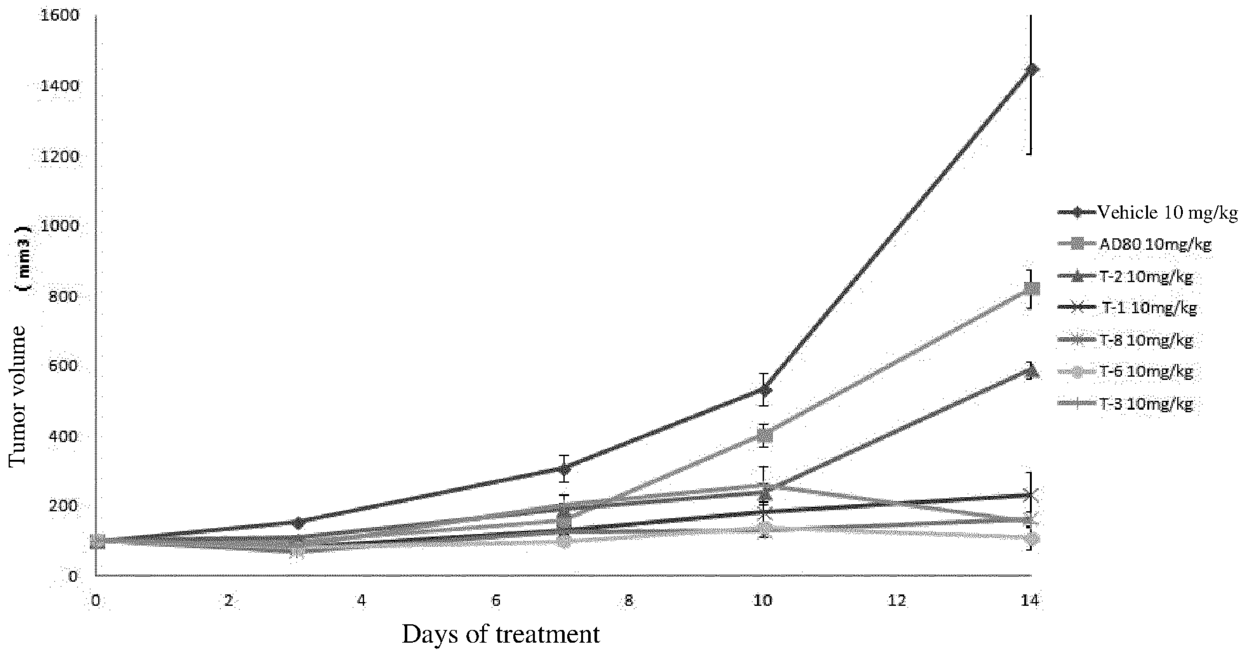
Tumor volume (mm3)
1600
1400
1200
1000
800
600
400
200
0
0
2
4
6
8
10
12
14
Days of treatment
Vehicle 10 mg/kg
AD80 10mg/kg
T-2 10mg/kg
T-1 10mg/kg
T-8 10mg/kg
T-6 10mg/kg
T-3 10mg/kg

FIG. 1

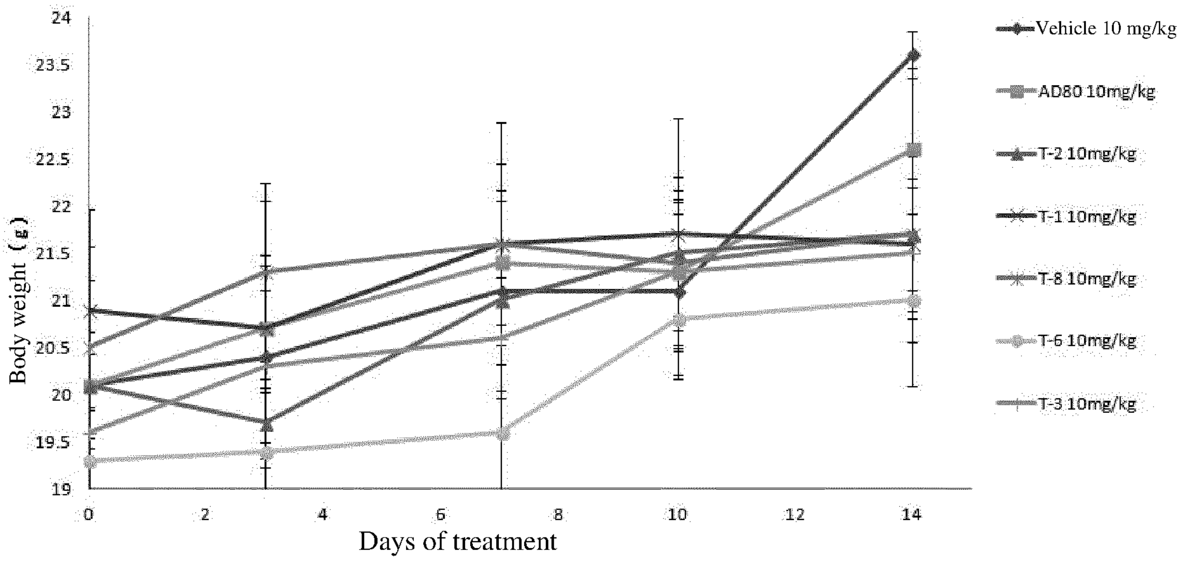
Body weight (g)
24
23.5
23
22.5
22
21.5
21
20.5
20
19.5
19
0
2
4
6
8
10
12
14
Days of treatment
Vehicle 10 mg/kg
AD80 10mg/kg
T-2 10mg/kg
T-1 10mg/kg
T-8 10mg/kg
T-6 10mg/kg
T-3 10mg/kg

FIG. 2

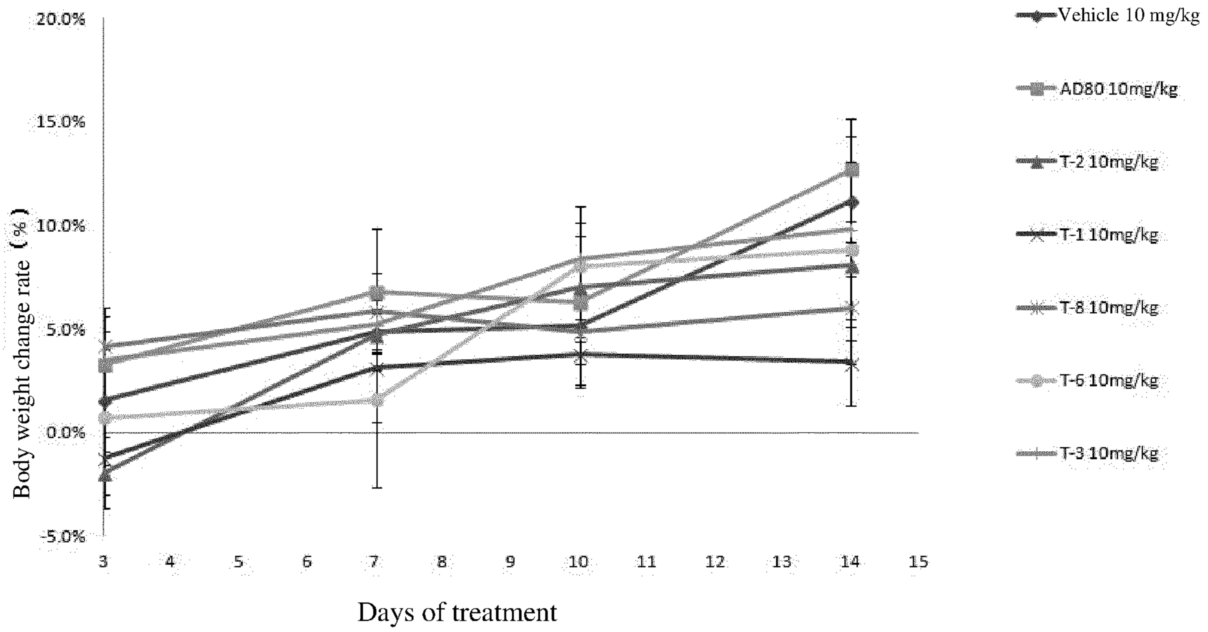
Vehicle 10 mg/kg
AD80 10mg/kg
T-2 10mg/kg
T-1 10mg/kg
T-8 10mg/kg
T-6 10mg/kg
T-3 10mg/kg
Body weight change rate（%）
20.0%
15.0%
10.0%
5.0%
0.0%
-5.0%
3
4
5
6
7
8
9
10
11
12
13
14
15
Days of treatment

FIG. 3

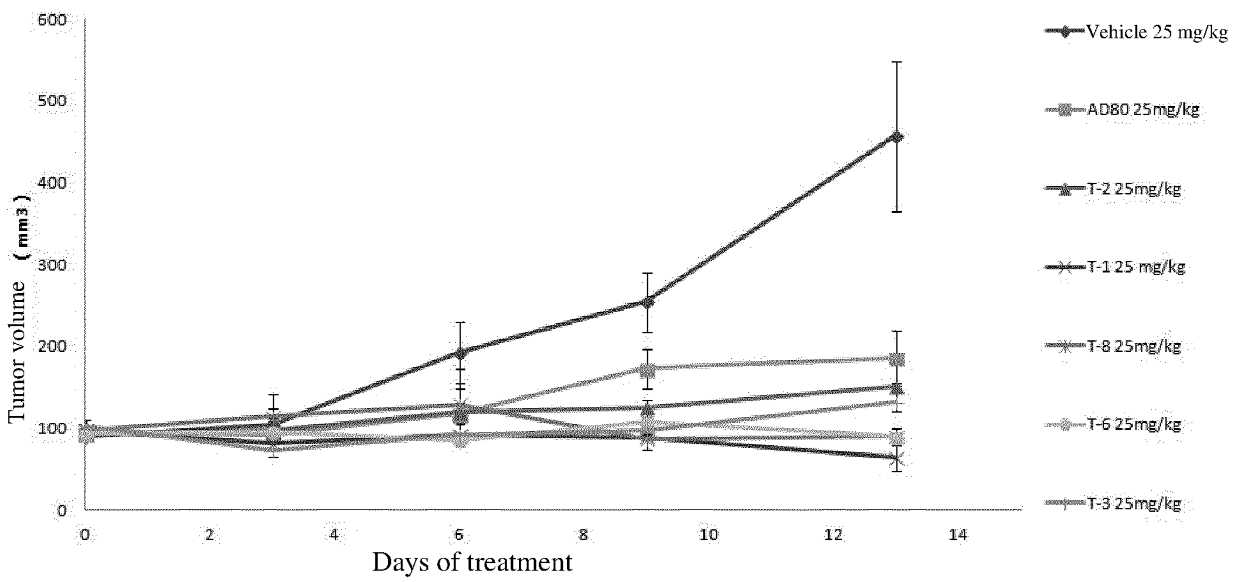
Vehicle 25 mg/kg
AD80 25mg/kg
T-2 25mg/kg
T-1 25 mg/kg
T-8 25mg/kg
T-6 25mg/kg
T-3 25mg/kg
Tumor volume（mm3）
600
500
400
300
200
100
0
0
2
4
6
8
10
12
14
Days of treatment

FIG. 4

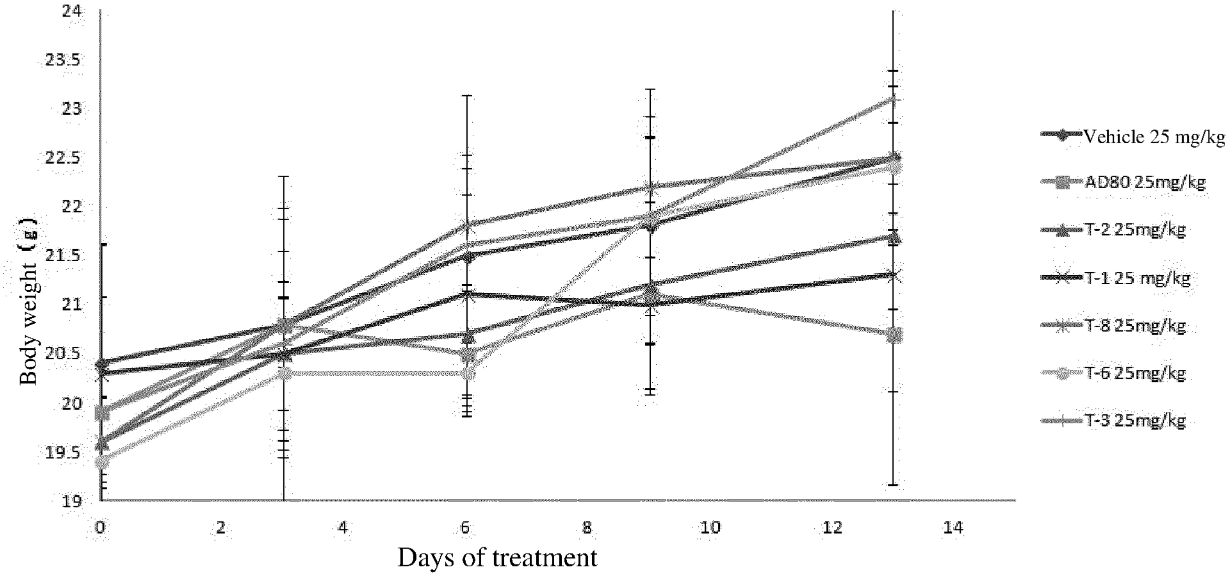
Body weight（g）
Days of treatment
24
23.5
23
22.5
22
21.5
21
20.5
20
19.5
19
0
2
4
6
8
10
12
14
Vehicle 25 mg/kg
AD80 25mg/kg
T-2 25mg/kg
T-1 25 mg/kg
T-8 25mg/kg
T-6 25mg/kg
T-3 25mg/kg

FIG. 5

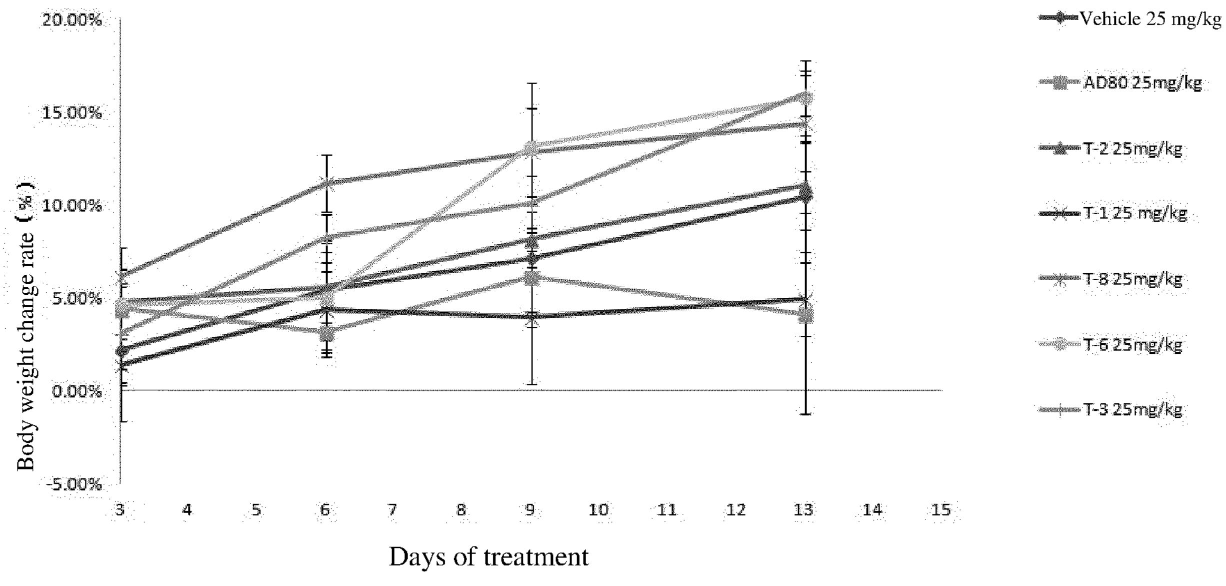
Body weight change rate（%）
Days of treatment
20.00%
15.00%
10.00%
5.00%
0.00%
-5.00%
3
4
5
6
7
8
9
10
11
12
13
14
15
Vehicle 25 mg/kg
AD80 25mg/kg
T-2 25mg/kg
T-1 25 mg/kg
T-8 25mg/kg
T-6 25mg/kg
T-3 25mg/kg

FIG. 6

## INTERNATIONAL SEARCH REPORT

International application No. **PCT/CN2020/117586**

### A. CLASSIFICATION OF SUBJECT MATTER

C07D 487/04(2006.01)i; A61K 31/41(2006.01)i; A61K 31/519(2006.01)i; A61K 31/5377(2006.01)i; A61P 35/00(2006.01)i; A61P 35/02(2006.01)i

According to International Patent Classification (IPC) or to both national classification and IPC

### B. FIELDS SEARCHED

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS; CNTXT; VEN; WOTXT; USTXT; EPTXT; CNKI; 万方; STN; ISI Web of Science: 塔吉瑞, 王义汉, 邢青峰, 艾义新, 李焕银, 蛋白激酶, 酪氨酸激酶, 癌, 瘤, 白血病, structure of claim 1, protein kinase, tyrosine kinase, cancer, tumor, leukemia

### C. DOCUMENTS CONSIDERED TO BE RELEVANT

| Category* | Citation of document, with indication, where appropriate, of the relevant passages | Relevant to claim No. |
|---|---|---|
| PX | CN 111646995 A (SICHUAN UNIVERSITY) 11 September 2020 (2020-09-11)<br>claims 1-21 | 1-38 |
| X | YUAN, Xue et al. "Identification of Pyrrolo[2, 3-d]pyrimidine-Based Derivatives as Potent and Orally Effective Fms-like Tyrosine Receptor Kinase 3(FLT3) Inhibitors for Treating Acute Myelogenous Leukemia"<br>*J. Med. Chem.*, Vol. 62, No. 8, 02 April 2019 (2019-04-02),<br>ISSN: 1520-4804,<br>abstract, page 4159 right-hand column paragraph 2 to page 4167 left-hand column paragraph 1 | 1-38 |
| X | CN 1993361 A (BAYER PHARMACEUTICALS CORP.) 04 July 2007 (2007-07-04)<br>description page 45 right-hand column paragraph 4 to page 49 paragraph 1, page 138 compound in embodiment 52 , page 210 compound in embodiment 161, page 293 compound in embodiment 284, page 294 compounds in embodiments 285, 286, page 295 compound in embodiment 287, page 296 compounds in embodiments 288, 289, page 297 compound in embodiment 291, page 332 paragraph 2 from the bottom to page 337 paragraph 2 from the bottom | 1-6, 8, 32-34, 38 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 November 2020** | **31 December 2020** |
| Name and mailing address of the ISA/CN<br>**China National Intellectual Property Administration (ISA/ CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/117586**

**C. DOCUMENTS CONSIDERED TO BE RELEVANT**

| Category* | Citation of document, with indication, where appropriate, of the relevant passages | Relevant to claim No. |
|---|---|---|
| A | CN 107176951 A (ANCUREALL PHARMACEUTICAL (SHANGHAI) CO., LTD.) 19 September 2017 (2017-09-19) entire document | 1-38 |

Form PCT/ISA/210 (second sheet) (January 2015)

**INTERNATIONAL SEARCH REPORT**
**Information on patent family members**

International application No.
**PCT/CN2020/117586**

| Patent document cited in search report | | | Publication date (day/month/year) | Patent family member(s) | | | Publication date (day/month/year) |
|---|---|---|---|---|---|---|---|
| CN | 111646995 | A | 11 September 2020 | None | | | |
| CN | 1993361 | A | 04 July 2007 | NZ | 551742 | A | 27 August 2010 |
| | | | | RU | 2401269 | C2 | 10 October 2010 |
| | | | | US | 8338595 | B2 | 25 December 2012 |
| | | | | US | 2013225809 | A1 | 29 August 2013 |
| | | | | SG | 127661 | B | 31 August 2009 |
| | | | | AU | 2005252217 | A1 | 04 January 2007 |
| | | | | ZA | 200700011 | A | 28 January 2009 |
| | | | | HK | 1109141 | A1 | 14 January 2011 |
| | | | | IN | 200606981 | P1 | 13 July 2007 |
| | | | | SG | 127661 | A1 | 30 January 2007 |
| | | | | WO | 2005121147 | A1 | 22 December 2005 |
| | | | | EP | 1765823 | A1 | 28 March 2007 |
| | | | | TW | 200608979 | A | 16 March 2006 |
| | | | | DE | 602005020293 | D1 | 12 May 2010 |
| | | | | MX | 279139 | B | 21 September 2010 |
| | | | | CA | 2569396 | C | 20 December 2011 |
| | | | | JP | 4958772 | B2 | 20 June 2012 |
| | | | | EP | 1765823 | B1 | 31 March 2010 |
| | | | | NO | 20070049 | A | 30 January 2007 |
| | | | | KR | 20070035028 | A | 29 March 2007 |
| | | | | US | 2007208013 | A1 | 06 September 2007 |
| | | | | MX | 2006013457 | A1 | 01 March 2007 |
| | | | | JP | 2008501703 | A | 24 January 2008 |
| | | | | PH | 12006502386 | B1 | 23 December 2010 |
| | | | | BR | 200510848 | A | 27 November 2007 |
| | | | | US | 7563791 | B2 | 21 July 2009 |
| | | | | US | 2010075958 | A1 | 25 March 2010 |
| | | | | CN | 1993361 | B | 26 May 2010 |
| | | | | KR | 101235602 | B1 | 21 February 2013 |
| | | | | ES | 2342722 | T3 | 13 July 2010 |
| CN | 107176951 | A | 19 September 2017 | WO | 2017152874 | A1 | 14 September 2017 |
| | | | | JP | 2019507779 | A | 22 March 2019 |
| | | | | EP | 3424924 | A4 | 07 August 2019 |
| | | | | US | 10647680 | B2 | 12 May 2020 |
| | | | | RU | 2018131539 | A | 13 April 2020 |
| | | | | KR | 20180132664 | A | 12 December 2018 |
| | | | | HK | 1258545 | A0 | 15 November 2019 |
| | | | | AU | 2017230437 | A1 | 06 September 2018 |
| | | | | BR | 112018016554 | A2 | 26 December 2018 |
| | | | | EP | 3424924 | A1 | 09 January 2019 |
| | | | | CN | 109071523 | A | 21 December 2018 |
| | | | | US | 2019047964 | A1 | 14 February 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5376645 A


### Non-patent literature cited in the description


- *J. Pharmaceutical Sciences,* 1977, vol. 66, 1-19
- Prodrugs as Novel Delivery Systems, A.C.S. Symposium Series. **T. HIGUCHI ; V. STELLA.** Bioreversible Carriers in Drug Design. American Pharmaceutical Association and Pergamon Press, 1987, vol. 14
- **D. FLEISHER ; S. RAMON ; H. BARBRA.** Improved oral drug delivery: solubility limitations overcome by the use of prodrugs. *Advanced Drug Delivery Reviews,* 1996, vol. 19 (2), 115-130
- Remington's Pharmaceutical Sciences. Mack Publishing Company, 1985